# EUROPEAN PATENT APPLICATION

(11) **EP 2 202 232 A1**
(43) Date of publication of application: **30.06.2010**
(21) Application number: 08382090.2
(22) Date of filing: 26.12.2008
(51) Int. Cl.: C07D 413/04, C07D 413/14, C07D 471/04, A61K 31/4245, A61P 35/00, A61P 29/00, A61P 25/00, A61P 31/00

(54) **1,2,4-oxadiazole derivatives and their therapeutic use**

(71) Applicant: Laboratorios Almirall, S.A., 08022 Barcelona (ES)
(72) Inventor: Giulio Matassa, Victor, La Floresta, Barcelona, 081898 (GB); Aguilar Izquierdo, Nuria, 08005, Barcelona (ES); Mir Cepeda, Marta, 08024, Barcelona (ES); Carrascal Riera, Marta, 08028, Barcelona (ES); Fonquerna Pou, Silvia, 08030, Barcelona (ES); Cardus Figueras, Aranzazu, 08780, Pallejá (Barcelona) (ES); Castro Palomino Laria, Julio Cesar, 08330, Premia de Mar (Barcelona) (DE); Erra Sola, Montserrat, 08903, L'Hospitalet de Llobregat (Barcel (ES)
(74) Representative: Elzaburu Marquez, Alberto

(57) **Abstract**

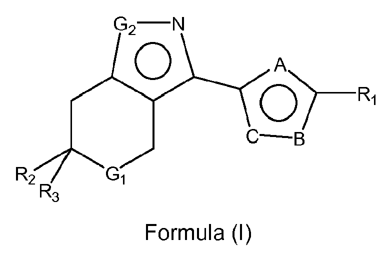

New derivatives of general formula (I), or pharmaceutically acceptable salts or N-oxides thereof wherein,
A is selected from the group consisting of -N-, -O- and -S-;
B and C are independently selected from the group consisting of-N- and -O-, with the proviso that at least two of A, B and C are nitrogen atoms;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 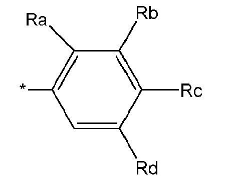 wherein:
• R^{a} represents a hydrogen atom, a halogen atom, a C₁₋₄ alkyl group, C₃₋₄ cycloalkyl group or a -CF₃ group;
• R^{b} represents a hydrogen atom, a halogen atom, a C₁₄ alkyl group, a -CF₃ group or a C₁₋₄ alkoxy group;
• R^{d} represents a hydrogen atom, a C₁₋₄ alkyl group or a C₁₋₄ alkoxy group;
• R^{c} represents:
○ a hydrogen atom, a C₁₋₄ hydroxyalkyl group, a C₁₋₄ aminoalkyl group which is optionally substituted with one or more substituents selected from halogen atoms, hydroxy groups and -CF₃ groups;
○ a 4 to 6-membered saturated N-containing heterocyclic ring optionally substituted with a C₁₋₂ carboxyalkyl group;
○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH²)₍₀₋₃₎-N H-(CH₂)₍₁₋₃₎-(NH)₍₀₋₁₎S(O)₂R' wherein,
■ R' represents a hydrogen atom or a C₁₋₄ alkyl group,
■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,

or R^{c} together with R^{d} form a C₅₋₆ cycloalkyl group optionally substituted by a -NHR^{f} group, wherein R^{f} is selected from the group consisting of a hydrogen atom and a carboxymethyl group;

R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

## Description

The present invention relates to new chemical compounds, in particular to 5-indazole derivatives, to processes for their preparation and to pharmaceutical compositions containing them. These compounds are potent agonists of S1P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1P) is a pleiotropic lipid mediator that exhibits a broad spectrum of biological activities, including cell proliferation, survival, lymphocyte trafficking, cytoskeletal organization, and morphogenesis. S1P is generated from endogenous sphingosine through phosphorylation by specific kinases, named sphingosine kinases 1 and 2. The levels of S1P in biological fluids and tissues are tightly regulated by the balance between its synthesis by sphingosine kinases and its degradation by S1P lyase. This tight control is important since an excessive production of S1P has been associated to various pathological conditions, such as angiogenesis and vascular permeability changes in cancer, inflammation, myocardial infarction or transplant rejection.

Gene deletion studies and reverse pharmacology have provided evidence that most of the effects of S1P are mediated via five G-protein coupled receptor subtypes, named S1P1 to S1P5 (Brinkmann, Pharmacology & therapeutics 115:84-105, 2007). The interest on this family of receptors increased following the discovery that they were the pharmacological target of FTY720. This compound, a synthetic analog of a natural product derived from the fungus *Isaria sinclairii*, exhibited a peculiar immunomodulatory potential in vivo. When administered in vivo, it caused lymphopenia, due to the sequestration of lymphocytes from the blood into the lymph nodes and Peyer's patches. The close structural similarity of FTY720 to sphingosine, together with the discovery of the formation of phosphorylated FTY720 in vivo (FTY720P) prompted to speculate that FTY720-P could be acting as a mimetic of S1P. This proven to be the case and it was later on demonstrated that FTY-P binds 4 of the five known S1P receptors, namely S1P1, S1P3, S1P4 and S1P5.

Expression analysis identified S1P1 as the dominant S1P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). FTY720 is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943) cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987),.

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes, systemic lupus erythematosis and Sjögrn's syndrome.

Transplant rejections include, but are not limited to, rejections of organs such as kidney, liver, heart, lung, pancreas, cornea and skin transplants and graft-versus-host disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies.

Angiogenesis-related disorders that may be prevented or treated include but are not limited to hemangiomas, ocular neovascularization, macular degeneration or diabetic retinopathy.

Pain, including neuropathic pain, that may be prevented or treated includes but is not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation, trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction and complications from cardiac surgery. Cardiovascular diseases may also refer to improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain 5-indazole derivatives are novel and potent agonists of S1P1 and can therefore be used in the treatment or prevention of these diseases.

Thus the present invention is directed to new 5-indazole derivatives of formula (I) or pharmaceutically acceptable salts or N-oxides thereof
1. of general formula (I), or a pharmaceutically acceptable salt or N-oxide thereof: 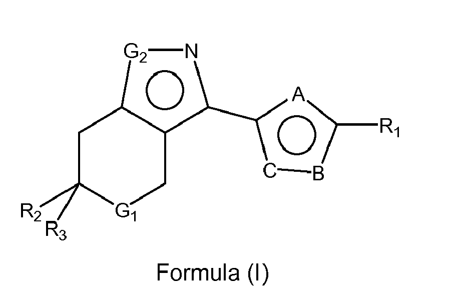 Wherein,
A is selected from the group consisting of -N-, -O- and -S-;
B and C are independently selected from the group consisting of-N- and -O-, with the proviso that at least two of A, B and C are nitrogen atoms;
G¹ is selected from the group consisting of -CH₂-, -NH- and -O-;
G² is selected from the group consisting of -NR⁴- and -O-;
R¹ represents:
   ➢ a 8 to 10 membered bicyclic N-containing heteroaryl group optionally substituted with a C₁₋₄ carboxyalkyl group or a C₁₋₄ aminoalkyl group,
   ➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
   ➢ a pyridone group substituted with one or more C₁₋₄ alkyl groups; C₁₋₄ haloalkyl groups or C₁₋₄ aminoalkyl groups, or
   ➢ a group of formula: 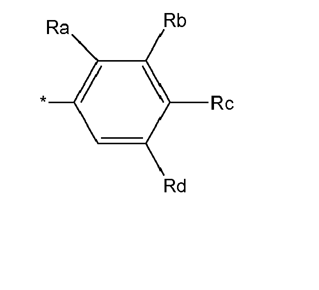 wherein:
      - R^{a} represents a hydrogen atom, a halogen atom, a C₁₋₄ alkyl group, C₃₋₄ cycloalkyl group or a -CF₃ group;
      - R^{b} represents a hydrogen atom, a halogen atom, a C₁₋₄ alkyl group, a -CF₃ group or a C₁₋₄ alkoxy group;
      - R^{d} represents a hydrogen atom, a C₁₋₄ alkyl group or a C₁₋₄ alkoxy group;
      - R^{c} represents:
         o A hydrogen atom, a C₁₋₄ hydroxyalkyl group, a C₁₋₄ aminoalkyl group which is optionally substituted with one or more substituents selected from halogen atoms, hydroxy groups and -CF₃ groups;
         o a 4 to 6-membered saturated N-containing heterocyclic ring optionally substituted with a C₁₋₂ carboxyalkyl group;
         ○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R'or -(CH₂)₍₀₋₃₎-NH-(CH₂)₍₁₋₃₎-(NH)₍₀₋₁₎S(O)₂R' wherein,
            ■ R' represents a hydrogen atom or a C₁₋₄ alkyl group,
            ■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
            ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
   or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

Further objectives of the present invention are to provide a method for preparing said compounds; pharmaceutical compositions comprising an effective amount of said compounds; compounds of formula I for use in the treatment of the human or animal body, the use of the compounds of the invention in the manufacture of a medicament for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases, and methods of treatment of pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of a therapeutically effective amount of a compound of the invention to a subject in need of treatment.

As used herein the term alkyl embraces optionally substituted, linear or branched hydrocarbon radicals having 1 to 8, preferably 1 to 4 carbon atoms. Examples include methyl, ethyl, *n*-propyl, *i*-propyl, *n*-butyl and *tert*-butyl radicals.

As used herein, a haloalkyl group is a said alkyl group, for example a C₁₋₄ or C₁₋₂ alkyl group, which is attached to 1, 2 or 3 halogen atoms. The halogen atom is preferably a fluorine atom. Preferably, said haloakyl group is chosen from -CH₂F -CF₂H, -CF₃ and -CH₂CF₃. -CF₃ and -CH₂CF₃ are preferred.

As used herein, the term hydroxyalkyl embraces linear or branched alkyl radicals having 1 to 4 carbon atoms, any one of which may be substituted with one or more hydroxyl radicals. Examples of such radicals include hydroxymethyl, hydroxyethyl, hydroxypropyl, hydroxybutyl and 1,2-dihydroxypropyl.

As used herein, the term aminoalkyl embraces linear or branched alkyl radicals having 1 to 4 carbon atoms, any one of which may be substituted with one or more amino groups. Examples of such radicals include aminomethyl, aminoethyl, aminopropyl and aminobutyl.

As used herein, the term carboxyalkyl embraces linear or branched alkyl radicals having 1 to 4 carbon atoms, any one of which may be substituted with one or more carboxy radicals. Examples of such radicals include carboxymethyl, carboxyethyl, carboxypropyl, carboxybutyl and 1,2-dicarboxypropyl.

As used herein the term alkoxy embraces optionally substituted, linear or branched oxy-containing radicals each having 1 to 8, preferably, 1 to 4 carbon atoms. Examples include methoxy, ethoxy, *n*-propoxy, *i*-propoxy, *n*-butoxy and *tert*-butoxy radicals.

As used herein, the term aminoalkoxy embraces linear or branched alkoxy radicals having 1 to 4 carbon atoms, any one of which may be substituted with one or more amino groups. Examples of such radicals include aminomethoxy, aminoethoxy, aminopropoxy and aminobutoxy.

As used herein, the term cycloalkyl embraces optionally substituted saturated carbocyclic radicals and, unless otherwise specified, a cycloalkyl radical typically has from 3 to 7, preferably from 3 to 4 carbon atoms. Examples include cyclopropyl, cyclobutyl, cyclopentyl and cyclohexyl. When a cycloalkyl radical carries 2 or more substituents, the substituents may be the same or different. Unless otherwise specified, the substitutents on a cycloalkyl radical are typically themselves unsubstituted.

As used herein, the term heteroaryl radical embraces typically optionally substituted 5-to 10- membered ring systems comprising at least one heteroaromatic ring and containing at least one heteroatom selected from O, S and N. A heteroaryl radical may be a single ring or two or more fused rings wherein at least one ring contains a heteroatom. A said optionally substituted heteroaryl radical is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidnyl and the various pyrrolopyridyl radicals.

As used herein, the term bicyclic N-containing heteroaryl group is typically an optionally substituted, fused 8 to 10 membered ring system comprising at least one heteroatomic ring, containing a nitrogen atom and optionally one or more, for example, 1, 2 or 3, preferably 1, further heteroatoms selected from O, S and N, preferably N. A said optionally substituted bicyclic N-containing heteroaryl group is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Example include benzofuranyl, benzoxazolyl, benzimidazolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, indolinyl, isoindolinyl, isoindolyl, pteridinyl, pyrazolopyrimidinyl, thienopyrimidnyl and pyrrolopyridyl. Pyrrolopyridyl is preferred. 1H-pyrrolo-2,3-b]pyridin-1-yl is more preferred.

As used herein, the term heterocyclic radical embraces typically optionally substituted non-aromatic, saturated or unsaturated C₃-C₁₀ carbocyclic ring systems, preferably C₄-C₆ carbocyclic rings, such as 4, 5 or 6 membered radicals, in which one or more, for example 1, 2, or 3 of the carbon atoms preferably 1 or 2 of the carbon atoms are replaced by a heteroatom selected from N, O and S. Saturated heterocyclic radicals are preferred. A heterocyclic radical may be a single ring or two or more fused rings wherein at least one ring contains a heteroatom. When a heterocyclyl radical carries 2 or more substituents, the substituents may be the same or different. Typically, the substituents on a heterocyclyl radical are themselves unsubstituted, unless otherwise specified.

Examples of heterocyclic radicals include azetidyl, piperidyl, pyrrolidyl, pyrrolinyl, piperazinyl, morpholinyl, thiomorpholinyl, pyrazolinyl, pirazolidinyl, and quinuclidinyl.

As used herein, the term saturated N-containing heterocyclic ring is typically a 4 to 6 membered, optionally substituted heterocyclic radical as defined herein, which is a saturated C4 to C6 carbocyclic ring, such as a 4, 5 or 6 membered radical, in which one of the carbon atoms is repaced by N and in which, optionally, one or more, for example 1 or 2, preferably 1 further carbon atom is repaced by a heteroatom selected from N, O and S.

Examples include azetidyl, piperidyl, pyrrolidyl, pyrrolinyl, piperazinyl, morpholinyl, thiomorpholinyl, and pirazolidinyl.

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge on the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

Typically, in compounds of formula I where R⁴ is a C₃₋₄ cycloalkyl-C₁₋₄ alkyl group, said group is bonded to the nitrogen atom through the alkyl group, i.e. -C₁₋₄ alkyl-C₃₋₄ cycloalkyl.

Typically, when R^{c} represents -(CH₂)₍₀₋₄₎-CONHS(O)₂R', -(CH₂)₍₀₋₄₎-NHS(O)₂R'or - (CH₂)₍₀₋₃₎-NH-(CH₂)₍₁₋₃₎-(NH)₍₀₋₁₎S(O)₂R' , then R' is not a hydrogen atom.

Typically, when R^{c} represents -(CH₂)₍₀₋₄₎-NHC(O)R", then R" is not a hydrogen atom.

As used herein, an N-oxide is formed from the tertiary basic amines or imines present in the molecule, using a convenient oxidising agent.

Typically, A is selected from the group consisting of -N- and -O-. Preferably A represents -N-.

More preferably, both A and B represent -N- and C represents -O-.

Typically, G¹ represents a -CH₂- or a -O- group. Preferably G¹ represents a -CH₂- group.

Typically, R² and R³ are independently selected from the group consisting of hydrogen atoms, fluorine atoms and methyl groups. Preferably, both R² and R³ are methyl groups.

Typically, R⁴ is selected from the group consisting of a C₃₋₄ cycloalkyl-C₁₋₄ alkyl group, C₁₋₄ haloalkyl group and a linear or branched unsubstituted C₁₋₄ alkyl group.

Preferably, G² represents -NR⁴-, and R⁴ is selected from the group consisting of a methyl group, ethyl group, t-butyl group, cyclopropylmethyl group and 2,2,2-trifluoroethyl group. More preferably, R⁴ represents a methyl or an ethyl group.

Typically, R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 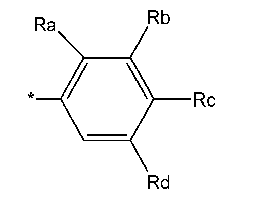 wherein:
   - R^{a} represents a hydrogen atom or a C₁₋₄ alkyl group;
   - R^{b} represents a hydrogen atom or C₁₋₄ alkyl group;
   - R^{d} represents a hydrogen atom or a C₁₋₄ alkyl group;
   - R^{c} represents:
      ○ a C₁₋₄ hydroxyalkyl group or a C₁₋₄ aminoalkyl substitued by one or more halogen atoms;
      ○ -(CH₂)₍₂₋₃₎-C(O)OR', -(CH₂)₍₀₋₂₎-C(O)NR'R", -O-(CH₂)₍₂₋₃₎NR'R", -(CH₂)₍₂₋₃₎-NHC(O)R", -S(O)₂NR'R", -(CH₂)₍₀₋₃₎-NR'R" or -(CH₂)₍₁₋₂₎-CONHS(O)₂R' wherein,
         ■ R' represents a hydrogen atom or a methyl group,
         ■ R" represents a hydrogen atom, a C₁₋₄ alkyl group, C₁₋₄ carboxyalkyl group, C₁₋₄ haloalkyl group or a C₁₋₄ hydroxyalkyl group, or
         ■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
   or R^{c} together with R^{d} form a cyclohexyl group substituted with a carboxymethylamino group.

Preferably, R¹ represents:
➢ a pyridyl group substituted with two or three substituents selected from hydroxy groups, methyl and ethyl groups, or
➢ a group of formula: 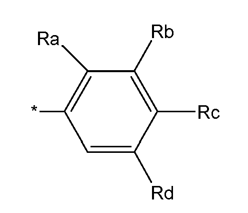 wherein:
   - R^{a} represents a hydrogen atom or a methyl group;
   - R^{b} represents a hydrogen atom, a methyl group;
   - R^{d} represents a hydrogen atom or a methyl group;
   - R^{c} represents: -(CH₂)₍₂₋₃₎-C(O)OR', -(CH₂)₂-C(O)NR'R" or -(CH₂)₍₂₋₃₎-NR'R",
      wherein
      ○ R' represents a hydrogen atom;
      ○ R" represents a hydrogen atom, a C₁₋₂ carboxyalkyl group, a C₁₋₄ haloalkyl group or a C₁₋₂ hydroxyalkyl group; or
      ○ R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 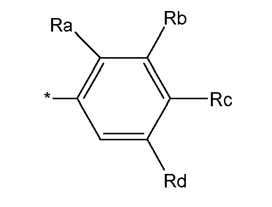 wherein:
○ R^{a} represents a hydrogen atom;
○ both R^{b} and R^{d} represent methyl groups; and
○ R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

Typically, G¹ represents a -CH₂- group, G² represents a -NR₄- group, wherein R⁴ represents a methyl or ethyl group, both R² and R³ represent a methyl group, and
R¹ represents:
➢ a pyridyl group substituted with two or three substituents selected from hydroxy groups, methyl or ethyl groups, or
➢ a group of formula: 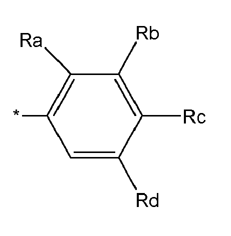 wherein:
   - R^{a} represents a hydrogen atom or a methyl group;
   - R^{b} represents a hydrogen atom, a methyl group,
   - R^{d} represents a hydrogen atom or a methyl group,
   - R^{c} represents: -(CH₂)₍₂₋₃₎-C(O)OR', -(CH₂)₂-C(O)NR'R" or -(CH₂)₍₂₋₃₎-NR'R",
      wherein:
      ○ R' represents a hydrogen atom;
      ○ R" represents a hydrogen atom, a C₁₋₂ carboxyalkyl group, a C₁₋₄ haloalkyl group or a C₁₋₂ hydroxyalkyl group, or
      ○ R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 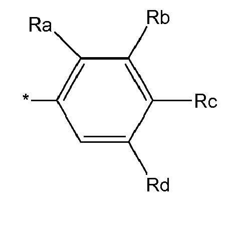 wherein
○ R^{a} represents a hydrogen atom;
○ both R^{b} and R^{d} represents a methyl group and
○ R^{c} represents -(CH₂)₍₂₋₃₎-C(O)OH or -(CH₂)₍₂₋₃₎-NHR", wherein R^{"} is selected from a hydrogen atom, a C₁₋₂ carboxyalkyl group and C₁₋₂ hydroxyalkyl group.

Typically, R¹ represents:
➢ an imidazo[1,2-a]pyridyl group or a 3H-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 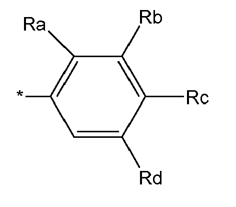 wherein:
   - R^{a} represents a hydrogen atom, a methyl group, cyclopropyl group or a CF₃ group;
   - R^{b} represents a hydrogen atom, a chlorine atom or a methyl group;
   - R^{d} represents a hydrogen atom or a methyl group;
   - R^{c} represents:
      ○ a C₁₋₄ hydroxyalkyl group or a C₁₋₄ aminoalkyl group substituted with one or more substituents selected from fluorine atoms and hydroxy groups;
      ○ a 4 to 6-membered saturated N-containing heterocyclic ring which is optionally substituted with a C₁₋₂ carboxyalkyl group
      ○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CONHS(O)₂R' -(CH₂)₍₀₋₄₎-NHS(O)₂R'or -(CH₂)₍₀₋₃₎-NH-(CH₂)₍₁₋₃₎-(NH)₍₀₋₁₎S(O)₂R' wherein
      ○ R' represents a hydrogen atom or a methyl group,
      ○ R" represents a hydrogen atom, a methyl group, a cyclopropyl group, a piperidyl group, a C₁₋₂ carboxyalkyl group, a CF₃ group, C₁₋₄ hydroxyalkyl group, or
      ○ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
   or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

Preferred compounds of the invention are represented by the formula (I'), or pharmaceutically acceptable salts or N-oxides thereof: 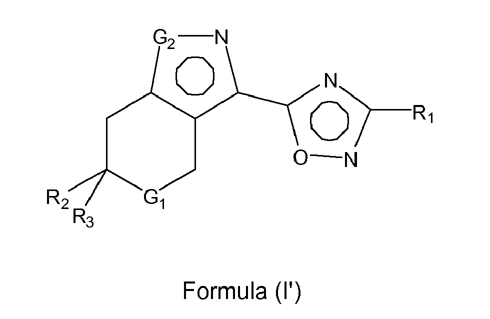 wherein,
G¹ is selected from the group consisting of -CH₂-, and -O-;
G² is selected from the group consisting of -NR⁴- and -O-;
R¹ represents:
   ➢ A pyrrolopyridyl group, which is unsubstituted or substituted with a C₁₋₂ carboxyalkyl group;
   ➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
   ➢ a pyridone group substituted with 1, 2 or 3 C₁₋₂ alkyl groups; or
   ➢ a group of formula: 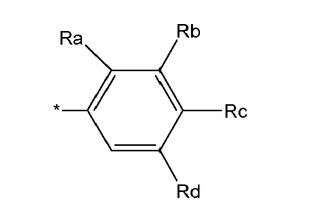 wherein:
      - R^{a} represents a hydrogen atom, a C₁₋₂ alkyl group, a cyclopropyl group or a -CF₃ group;
      - R^{b} represents a hydrogen atom, a chlorine atom, or a C₁₋₂ alkyl group;
      - R^{d} represents a hydrogen atom, or a C₁₋₂ alkyl group;
      - R^{c} represents:
         ○ a C₁₋₃ hydroxyalkyl group;
         ○ a carboxyethylpiperazine group;
         ○ -(CH₂)₍₀₋₂₎-C(O)OR', -(CH₂)₍₀₋₂₎-C(O)NR'R", -S(O)₂NR'R", or -(CH₂)₍₀₋₄₎-NR'R", wherein,
            ■ R' represents a hydrogen atom,
            ■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
            ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
      or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group,
   wherein said pyrrolopyridyl groups are typically 1H-pyrrolo-[2, 3-b]pyridyl groups.

Particular individual compounds of the invention include:
4-(5-(6,6-Dimethyl-4,5,6,7-tetrahydro-2H-indazol-3-yl)-1,2,4-oxadiazol-3-yl)benzenesulfonamide
(4-(5-(6,6-Dimethyl-4,5,6,7-tetrahydro-2H-indazol-3-yl)-1,2,4-oxadiazol-3-yl)phenyl)metanol
(4-(5-(1,6,6-Trimethyl-4,5,6,7-tetrahydro-1H-indazol-3-yl)-1,2,4-oxadiazol-3-yl)phenyl)methanol
4-(5-(1,6,6-Trimethyl-4,5,6,7-tetrahydro-1H-indazol-3-yl)-1,2,4-oxadiazol-3-yl)benzenesulfonamide
4-(5-(1-Ethyl-6,6-dimethyl-4,5,6,7-tetrahydro-1H-indazol-3-yl)-1,2,4-oxadiazol-3-yl)benzenesulfonamide
5-(1-Ethyl-6,6-dimethyl-4,5,6,7-tetrahydro-1H-indazol-3-yl)-3-(pyridin-4-yl)-1,2,4-oxadiazole
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid
4-(5-(1-Ethyl-6,6-dimethyl-4,5,6,7-tetrahydro-1H-indazol-3-yl)-1,2,4-oxadiazol-3-yl)benzoic acid
4-(5-(1-Ethyl-6,6-dimethyl-4,5,6,7-tetrahydro-1H-indazol-3-yl)-1,2,4-oxadiazol-3-yl)benzamide
5-(1-Ethyl-6,6-dimethyl-4,5,6,7-tetrahydro-1H-indazol-3-yl)-3-(4-(piperazin-1-yl)phenyl)-1,2,4-oxadiazole
2-(4-(5-(1-Ethyl-6,6-dimethyl-4,5,6,7-tetrahydro-1H-indazol-3-yl)-1,2,4-oxadiazol-3-yl)phenyl)acetic acid
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid
2-(4-(5-(1-Ethyl-6,6-dimethyl-4,5,6,7-tetrahydro-1H-indazol-3-yl)-1,2,4-oxadiazol-3-yl)phenyl)acetamide
4-(5-(1-Ethyl-6,6-dimethyl-4,5,6,7-tetrahydro-1H-indazol-3-yl)-1,2,4-oxadiazol-3-yl)pyridine 1-oxide
N-(4-(5-(1-Ethyl-6,6-dimethyl-4,5,6,7-tetrahydro-1H-indazol-3-yl)-1,2,4-oxadiazol-3-yl)phenyl)piperidin-4-amine
5-(1-Ethyl-6,6-dimethyl-4,5,6,7-tetrahydro-1H-indazol-3-yl)-3-(6-methoxypyridin-3-yl)-1,2,4-oxadiazole
5-(5-(1-Ethyl-6,6-dimethyl-4,5,6,7-tetrahydro-1H-indazol-3-yl)-1,2,4-oxadiazol-3-yl)pyridin-2-ol
4-(5-(1-Ethyl-6,6-dimethyl-4,5,6,7-tetrahydro-1H-indazol-3-yl)-1,2,4-oxadiazol-3-yl)pyridin-2-ol
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide
4-(5-(1-Benzyl-6,6-dimethyl-4,5,6,7-tetrahydro-1H-indazol-3-yl)-1,2,4-oxadiazol-3-yl)benzamide
4-(5-(1-tert-Butyl-6,6-dimethyl-4,5,6,7-tetrahydro-1H-indazol-3-yl)-1,2,4-oxadiazol-3-yl)benzamide
5-(1-Ethyl-6,6-dimethyl-4,5,6,7-tetrahydro-1H-indazol-3-yl)-3-(2-methoxypyridin-4-yl)-1,2,4-oxadiazole
4-(5-(1-Ethyl-6,6-dimethyl-4,5,6,7-tetrahydro-1H-indazol-3-yl)-1,2,4-oxadiazol-3-yl)-1-methylpyridin-2(1H)-one
1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid
(4-(5-(1-Ethyl-6,6-dimethyl-4,5,6,7-tetrahydro-1H-indazol-3-yl)-1,2,4-oxadiazol-3-yl)phenyl)methanol
4-(5-(6,6-Dimethyl-1-phenyl-4,5,6,7-tetrahydro-1H-indazol-3-yl)-1,2,4-oxadiazol-3-yl)benzamide
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol
5-(5-(1-Ethyl-6,6-dimethyl-4,5,6,7-tetrahydro-1H-indazol-3-yl)-1,2,4-oxadiazol-3-yl)-3-methylpyridin-2-ol
4-(5-(1-Ethyl-6,6-dimethyl-4,5,6,7-tetrahydro-1H-indazol-3-yl)-1,2,4-oxadiazol-3-yl)-3-methylbenzamide
4-(4-(5-(1-Ethyl-6,6-dimethyl-4,5,6,7-tetrahydro-1H-indazol-3-yl)-1,2,4-oxadiazol-3-yl)benzyl)morpholine
3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one
5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one
N-Cyclopropyl-4-(5-(1-ethyl-6,6-dimethyl-4,5,6,7-tetrahydro-1H-indazol-3-yl)-1 ,2,4-oxadiazol-3-yl)benzamide
1-(4-(5-(1-Ethyl-6,6-dimethyl-4,5,6,7-tetrahydro-1H-indazol-3-yl)-1,2,4-oxadiazol-3-yl)phenyl)-N-methylmethanamine
5-(1-Ethyl-6,6-dimethyl-4,5,6,7-tetrahydro-1H-indazol-3-yl)-3-(3-methylpyridin-4-yl)-1,2,4-oxadiazole
4-(5-(6,6-Dimethyl-1-(2,2,2-trifluoroethyl)-4,5,6,7-tetrahydro-1H-indazol-3-yl)-1 ,2,4-oxadiazol-3-yl)benzamide
(4-(5-(1-Ethyl-6,6-dimethyl-4,5,6,7-tetrahydro-1H-indazol-3-yl)-1,2,4-oxadiazol-3-yl)phenyl)methanamine
5-(1-Ethyl-6,6-dimethyl-4,5,6,7-tetrahydro-1H-indazol-3-yl)-3-(3-methylpyridin-2-yl)-1,2,4-oxadiazole
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine
5-(5-(1-Ethyl-6,6-dimethyl-4,5,6,7-tetrahydro-1H-indazol-3-yl)-1,2,4-oxadiazol-3-yl)-4-methylpyridin-2-ol
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid
5-(5-(1-Ethyl-6,6-dimethyl-4,5,6,7-tetrahydro-1H-indazol-3-yl)-1,2,4-oxadiazol-3-yl)-6-methylpyridin-2-ol
5-(1-Ethyl-6,6-dimethyl-4,5,6,7-tetrahydro-1H-indazol-3-yl)-3-(4-methylpyridin-3-yl)-1,2,4-oxadiazole
5-(1-Ethyl-6,6-dimethyl-4,5,6,7-tetrahydro-1H-indazol-3-yl)-3-(4-(trifluoromethyl)pyridin-3-yl)-1,2,4-oxadiazole
5-(1-Ethyl-6,6-dimethyl-4,5,6,7-tetrahydro-1H-indazol-3-yl)-3-(imidazo[1,2-a]pyridin-6-yl)-1,2,4-oxadiazole
3-(4-(5-(1-Ethyl-6,6-dimethyl-4,5,6,7-tetrahydro-1H-indazol-3-yl)-1,2,4-oxadiazol-3-yl)-3-methylphenyl)propanamide
2-(4-(5-(1-Ethyl-6,6-dimethyl-4,5,6,7-tetrahydro-1H-indazol-3-yl)-1,2,4-oxadiazol-3-yl)-3-methylphenyl)ethanamine
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine
4-(5-(1-Ethyl-6,6-dimethyl-4,5,6,7-tetrahydro-1H-indazol-3-yl)-1,2,4-oxadiazol-3-yl)-3-(trifluoromethyl)benzoic acid
4-(5-(1-Ethyl-6,6-dimethyl-4,5,6,7-tetrahydro-1H-indazol-3-yl)-1,2,4-oxadiazol-3-yl)-3-(trifluoromethyl)benzamide
5-(1-Ethyl-6,6-dimethyl-4,5,6,7-tetrahydro-1H-indazol-3-yl)-3-(2-methylpyridin-3-yl)-1,2,4-oxadiazole
5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine
3-Cyclopropyl-4-[5-(1-ethyl-6,6-dimethyl-4,5,6,7-tetrahydro-1H-indazol-3-yl)-1,2,4-oxadiazol-3-yl]benzamide
5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol
5-(5-(6,6-Dimethyl-1-(2,2,2-trifluoroethyl)-4,5,6,7-tetrahydro-1H-indazol-3-yl)-1 ,2,4-oxadiazol-3-yl)-3-ethyl-6-methylpyridin-2-ol
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide
3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol
3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine
2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine
3-(2,6-Dimethyl-4-(5-(1,6,6-trimethyl-4,5,6,7-tetrahydro-1H-indazol-3-yl)-1,2,4-oxadiazol-3-yl)phenyl)propanoic acid
3-(2,6-Dimethyl-4-(5-(1,6,6-trimethyl-4,5,6,7-tetrahydro-1H-indazol-3-yl)-1,2,4-oxadiazol-3-yl)phenyl)propanamide
3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine
2-(2,6-Dimethyl-4-(5-(1,6,6-trimethyl-4,5,6,7-tetrahydro-1H-indazol-3-yl)-1,2,4-oxadiazol-3-yl)phenyl)ethanamine
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine
5-(5-(6,6-Dimethyl-1-(pyridin-3-ylmethyl)-4,5,6,7-tetrahydro-1H-indazol-3-yl)-1 ,2,4-oxadiazol-3-yl)-3-ethyl-6-methylpyridin-2(1H)-one
2-(Dimethyl-4,5,6,7-tetrahydrobenzo[d]isoxazol-3-yl)-1,2,4-oxadiazol-3-yl)-2,6-dimethylphenethylamino)ethanoic acid
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid
3-(Dimethyl-4,5,6,7-tetrahydrobenzo[d]isoxazol-3-yl)-1,2,4-oxadiazol-3-yl)-2,6-dimethylphenethylamino)propanoic acid
3-Ethyl-5-(5-(1-ethyl-4,5,6,7-tetrahydro-1H-indazol-3-yl)-1,2,4-oxadiazol-3-yl)-6-methylpyridin-2(1H)-one
3-Ethyl-6-methyl-5-(5-(1,6,6-trimethyl-4,5,6,7-tetrahydro-1H-indazol-3-yl)-1,2,4-oxadiazol-3-yl)pyridin-2-ol
5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one
5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine
2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine
3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid
3-Ethyl-5-(5-(1-ethyl-6,6-difluoro-4,5,6,7-tetrahydro-1H-indazol-3-yl)-1 ,2,4-oxadiazol-3-yl)-6-methylpyridin-2(1H)-one
3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid
5-(5-(1-(Cyclopropylmethyl)-6,6-dimethyl-4,5,6,7-tetrahydro-1H-indazol-3-yl)-1 ,2,4-oxadiazol-3-yl)-3-ethyl-6-methylpyridin-2(1H)-one
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid
3-(2-Methyl-4-(5-(1,6,6-trimethyl-4,5,6,7-tetrahydro-1H-indazol-3-yl)-1,2,4-oxadiazol-3-yl)phenyl)propanoic acid
4-(2,6-Dimethyl-4-(5-(1,6,6-trimethyl-4,5,6,7-tetrahydro-1H-indazol-3-yl)-1,2,4-oxadiazol-3-yl)phenethyl)morpholine
3-(4-(5-(6,6-Dimethyl-4,5,6,7-tetrahydro-1H-indazol-3-yl)-1,2,4-oxadiazol-3-yl)-2,6-dimethylphenyl)propane-1,2-diol
3-(4-(5-(1-Ethyl-6,6-dimethyl-4,5,6,7-tetrahydro-1H-indazol-3-yl)-1,2,4-oxadiazol-3-yl)phenylamido)propanoic acid
3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol
3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid
3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol
3-(1-Ethyl-6,6-dimethyl-4,5,6,7-tetrahydro-1H-indazol-3-yl)-5-(pyridin-4-yl)-1,2,4-oxadiazole
5-(1-Ethyl-6,6-dimethyl-4,5,6,7-tetrahydro-1H-indazol-3-yl)-3-o-tolyl-1,2,4-oxadiazole
3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid
3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid
or a pharmaceutically acceptable salt or N-oxide thereof

Of outstanding interest are:
4-(5-(1-Ethyl-6,6-dimethyl-4,5,6,7-tetrahydro-1H-indazol-3-yl)-1,2,4-oxadiazol-3-yl)benzenesulfonamide
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid
4-(5-(1-Ethyl-6,6-dimethyl-4,5,6,7-tetrahydro-1H-indazol-3-yl)-1,2,4-oxadiazol-3-yl)benzamide
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid
2-(4-(5-(1-Ethyl-6,6-dimethyl-4,5,6,7-tetrahydro-1H-indazol-3-yl)-1,2,4-oxadiazol-3-yl)phenyl)acetamide
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide
4-(5-(1-Benzyl-6,6-dimethyl-4,5,6,7-tetrahydro-1H-indazol-3-yl)-1,2,4-oxadiazol-3-yl)benzamide
4-(5-(1-tert-Butyl-6,6-dimethyl-4,5,6,7-tetrahydro-1H-indazol-3-yl)-1,2,4-oxadiazol-3-yl)benzamide
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol
5-(5-(1-Ethyl-6,6-dimethyl-4,5,6,7-tetrahydro-1H-indazol-3-yl)-1,2,4-oxadiazol-3-yl)-3-methylpyridin-2-ol
4-(5-(1-Ethyl-6,6-dimethyl-4,5,6,7-tetrahydro-1H-indazol-3-yl)-1,2,4-oxadiazol-3-yl)-3-methylbenzamide
5-(1-Ethyl-6,6-dimethyl-4,5,6,7-tetrahydro-1H-indazol-3-yl)-3-(3-methylpyridin-4-yl)-1,2,4-oxadiazole
4-(5-(6,6-Dimethyl-1-(2,2,2-trifluoroethyl)-4,5,6,7-tetrahydro-1H-indazol-3-yl)-1 ,2,4-oxadiazol-3-yl)benzamide
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid
5-(5-(1-Ethyl-6,6-dimethyl-4,5,6,7-tetrahydro-1H-indazol-3-yl)-1,2,4-oxadiazol-3-yl)-6-methylpyridin-2-ol
3-(4-(5-(1-Ethyl-6,6-dimethyl-4,5,6,7-tetrahydro-1H-indazol-3-yl)-1,2,4-oxadiazol-3-yl)-3-methylphenyl)propanamide
2-(4-(5-(1-Ethyl-6,6-dimethyl-4,5,6,7-tetrahydro-1H-indazol-3-yl)-1,2,4-oxadiazol-3-yl)-3-methylphenyl)ethanamine
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine
5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine
3-(2,6-Dimethyl-4-(5-(1,6,6-trimethyl-4,5,6,7-tetrahydro-1H-indazol-3-yl)-1,2,4-oxadiazol-3-yl)phenyl)propanamide
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine
2-(2,6-Dimethyl-4-(5-(1,6,6-trimethyl-4,5,6,7-tetrahydro-1H-indazol-3-yl)-1,2,4-oxadiazol-3-yl)phenyl)ethanamine
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine
2-(Dimethyl-4,5,6,7-tetrahydrobenzo[d]isoxazol-3-yl)-1,2,4-oxadiazol-3-yl)-2,6-dimethylphenethylamino)ethanoic acid
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid
3-(Dimethyl-4,5,6,7-tetrahydrobenzo[d]isoxazol-3-yl)-1,2,4-oxadiazol-3-yl)-2,6-dimethylphenethylamino)propanoic acid
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid

Compounds of general formula (I) may be obtained following the synthetic scheme depicted in figure 1.

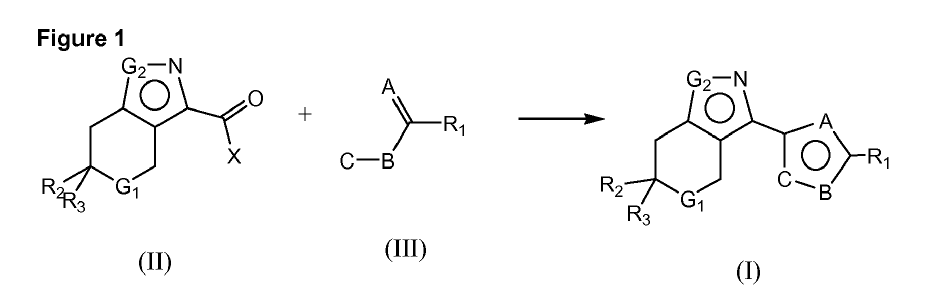

Compounds of general formula (I) may be prepared by reacting intermediates of general formula (II) wherein X represents a hydroxy group or a chlorine atom, with intermediates formula (III) in a one pot reaction. This reaction is carried out in a solvent such as DMFor THF, optionally in the presence of one or more coupling agents such as 2-(1H-benzotriazole-1-yl)-1,2,3,3-tetramethyluronium tetrafluoroborate (TBTU), N-(3-dimethylaminopropyl)-N'-ethyl-carbodiimide (EDC), dicyclohexyl carbodiimide (DCC), 1-hydroxybenzotriazole (HOBt), O-(benzotriazol-1-yl)-N,N,N',N'-tetramethyluroniium hexafluorophosphate (HBTU), carbonyl diimidazole (CDI), or the like and optionally in the presence of a base such as triethylamine, Hünig's base, sodium hydride, potassium carbonate, or the like. The temperature of the reaction is between 40 and 150°C and is carried out in a standard reactor.

An alternative method for the preparation of compounds of formula (I) may be done following a two steps synthesis. The first step is carried out by a coupling intermediates of formula (I) with one or more coupling agent as described before and then, in a second step, a subsequent cyclization in a solvent such as xylene, toluene, benzene, pyridine, DMF, dichloromethane, acetic acid, trifluoroacetic acid,. at room temperature or elevated temperatures, optionally in the presence of auxiliaries such as acid (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents such as oxalyl chloride, a carboxylic acid anhydride, phosphoryl trichloride (POCl₃), tetrabutylammonium fluoride (TBAF), molecular sieves, etc.

Alternatively compounds of general formula (I) may be prepared as shown in figure 2,

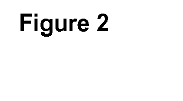

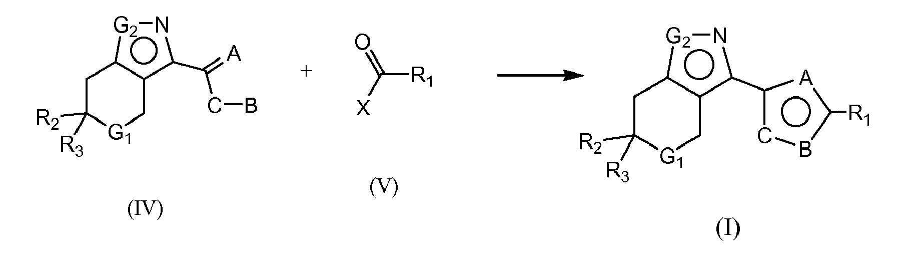

The reaction is carried out by reacting intermediates of general formula (IV) with intermediates of general formula (V) following the same synthetic procedures described above in figure (I)

Intermediates of general formula (III) and (IV) may be prepared following the synthetic schemes depicted in figure 3. 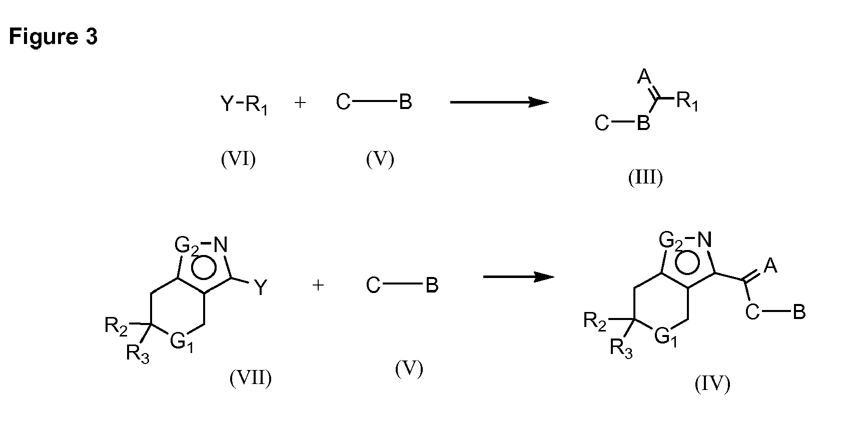 Intermediates of formula (III) and (IV) may be obtained from the corresponding intermediates of formula (VI) and (VII), respectively wherein Y represents -CN, -COOH, -COCI or -COOR'. The synthesis is carried out by reacting intermediates of formula (VI) or (VII), with hydroxylamine or hydrazine or any salt thereof as intermediates of formula (V), respectively, in a solvent such as THF, methanol, ethanol, pyridine, optionally in the presence of a base such as sodium bicarbonate, sodium carbonate, potassium carbonate, triethylamine, sodium ethoxide, and at a temperature ranging from room temperature to the boiling point of the solvent. This reaction may be optionally carried out in the presence of a coupling agent such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole,.

In the particular case of compounds of general formula (I) wherein A and B are nitrogen and C is an oxygen, compounds of general formula (la) may be prepared following the synthetic scheme depicted in figure 4.

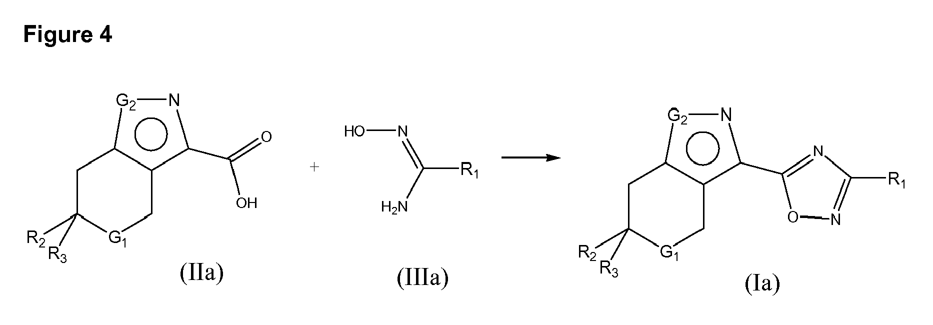

The compounds of general formula (la) may be prepared by the condensation of the tetrahidroindazole-3-carboxylic acid or tetrahidroisoxazole-3-carboxylic acid derivatives of formula (IIa) with the corresponding carboximidamide derivative of formula (IIIa) in a solvent such as DMF, THF, in the presence of one or more coupling agents such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole, O-(benzotriazol-1-yl)-N,N,N',N'-tetramethyluroniium hexafluorophosphate, carbonyl diimidazole, and optionally in the presence of a base such as triethylamine, Hünig's base, sodium hydride, potassium carbonate, at a temperature between 40 and 150°C and in a standard reactor.

An alternative synthetic method may be carried out by first coupling intermediates of formula (IIa) as described before, and subsequent cyclazation in a solvent such as xylene, toluene, benzene, pyridine, dimethylformamide, dichloromethane, acetic acid, trifluoroacetic acid, at room temperature or elevated temperatures, optionally in the presence of auxiliaries such as acid (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents (e.g. oxalyl chloride, a carboxylic acid anhydride, phosphoryl trichloride, tetrabutilamonium fluoride, molecular sieves etc.)

Compounds of general formula (IIa) may be prepared following the synthetic scheme depicted in figure 5.

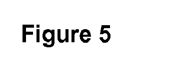

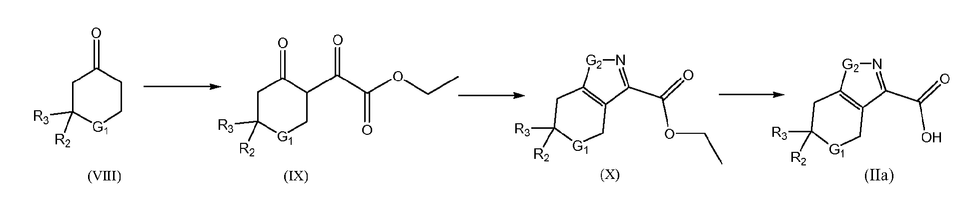

Intermediates of general formula (IX) may be prepared by the reaction of the corresponding commercial ketone derivatives of formula (VIII) with diethyloxalate in a basic media such as sodium ethoxide in an protic solvent such as ethanol and at a temperature between 20°C and the boiling point of the solvent. Ketones of general formula (VIII) are either commercially available or may be prepared using synthetic methods known in the art.

Intermediates of general formula (X) may be prepared by the condensation of the intermediates of formula (IX) with the corresponding hydrazine or hydroxylamine in basic media such as triethylamine and in a protic solvent such as ethanol at a temperature between 20°C and the boiling point of the solvent.

Hydrolysis of the ethyl ester derivatives of formula (X) in basic conditions with a base such as aqueous sodium hydroxide or litium hydroxide in a solvent such as methanol, ethanol or THF or a mixture of them at a temperature between 20 and 80°C or in acidic condicions with an acid such as HCl and a solvent such as water or ethanol or a mixture of them at a temperature between 20 and 80°C gives the acid intermediates of formula (IIa).

Intermediates of general formula (IIIa) may be prepared following the synthetic scheme depicted in figure 6.

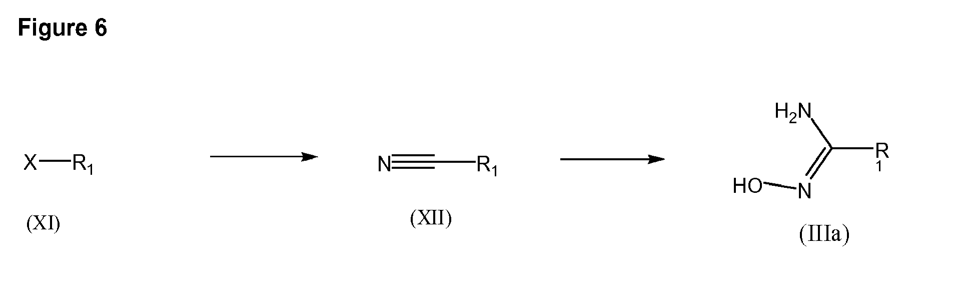

Internmediates of formula (IIIa) may be obtained by the reaction of hydroxylamine hydrochloride or any of its salts with the corresponding nitrile (XII) in basic media such as sodium bicarbonate or triethylamine in a solvent such as THF, methanol or ethanol and at a temperature from 40 to 100°C. If the cyanoaryl derivative of formula (XII) is not commercially available it may be obtained from the corresponding derivative of formula (XI) wherein X represents a bromide or a triflate, by reacting with a source of a cyanide such as copper (I) cyanide, in a high boiling point solvent such as N-methylpirrrolidine, dimethylformamide or dimethylsulfoxide at a temperature between 150-200°C. Alternatively dicyanozinc may be used with a palladium catalyst such as tetrakis(triphenylphosphine)-palladium(0) in a high boiling point solvent, in a standard reactor or a microwave reactor.

Starting compounds are commercially available or may be obtained following the conventional synthetic methods already known in the art.

Depending on the nature of the functionalities present in R₁ and in the residues Rₐ to R_{d}, these functionalities may require temporary protection. Appropriate protecting groups are known in the art and include e.g a *tert*-butyl or ethyl or methyl to protect an acid, a *tert*-butyloxycarbonyl (BOC) to protect an amine, etc. These protecting groups may be employed according to standard methodology (e.g. T.W. Greene, P. G. M. Wuts, Protective Groups in Organic Synthesis, 3rd Edition, Wiley New York, 1991).

In the particular case where R₁ represents a group of formula: 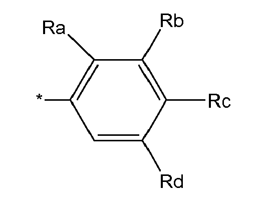 wherein R^{c} is -(CH₂)₂COOR', intermediates of formula (IIIb) may be obtained following the synthetic path shown in Figure 7.

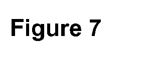

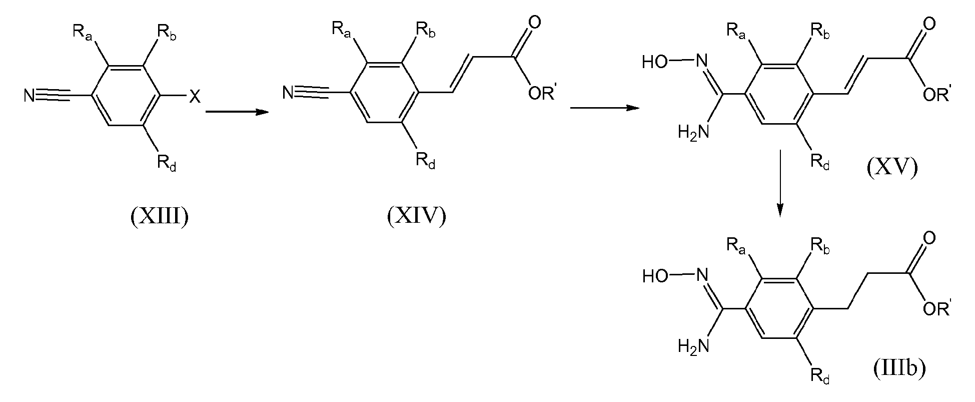

A Heck reaction of the corresponding precursor (XIII), wherein X represents triflate, bromo or iodo, which are known or may be prepared according to known procedures, with acrylate yields the intermediate of formula (XIV). Reaction of these intermediates with hydroxylamine as described above followed by a catalytic hydrogenation, gives intermediates of general formula (IIIb).

In the particular case where R_{c} is -(CH₂)₍₀₋₄₎-NR'R" group or a -(CH₂)₍₀₋₄)-CONR'R" group compounds of formula (Id), (Ie) and (If) may be obtained from compounds of general formula (Ib) wherein R_{c} is a -(CH₂)₍₀₋₄)-COOR'group following the synthetic path shown in Figure 8. 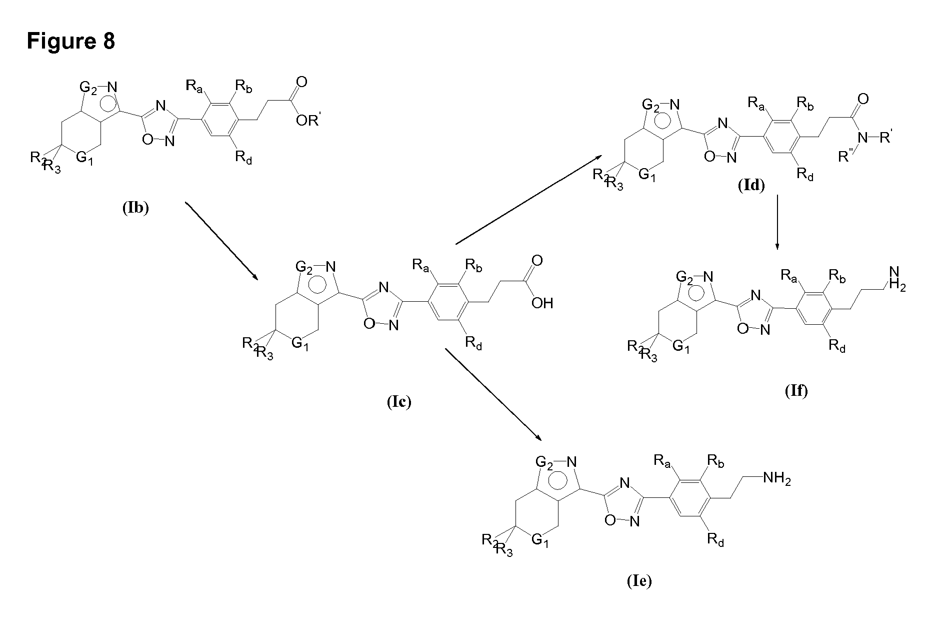 From compounds of formula (lb), the corresponding acid derivatives may be prepared by basic hydrolysis in a protic solvent such as methanol, ethanol or water with a base such as litium hydroxide or sodium hydroxide or by acidic hydrolysis in trifluoroacetic acid, clorhidric acid or dioxane/HCl, thus yielding to compounds of formula (lc)

The amides derivatives of formula (ld) may be prepared by reacting compounds of formula (lc) with ammonia, an amine or aminoacid of general formula HNR'R" in the presence of an activating agent such as N-(3-dimethylaminopropyl)-N'-ethylcarbodiimide (EDC), dicyclohexyl carbodiimide (DCC), 1-hydroxybenzotriazole (HOBt), (benzotriazol-1-yloxy)-tris-(dimethylamino)-phosphonium hexafluorophosphate (BOP), bis-(2-oxo-3-oxazolidinyl)-phosphonic acid chloride (BOP-Cl), in a solvent such as tetrahidrofurane, dioxane, dimethylformamide, dichloromethane, acetonitrile.

Reduction of primary amides of general formula (Id) wherein R'=R"=H with borane-methyl sulphide complex in a solvent such as tetrahidrofurane, yields to the corresponding primary amine of general formula (If).

Moreover, primary amines of general formula (Ie) may be prepared by Curtius rearrangement of the acid derivatives of general formula (Ic) using an azide such as sodium azide, diphenylphosphoryl azide (DPPA), etc, in acidic media such as sulphuric acid or basic media such as triethylamine, in solvent such as toluene, chloroform, THF, etc. or in a protic solvent such as *tert*-butanol or benzyl alcohol to yield the *tert-*butylcarbonyl (BOC) or benzyloxycarbonyl (CBZ or Z) protected amine and subsequent deprotection as known in the art.

General compounds of formula (lh) may be obtained following the synthetic path shown in Figure 9.

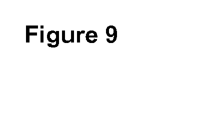

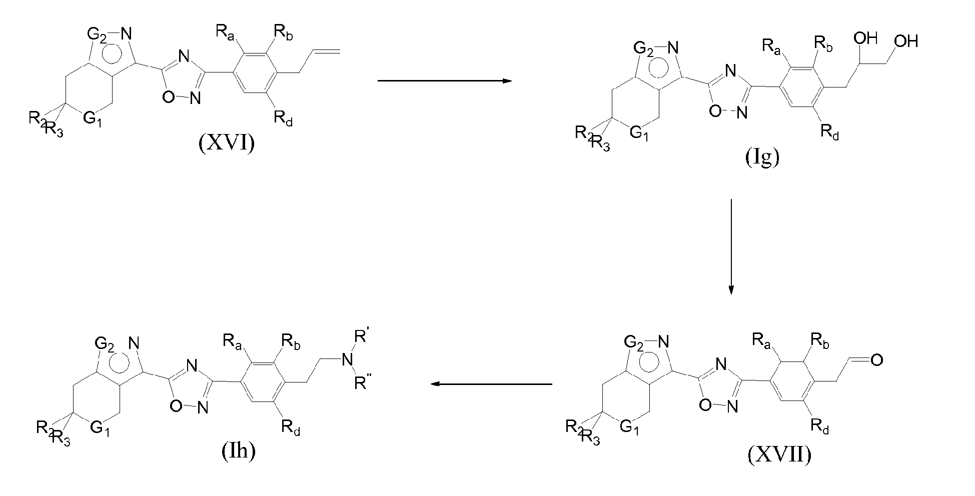

The compounds of general formula (lh) may be prepared by the reductive amination of the aldehyde derivatives of general formula (XVII) with the corresponding amine or aminoacid in acid media such as acetic acid, in a protic solvent such as methanol or ethanol and with a reductive agent such as sodium borohydride or sodium cyanoborohydride at a temperature from 0°C to the boiling point of the solvent.

Intermediates of formula (XVII) may be obtained by oxidation of diols of general formula (lg) with an oxidative reagent such as sodium periodate, sodium perchlorate, potassium periodate, etc. in a solvent such as methanol, ethanol, tetrahidrofurane, dioxane, ether, optionally with the presence of water.

Diol derivatives of general formula (lg) may be prepared by oxidation of the allyl derivatives of general formula (XVI) using an oxidative reagent such as sodium periodate, sodium perchlorate, potassium periodate, etc. in the presence of a catalyst such us sodium tetraoxide solid or a solution in water or tert-butanol, in a solvent such as methanol, ethanol, tetrahydrofurane, dioxane, ether, acetone, optionally with the presence of water.

Allyl derivatives of general formula (XVI) may be prepared by the condensation of the corresponding carboximidamide of formula (IIIc) with the corresponding acid formula (IIa) as described in figure 10.

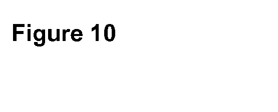

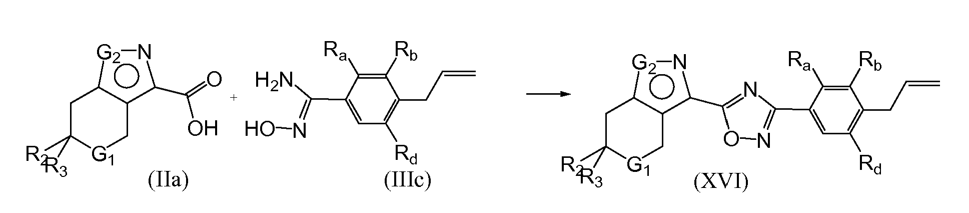

Intermediates of formula (IIIc) may be obtained by standard Stille reaction of the corresponding precursor (XIII) wherein X represents triflate, bromo or iodo, using an allylstannane and a catalyst such as tetrakis(triphenylphosphine)-palladium(0), palladium acetate, bis(triphenylphosphine)palladium(II) chloride or tris(dibenzylideneacetone)-dipalladium(0), in a solvent such dimethylformamide, acetonitrile, and subsequent reaction with hydroxylamine as described before.

General compounds of formula (li) may be obtained following the synthetic path shown in Figure 11.

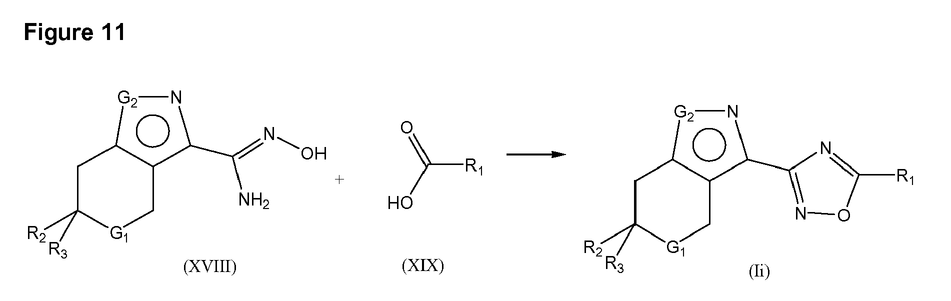

The compounds of general formula (li) may be prepared by the condensation of the tetrahidroindazole-3-carboximidamide or tetrahidroisoxazole-3-carboximidamide derivative of formula (XVIII) with the corresponding carboxylic acid derivatives of formula (XIX) following the same synthetic procedure described for the preparation of compounds of general formula (Ia).

Intermediates of general formula (XIX) are commercially available or may be obtained following the conventional synthetic methods already known in the art.

Compounds of general formula (XVIII) may be prepared following the synthetic scheme depicted in figure 12.

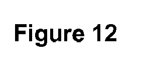

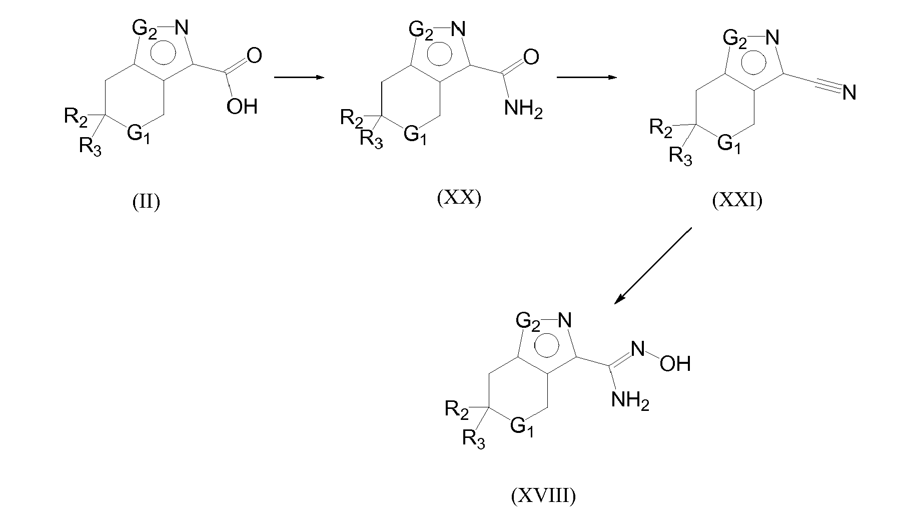

Intermediates of general formula (XX) may be prepared from intermediates of general formula (II) by reaction with a coupling agent such as N-(3-dimethylaminopropyl)-N'-ethyl-carbodiimide (EDC) and 1-hydroxybenzotriazole (HOBt) as a catalyst and a 32% aqueous ammonia in a solvent such as dimethylformamide at a temperature between 20 and the boiling point of the solvent in a standard reactor.

Intermediates of general formula (XXI) may be prepared from intermediates of general formula (XX) by reaction with phosphoryl trichloride in pyridine at a temperature between O°C and 25°C.

Intermediates of general formula (XVIII) may be prepared from compounds of general formula (XXI) following the same synthetic procedure described for the preparation of intermediates of general formula (IIIa)

The syntheses of the compounds of the invention are illustrated by the following Examples (1 to 102) including Preparation Examples (1 to 186) which do not limit the scope of the invention in any way.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1min |
| C | 0min | 20min | 4min |

### General Purification Method:

The solid was dissolved in DMSO/MeOH, injected into a Biotage C18 silica column (40M, 25M or 25S according to the crude amount) and eluted on the SP1® automated purification system from Biotage. The gradient used was H2O/Acetonitrile/MeOH (1:1) (0.1% v/v HCOONH4 both phases) from 0% to 100% acetonitrile/MeOH (1:1) in 80 column volumes. The appropriate fractions were collected and the organic solvent evaporated under reduced pressure or liofilized.

### GENERAL SYNTHETIC METHODS:

### General Method 1:

A mixture of the corresponding benzonitrile (39.2 mmol) in methanol (50ml), hydroxylamine hydrochloride (5.45g, 78.4mmol) and sodium bicarbonate (13.17g, 156.8 mmol) was stirred at 75°C for 6h. The mixture was filtered off and the filtrate evaporated to dryness to yield the title compound (75-100% yield) as a white solid.

### General Method 2:

A mixture of the corresponding acid derivative (1.13mmol), EDC (1.13mmol) and HOBt (1.13mmol) in DMF (5ml) was stirred at room temperature for 10 min. Then the corresponding benzimidamide (1.13mmol) was added and the mixture stirred at 140°C for 4h. The reaction mixture was poured over basic ice/water and the solid formed filtered and washed with water, dried in a vacuum oven to give the desired compound (yield=10-90%) as a white solid.

### General Method 3:

To a mixture of the corresponding methyl or ethyl esther (0.67mmol) in methanol or ethanol (3ml) respectively was added a 2M solution of aqueous NaOH (12mmol) and the reaction mixture stirred at 90°C overnight. The organic solvent was evaporated, water was added and the mixture extracted with diethyl ether. The pH of the aqueous layer was adjusted to 5-6 and the solid formed filtered and dried in the vacuum oven. The desired compound (60-95% yield) was obtained as a white solid.

### General Method 4:

A mixture of the corresponding tert-butyl esther (0.56mmol) in 4M HCl in dioxane (3.5ml) was stirred at r.t. overnight. The solvent was evaporated and the solid obtained washed with diisopropyl ether twice. The solid was dried in the vacuum oven to yield (70-95% yield) of the desired compound.

### General Method 5:

A mixture of the corresponding acid (0.46mmol), EDC (133mg, 0.69mmol), HOBt (94mg, 0.69mmol) and 32% solution of aqueous ammonia (85 µl, 0.69mmol) in DMF (6.5ml) was stirred overnight at room temperature. Then ethyl acetate and water were added and the organic layer separated, washed with 4% NaHCO3, water and brine and dried to give the title compound (5-77% yield) as a white solid.

### General Method 6:

A mixture of the corresponding acid derivative (0.77mmol), DPPA (189 µl, 0.88mmol) and triethylamine (235µl, 0.51mmol) in *tert*-butanol (4ml) was stirred at 100°C overnight. Ethyl acetate was added and the organic layer washed with 4% NaHCO3 and brine, dried and concentrated. The residue was redissolved in 4M HCl in dioxane (10ml) and the mixture stirred overnight at room temperature. The reaction mixture was concentrated and the residue purified according to General Purification Method. The solid obtained was redissolved in 4M HCl in dioxane and stirred for 2h at r.t. Then the solvent was concentrated and the product crystallized in diethyl ether. The title compound was obtained (5-40% yield) as hydrochloride salt.

### General Method 7:

To a solution of the corresponding amide (75mg, 0.18mmol) in tetrahidrofurane (4ml) was added BH₃.SMe₂ (107µl, 0.21mmol) drop wise. The reaction mixture was stirred overnight at 65°C. Solvent was concentrated and ethyl acetate was added. The organic layer was washed with 4% NaHCO3 and brine, dried and concentrated. The residue was purified according to General Purification Method. The solid obtained was redissolved in 5N HCl in dioxane and stirred for 2h at r.t. Then the solvent was concentrated and the product crystallized in diethyl ether. The title compound was obtained (20-65% yield) as hydrochloride salt.

### General Method 8:

To a solution of the corresponding aldheid (200mg, 0,46mmol) in methanol (10ml) was added the corresponding amine (44µl, 0,55) and acetic acid (236µl, 4,14mmol) and was stirred at room temperature for 30min. Then NaBH₃CN (15mg, 0.23mmol) was added and the reaction mixture stirred at r.t. overnight. Solvent was concentrated and the residue dissolved in ethyl acetate, washed with water and brine, dried over magnesium sulphate, filtered and concentrated. The crude obtained was purified according to the General Purification Method to give the desired compound (25-85% yield).

### PREPARATION EXAMPLES

### Preparation 1

### (Z)-N'-hydroxy-4-(hydroxymethyl)benzimidamide

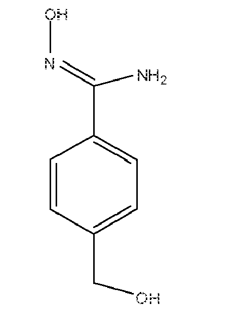

Obtained from 4-(hydroxymethyl)benzonitrile (100% yield) following the General Method 1.
LRMS: m/z 167 (M+1)⁺
Retention time: 0.68 min (Method B)

### Preparation 2

### (E)-N'-hydroxy-4-sulfamoylbenzimidamide

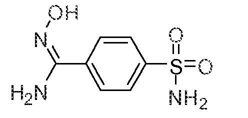

Obtained (100% yield) from 4-cyanobenzenesulfonamide following the General Method 1.
LRMS: m/z 216 (M+1)⁺
Retention time: 0.70 min (Method B)
¹H NMR (200 MHz, DMSO-D₆) δ ppm 5.9 (s, 2 H) 7.8 (s, 4 H).

### Preparation 3

### Ethyl 3-(4-(4-cyanophenyl)piperazin-1-yl)propanoate

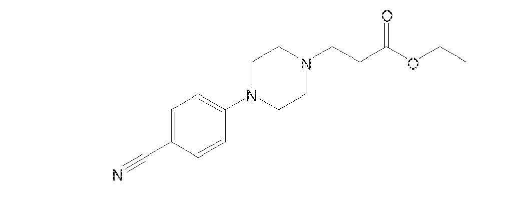

To a solution of 4-(piperazin-1-yl)benzonitrile (3g, 16.02mmol) in anhydrous acetonitrile (40ml) under nitrogen atmosphere was added Yb(OTf)₃ (0.2g, 0.32mmol) and then ethyl acrylate (3.49ml, 32.04mmol). The reaction mixture was stirred overnight at room temperature. The catalyst was filtered off and the filtrate concentrated and chromatographed to provide the title compound (86% yield) of a solid.
LRMS: m/z 288 (M+1)⁺
Retention time: 3.60 min (Method B)
¹H NMR (200 MHz, CHLOROFORM-D) δ ppm 1.3 (t, *J*=7.2 Hz, 3 H) 2.6 (m, 6 H) 2.8 (t, *J*=7.0 Hz, 2 H) 3.3 (m, 4 H) 4.2 (q, *J*=7.0 Hz, 2 H) 6.8 (d, *J*=8.6 Hz, 2 H) 7.5 (d, *J*=8.6 Hz, 2 H)

### Preparation 4

### (Z)-Ethyl 3-(4-(4-(N'-hydroxycarbamimidoyl)phenyl)piperazin-1-yl)propanoate

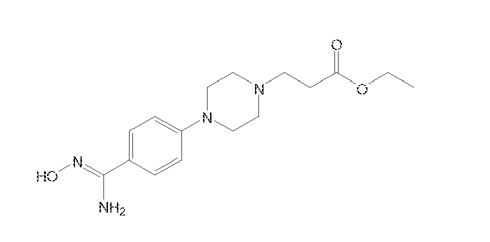

Obtained (37% yield) from Preparation 3 following the General Method 1.
LRMS: m/z 321 (M+1)⁺
Retention time: 0.58 min (Method B)
¹H NMR (200 MHz, DMSO-D6) δ ppm 1.2 (t, *J*=7.2 Hz, 3 H) 2.5 (m, 8 H) 2.6 (t,
*J*=6.4 Hz, 2 H) 3.1 (m, 2 H) 4.1 (q, *J*=7.3 Hz, 2 H) 5.6 (s, 2 H) 6.9 (d, *J*=9.0 Hz, 2 H) 7.5 (d, *J*=9.0 Hz, 2 H) 8.3 (s, 1 H).

### Preparation 5

### 4-Cyano-2,6-dimethylphenyl trifluoromethanesulfonate

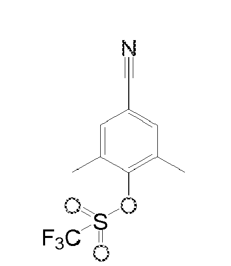

To a suspension of 4-hydroxy-3,5-dimethylbenzonitrile (5.10g, 34.65mmol) in DCM (100ml) was added triethylamine (7.25ml, 52.02mmol). To the solution obtained was added 1,1,1-trifluoro-N-phenyl-N-(trifluoromethylsulfonyl)methansulfonamide (14.9g, 41.7mmol) and the mixture stirred overnight at room temperature. More DCM was added and then washed with 0.5N NaOH, water and brine. The organic layer was dried over magnesium sulphate and concentrated to yield 11.8g of the desired compound as a solid (yield=100%).
LRMS: no signal
Retention time: 6.90 min (Method B)

### Preparation 6

### (E)-Methyl 3-(4-cyano-2,6-dimethylphenyl)acrylate

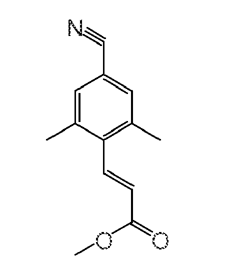

To a mixture of Preparation 5 (6.07g, 21.74mmol), methyl acrylate (5.87ml, 65.18mmol), 1,3-bis-(diphenylphosphino)propane (0.45g, 1.09mmol) and triethylamine (6.06ml, 43.48mmol) in DMF (30ml), was added palladium acetate (0.25g, 1.11 mmol) under nitrogen atmosphere. The reaction mixture was stirred overnight at 110°C. After cooling to room temperature, the mixture was poured over water and extracted with diethyl ether twice. The combined organic layer was washed with water and brine, dried over magnesium sulphate and concentrated. A brown oil was obtained (68% yield) as the desired compound.
LRMS: no signal
Retention time: 6.13 min (Method B)

### Preparation 7

### (E)-Methyl 3-(4-((Z)-N'-hydroxycarbamimidoyl)-2,6-dimethylphenyl)acrylate

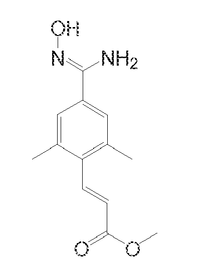

Obtained (75% yield) from (E)-methyl 3-(4-cyano2,6-dimethylphenyl)acrylate following the General Method 1.
LRMS: m/z 249 (M+1)⁺
Retention time: 3.88 min (Method B)

### Preparation 8

### (E)-Methyl 3-(4-(N'-hydroxycarbamimidoyl)-2,6-dimethylphenyl)propanoate

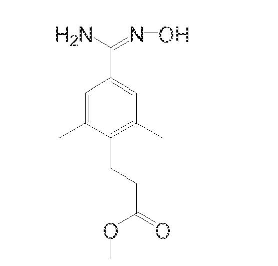

Preparation 7 (1.1 g, 4.43mmol) was dissolved in methanol (35ml) and sodium acetate (0.55g, 6.70mmol) was added. Finally palladium chloride (0.16g, 0.90mmol) was added and the mixture hydrogenated at 15 psi for 5h. The catalyst was filtered off and the filtrate concentrated. The residue was redissolved in DCM and washed with water. The organic layer was dried over magnesium sulphate and concentrated to yield an oil (82% yield) as the desired compound.
LRMS: m/z 251 (M+1)⁺
Retention time: 3.64 min (Method B)

### Preparation 9

### (E)-Tert-butyl 3-(4-cyano-2,6-dimethylphenyl)acrylate

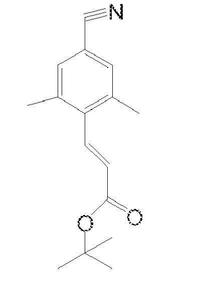

Obtained (64%) from Preparation 5 and tert-butyl acrylate following the experimental procedure describe for Preparation 6.
LRMS: m/z 258 (M+1)⁺
Retention time: 7.18 min (Method B)

### Preparation 10

### (E)-tert--Butyl 3-(4-((E)-N'-hydroxycarbamimidoyl)-2,6-dimethylphenyl)acrylate

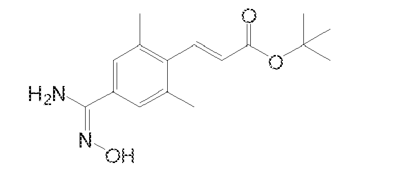

Obtained (76%) from Preparation 9 following the General Method 1.
LRMS: m/z 291 (M+1)⁺
Retention time: 4.89 min (Method B)

### Preparation 11

### (E)-tert-Butyl 3-(4-(N'-hydroxycarbamimidoyl)-2,6-dimethylphenyl)propanoate

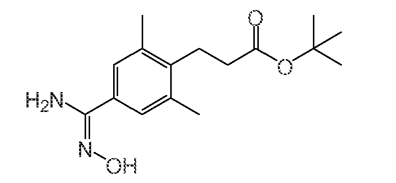

Obtained (77%) from Preparation 10 following the experimental procedure described for Preparation 8.
LRMS: m/z 293 (M+1)⁺
Retention time: 4.66 min (Method B)

### Preparation 12

### Ethyl 2-(4,4-dimethyl-2-oxocyclohexyl)-2-oxoacetate

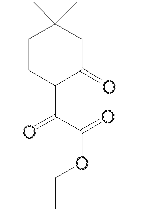

To ethanol (500ml) was added slowly sodium (8.47g, 0.37mol), then 3,3-dimethylcyclohexanone (15.5g, 0.12mol) in ethanol (200ml) was added and the mixture stirred at room temperature for 15min. Finally diethyl oxalate (16.65ml, 0.12mol) in ethanol (100ml) was added and the mixture stirred overnight at r.t. The solvent was concentrated and the crude redissolved in water and dichloromethane. A 5N solution of HCl was added until pH acid and the layers separated. The aqueous layer was extracted the more DCM and the combined organic layer was washed with brine, dried over sodium sulphate and concentrated. An oil (19.16g) was obtained which contained the desired compound and the desired compound in the acid form. The mixture was used for the next reaction without further purification. Yield=69%.
LRMS: m/z 227 (M+1)⁺
Retention time: 6.50min (Method B)

### Preparation 13

### Ethyl 1-ethyl-6,6-dimethyl-4,5,6,7-tetrahydro-1H-indazole-3-carboxylate

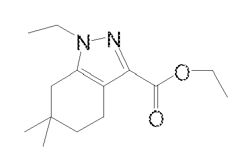

To a suspension of oxalic acid salt of ethylhydrazine (17.8g,d 0.12mol) in ethanol (200ml) was added triethylamine (18ml, 0.13mol). A solution was obtained and poured over the crude product of Preparation 12 in ethanol (300ml). The reaction mixture was stirred at r.t. for 4h and then the solvent concentrated. The residue was redissolved in ethyl acetate/water and the layers separated. The aqueous layer was extracted the more ethyl acetate and the combined organic layer was washed with brine, dried over sodium sulphate and concentrated. An oil (20.5g) was obtained which contained a mixture of the desired compound and the desired compound in the acid form. The mixture was used for the next reaction without further purification. Yield=82%.
LRMS: m/z 251 (M+1)⁺
Retention time: 6.59min (Method B)

### Preparation 14

### 1-Ethyl-6,6-dimethyl-4,5,6,7-tetrahydro-1H-indazole-3-carboxylic acid

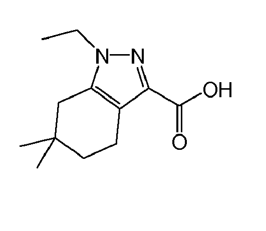

Obtained (86% yield) from Preparation 13 following the General Method 3.
LRMS: m/z 223 (M+1)⁺
Retention time: 5.66min (Method B)
¹H NMR (200 MHz, DMSO-D6) δ ppm 1.0 (s, 6 H) 1.2 (t, *J*=7.2 Hz, 2 H) 1.4 (t, *J*=6.2 Hz, 2 H) 2.3 (s, 2 H) 2.6 (t, *J*=6.2 Hz, 2 H) 4.0 (q, *J*=7.0 Hz, 2 H).

### Preparation 15

### Ethyl 6,6-dimethyl-4,5,6,7-tetrahydro-2H-indazole-3-carboxylate

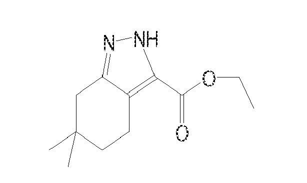

Obtained (90% yield) from Preparation 12 and hydrazine hydrate following the experimental procedure described for Preparation 13.
LRMS: m/z 223 (M+1)⁺
Retention time: 6.13 min (Method B)

### Preparation 16

### 6,6-dimethyl-4,5,6,7-tetrahydro-2H-indazole-3-carboxylic acid

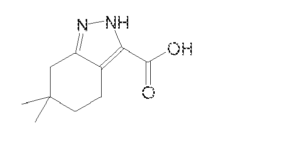

Obtained (95% yield) from Preparation 15 following the General Method 3.
LRMS: m/z 195 (M+1)⁺
Retention time: 5.23 min (Method B)

### Preparation 17

### Ethyl 1,6,6-trimethyl-4,5,6,7-tetrahydro-1H-indazole-3-carboxylate

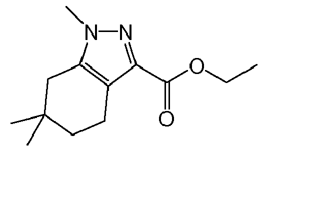

Obtained (71% yield) from Preparation 12 and methyl hydrazine following the experimental procedure described for Preparation 13.
LRMS: m/z 237 (M+1)⁺
Retention time: 6.39 min (Method B)

### Preparation 18

### 1,6,6-trimethyl-4,5,6,7-tetrahydro-1H-indazole-3-carboxylic acid

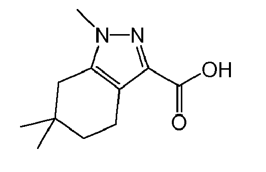

Obtained (95% yield) from Preparation 17 following the General Method 3.
LRMS: m/z 209 (M+1)⁺
Retention time: 5.35 min (Method B)

### Preparation 19

### Ethyl 4-[(Z)-amino(hydroxyimino)methyl]benzoate

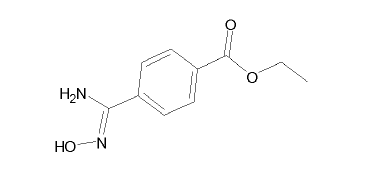

Obtained (80% yield) from ethyl 4-cyanobenzoate following the General Method 1.
LRMS: m/z 209 (M+1)⁺
Retention time: 3.65 min (Method B)
¹H NMR (300 MHz, DMSO-*d*₆) d ppm 1.37 (t, *J*=7.00 Hz, 3 H) 4.36 (q, *J*=6.96
Hz, 2 H) 5.99 (s, 2 H) 7.86 (d, 2 H) 7.99 (d, *J*=8.51 Hz, 2 H) 9.95 (s, 1 H)

### Preparation 20

### Ethyl 4-[5-(1-ethyl-6,6-dimethyl-4,5,6,7-tetrahydro-1H-indazol-3-yl)-1,2,4-oxadiazol-3-yl]benzoate

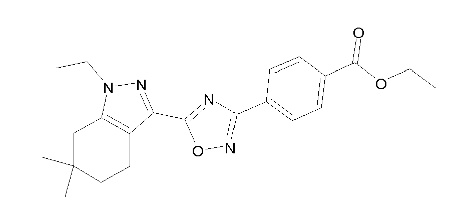

Obtained (45% yield) from Preparation 19 and Preparation 14 following the General Method 2.
LRMS: m/z 395 (M+1)⁺
Retention time: 7.85 min (Method B)
¹H NMR (300 MHz, CHLOROFORM-*d*) d ppm 1.07 (s, 6 H) 1.44 (m, 6 H) 1.63 (t, *J*=6.32 Hz, 2 H) 2.42 (s, 2 H) 2.91 (t, *J*=6.18 Hz, 2 H) 4.17 (t, *J*=7.14 Hz, 2 H) 4.40 (m, 2 H) 8.16 (d, *J*=8.24 Hz, 2 H) 8.27 (m, 2 H)

### Preparation 21

### tert-Butyl 4-{4-[5-(1-ethyl-6,6-dimethyl-4,5,6,7-tetrahydro-1H-indazol-3-yl)-1,2,4-oxadiazol-3-yl]phenyl}piperazine-1-carboxylate

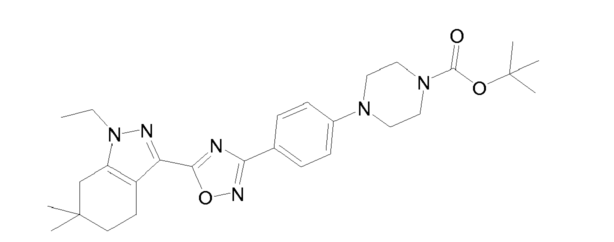

Obtained (5% yield) from Preparation 22 and Preparation 14 following the General Method 2.
LRMS: m/z 507 (M+1)⁺
Retention time: 7.88 min (Method B)
¹H NMR (300 MHz, CDCl₃) δ ppm: 1.07 (s, 6H), 1.44-1.47 (t, 3H), 1.49 (s, 9H),1.61-1.66 (m, 2H), 2.40 (s, 2H), 2.88-2.92 (m, 2H), 3.58-3.62 (m, 4H), 3.26-3.29 (m, 4H), 4.14-4.21 (q, 2H), 6.95-6.98 (d, 2H), 8.09-8.12 (m, 2H).

### Preparation 22

### tert-butyl 4-{4-[(Z)-amino(hydroxyimino)methyl]phenyl}piperazine-1-carboxylate

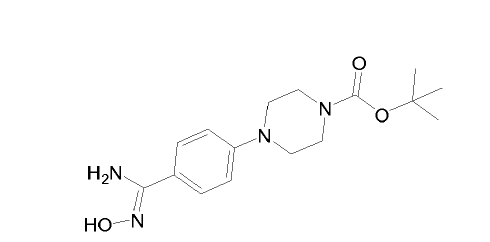

Obtained (73% yield) from Preparation 23 following the General Method 1.
LRMS: m/z 321 (M+1)⁺
Retention time: 4.23 min (Method B)
¹H NMR (300 MHz, CDCl₃) δ ppm: 1.50 (s, 9H), 3.11-3.27 (m, 4H), 3.52-3.64 (m, 4H), 4.76-4.82 (m, 2H), 6.89-6.92 (m, 2H), 7.52-7.55 (m, 2H).

### Preparation 23

### tert-butyl 4-(4-cyanophenyl)piperazine-1-carboxylate

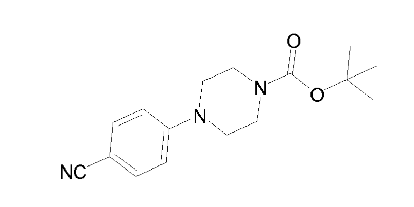

4-(piperazin-1-yl)benzonitrile (0.7g, 3.74mmol) was dissolved in a mixture of dioxane/water (15ml/7ml), then 1 N solution of NaOH was added (5.2ml) and finally BOC₂O (0.82g, 3.78mmol). The reaction mixture was stirred at r.t. for 2h. The solvent was concentrated and the residue redissolved in water and the pH adjusted to 7 by adding 2N HCl. The aqueous layer was extracted with chloroform and the organic layer washed with brine, dried over magnesium sulphate and evaporated under reduced pressure to give 1 g of the desired compound (yield=93%) as a white solid.
LRMS: m/z 288 (M+1)⁺
Retention time: 6.33 min (Method B)
¹H NMR (300 MHz, CDCl₃) δ ppm: 1.49 (s, 9H), 3.33-3.35 (m, 2H), 3.57-3.59 (m, 2H), 6.84-6.87 (m, 2H), 7.50-7.53 (m, 2H).

### Preparation 24

### Ethyl {4-[5-(1-ethyl-6,6-dimethyl-4,5,6,7-tetrahydro-1H-indazol-3-yl)-1,2,4-oxadiazol-3-yl]phenyl}acetate

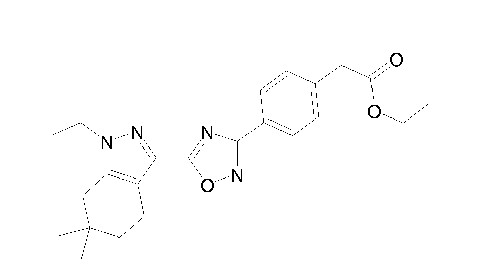

Obtained (40% yield) from Preparation 25 and Preparation 14 following the General Method 2.
LRMS: m/z 409 (M+1)⁺
Retention time: 7.60 min (Method B)
¹H NMR (300 MHz, CHLOROFORM-*d*) d ppm 1.08 (s, 6 H) 1.25 (m, 3 H) 1.45 (m, 3 H) 1.6 (m, 2 H) 2.41 (s, 2 H) 2.91 (t, *J*=6.32 Hz, 2 H) 3.67 (m, 2 H) 4.13 - 4.24 (m, 4 H) 7.42 (d, *J*=8.24 Hz, 2 H) 8.19 (d, *J*=8.24 Hz, 2 H)

### Preparation 25

### Ethyl {4-[(Z)-amino(hydroxyimino)methyl]phenyl}acetate

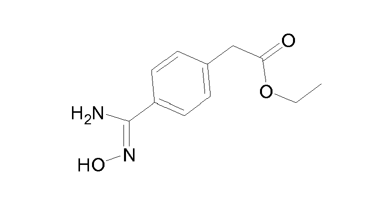

Obtained (95% yield) from Preparation 26 following the General Method 1.
LRMS: m/z 223 (M+1)⁺
Retention time: 3.82 min (Method B)
¹H NMR (300 MHz, CHLOROFORM-*d*) d ppm 1.26 (t, 3 H) 3.64 (s, 2 H) 4.16 (q, 2 H) 4.90 (s, 2 H) 7.33 (d, *J*=8.51 Hz, 2 H) 7.60 (m, 2 H)

### Preparation 26

### Ethyl (4-cyanophenyl)acetate

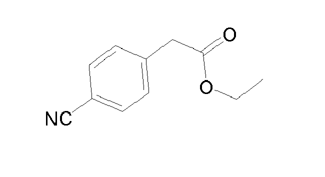

2-(4-cyanophenyl)acetic acid (1 g, 6.21 mmol) was dissolved in 1.25M solution of HCl in ethanol (11ml) and the mixture heated to reflux for 3h. After cooling down to room temperature the solid formed was filtered off and dried in the vacuum oven. Yield=89%.
LRMS: m/z 207 (M+17)⁺
Retention time: 5.33 min (Method B)
¹H NMR (300 MHz, CHLOROFORM-*d*) d ppm 1.26 (t, 3 H) 3.64 (s, 2 H) 4.16 (q, 2 H) 4.90 (s, 2 H) 7.33 (d, *J*=8.51 Hz, 2 H) 7.60 (m, 2 H)

### Preparation 27

### 4-[5-(1-Ethyl-6,6-dimethyl-4,5,6,7-tetrahydro-1H-indazol-3-yi)-1,2,4-oxadiazol-3-yl]benzaldehyde

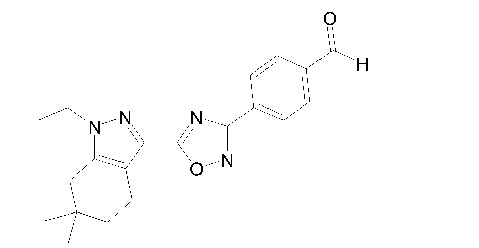

To a solution of oxalyl chloride (0.73ml, 8.35mmol) in DCM (20ml) under argon at - 60°C , DMSO (0.89g, 11.39mmol) was slowly added keeping the temperature at -60°C and the mixture stirred at this temperature for 15min. A suspension of the title compound of Preparation 28 (1,3g, 3.8mmol) in 10ml of DCM was slowly added to this mixture. Finally, diisopropylethylamine was added (4.40ml, 25.3mmol) and the mixture stirred at -60°C for 1h and at room temperature overnight. Solvent was removed and the residue was solved in ethyl acetate and washed with a 4% solution of NaHCO₃. The organic layer was dried, solvent was removed in vaccuo and the crude was purified according to General Purification Method to yield the title compound as a solid (yield=98%).
LRMS: m/z 351 (M+1)⁺
Retention time: 7.45 min (Method B)

### Preparation 28

### {4-[5-(1-Ethyl-6,6-dimethyl-4,5,6,7-tetrahydro-1H-indazol-3-yl)-1,2,4-oxadiazol-3-yl]phenyl}methanol

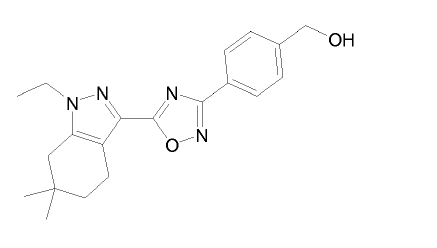

Obtained (63% yield) from Preparation 1 and Preparation 14 following the General Method 2.
LRMS: m/z 353 (M+1)⁺
Retention time: 7.67 min (Method B)
¹H NMR (300 MHz, CHLOROFORM-*d*) d ppm 1.08 (s, 6 H) 1.46 (t, *J*=7.28 Hz, 3 H) 1.62 (t, *J*=6.45 Hz, 2 H) 2.41 (s, 2 H) 2.92 (t, *J*=6.32 Hz, 2 H) 4.19 (q, *J*=7.42 Hz, 2 H) 4.79 (s, 2 H) 7.50 (d, *J*=8.52 Hz, 2 H) 8.22 (d, *J*=8.24 Hz, 2 H)

### Preparation 29

### tert-butyl 4-({4-[5-(1-ethyl-6,6-dimethyl-4,5,6,7-tetrahydro-1H-indazol-3-yl)-1,2,4-oxadiazol-3-yl]phenyl}amino)piperidine-1-carboxylate

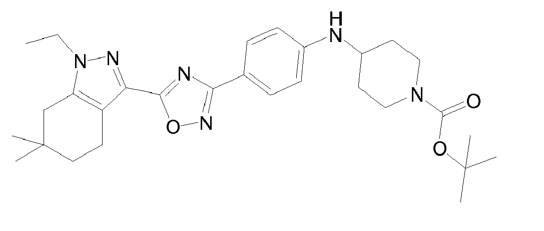

Obtained (10% yield) from Preparation 30 and Preparation 14 following the General Method 2.
LRMS: m/z 521 (M+1)⁺
Retention time: 7.80 min (Method B)
1 H NMR (300 MHz, CHLOROFORM-*d*) d ppm 1.07 (s, 6 H) 1.45 (m, 3 H) 1.48 (s, 9 H) 1.62 (m, 4 H) 2.08 (m, 2 H) 2.40 (s, 2 H) 2.85 - 3.02 (m, 4 H) 3.52 (m, 1 H) 4.07 (m, 2 H) 4.18 (q, *J*=7.42 Hz, 2 H) 6.65 (d, *J*=8.51 Hz, 2 H) 8.03 (d, *J*=8.51 Hz, 2 H)

### Preparation 30

### tert-Butyl 4-({4-[(Z)-amino(hydroxyimino)methyl]phenyl}amino)piperidine-1-carboxylate

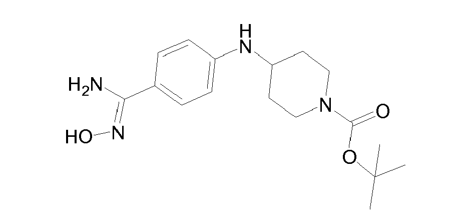

Obtained (79% yield) from Preparation 31 following the General Method 1.
LRMS: m/z 335 (M+1)⁺
Retention time: 4.28 min (Method B)
¹H NMR (300 MHz, CHLOROFORM-*d*) d ppm 1.25 (m, 2 H) 1.47 (s, 9 H) 2.04 (d, *J*=12.64 Hz, 2 H) 2.92 (m, 2 H) 3.46 (m, 1 H) 3.73 (m, 1 H) 4.07 (m, 2 H) 4.79 (s, 2 H) 6.59 (d, *J*=8.79 Hz, 2 H) 7.45 (d, *J*=8.79 Hz, 2 H)

### Preparation 31

### tert-butyl 4-[(4-cyanophenyl)amino]piperidine-1-carboxylate

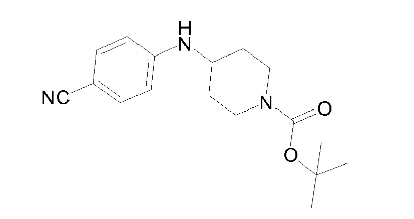

To a mixture of 4-aminobenzonitrile (0.5g, 4.23mmol) and tert-butyl 4-oxocyclohexanecarboxylate (1.26g, 6.35mmol) in THF (5ml) at 0°C was added acetic acid (0.5ml, 8.5mmol) and sodium triacetoxyborohydride (1.35g, 6.35mmol). The mixture was stirred at this temperature for 15min and at room temperature for 3h. Ethyl acetate and 5% solution of NaHCO₃ were added and the organic layer separated, washed with water, brine and dried over magnesium sulphate. The solvent was concentrated and the residue purified by column chromatography with a mixture of hexane/ethyl acetate (from 5/1 to 1/1) to give the desired compound (yield=47%).
LRMS: m/z 302 (M+1)⁺
Retention time: 6.33 min (Method B)
¹H NMR (300 MHz, CHLOROFORM-d) d ppm 1.37 (m, 2 H) 1.47 (s, 9 H) 2.03 (m, 2 H) 2.93 (t, J=11.95 Hz, 2 H) 3.40 - 3.54 (m, 1 H) 4.04 - 4.13 (m, 3 H) 6.56 (d, J=9.06 Hz, 2 H) 7.43 (d, J=8.79 Hz, 2 H)

### Preparation 32

### N'-hydroxy-6-methoxypyridine-3-carboximidamide

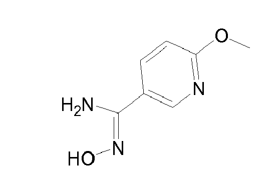

Obtained (100% yield) from 6-methoxynicotinonitrile following the General Method 1.
LRMS: m/z 168 (M+1)⁺
Retention time: 0.65 min (Method B)
¹H NMR (300 MHz, CHLOROFORM-*d*) d ppm 3.94 (s, 3 H) 6.75 (d, *J*=8.79 Hz, 1 H) 7.82 (dd, *J*=8.93, 2.33 Hz, 1 H) 8.38 (d, *J*=2.47 Hz, 1 H)

### Preparation 33

### 4-[5-(1-benzyl-6,6-dimethyl-4,5,6,7-tetrahydro-1H-indazol-3-yl)-1,2,4-oxadiazol-3-yl]benzoic acid

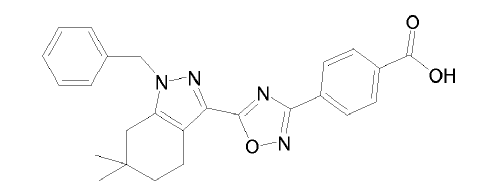

Obtained (95% yield) from Preparation 34 following the General Method 3.
LRMS: m/z 429 (M+1)⁺
Retention time: 7.57 min (Method B)
¹H NMR (300 MHz, CHLOROFORM-*d*) d ppm 1.00 (s, 6 H) 1.60 (s, 2 H) 2.27 (s, 2 H) 2.94 (s, 2 H) 5.40 (s, 2 H) 7.15 (d, 1 H) 7.23 - 7.37 (m, 4 H) 8.23 (d,
*J*=6.98 Hz, 2 H) 8.34 (d, *J*=6.35 Hz, 2 H)

### Preparation 34

### ethyl 4-[5-(1-benzyl-6,6-dimethyl-4,5,6,7-tetrahydro-1H-indazol-3-yl)-1,2,4-oxadiazol-3-yl]benzoate

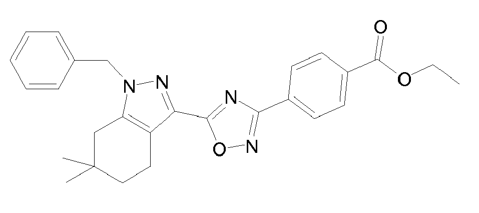

Obtained (37% yield) from Preparation 19 and Preparation 35 following the General Method 2.
LRMS: m/z 457 (M+1)⁺
Retention time: 7.98 min (Method B)
¹H NMR (300 MHz, CHLOROFORM-*d*) d ppm 1.00 (s, 6 H) 1.43 (t, *J*=7.14 Hz, 3 H) 1.60 (m, 2 H) 2.27 (s, 2 H) 2.93 (t, *J*=6.45 Hz, 2 H) 4.43 (q, *J*=7.14 Hz, 2 H) 5.40 (s, 2 H) 7.16 (m, 2 H) 7.34 (m, 3 H) 8.17 (m, 2 H) 8.31 (m, 2 H)

### Preparation 35

### 1-benzyl-6,6-dimethyl-4,5,6,7-tetrahydro-1H-indazole-3-carboxylic acid

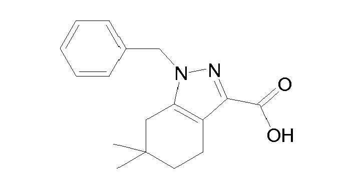

Obtained (75% yield) from Preparation 36 following the General Method 3.
LRMS: m/z 285 (M+1)⁺
Retention time: 6.37 min (Method B)
¹H NMR (300 MHz, CHLOROFORM-*d*) d ppm 0.96 (s, 6 H) 1.50 (t, *J*=6.45 Hz, 2 H) 2.21 (s, 2 H) 2.78 (t, *J*=6.32 Hz, 2 H) 5.29 (s, 2 H) 7.09 (d, *J*=6.87 Hz, 2 H) 7.29 - 7.34 (m, 3 H)

### Preparation 36

### ethyl 1-benzyl-6,6-dimethyl-4,5,6,7-tetrahydro-1H-indazole-3-carboxylate

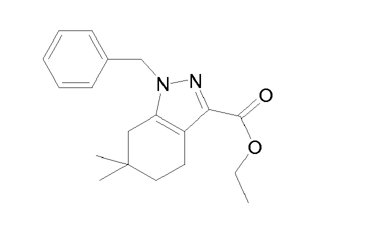

Obtained (32% yield) from Preparation 12 and benzylhydrazine following the experimental procedure described for Preparation 13.
LRMS: m/z 313 (M+1)⁺
Retention time: 7.10 min (Method B)
¹H NMR (300 MHz, CHLOROFORM-*d*) d ppm 0.93 (s, 6 H) 1.39 (t, *J*=7.00 Hz, 3 H) 1.48 (t, *J*=6.45 Hz, 2 H) 2.16 (s, 2 H) 2.75 (t, *J*=6.32 Hz, 2 H) 4.40 (q, *J*=7.14 Hz, 2 H) 5.30 (s, 2 H) 7.05 (m, 2 H) 7.27 (m, 3 H)

### Preparation 37

### 1-tert-butyl-6,6-dimethyl-4,5,6,7-tetrahydro-1H-indazole-3-carboxylic acid

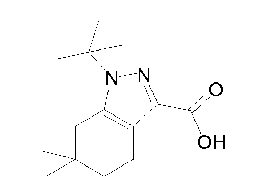

Obtained (65% yield) from Preparation 38 following the General Method 3.
LRMS: m/z 251 (M+1)⁺
Retention time: 7.12 min (Method B)
HPLC/EM 9 min: tr 6.32, M+251
¹H NMR (300 MHz, CHLOROFORM-*d*) d ppm 1.02 (s, 6 H) 1.52 (t, *J*=6.59 Hz, 2 H) 1.63 (s, 9 H) 2.59 (s, 2 H) 2.79 (t, *J*=6.45 Hz, 2 H)

### Preparation 38

### Ethyl 1-tert-butyl-6,6-dimethyl-4,5,6,7-tetrahydro-1H-indazole-3-carboxylate

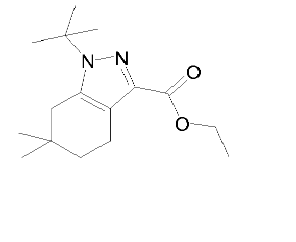

Obtained (24% yield) from Preparation 12 and tert-butylhidrazine following the experimental procedure described for Preparation 13.
LRMS: m/z 279 (M+1)⁺
Retention time: 7.12 min (Method B)
¹H NMR (300 MHz, CHLOROFORM-*d*) d ppm 1.01 (s, 6 H) 1.39 (t, *J*=7.14 Hz, 3 H) 1.50 (t, *J*=6.59 Hz, 2 H) 1.63 (s, 9 H) 2.58 (s, 2 H) 2.76 (t, *J*=6.18 Hz, 2 H) 4.36 (m, 2 H)

### Preparation 39

### 6,6-Dimethyl-1-phenyl-4,5,6,7-tetrahydro-1H-indazole-3-carboxylic acid

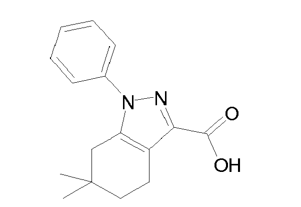

Obtained (70% yield) from Preparation 40 following the General Method 3.
LRMS: m/z 271 (M+1)⁺
Retention time: 6.37 min (Method B)
¹H NMR (300 MHz, CHLOROFORM-*d*) d ppm 1.01 (s, 6 H) 1.61 (t, *J*=6.59 Hz, 2 H) 2.49 (s, 2 H) 2.87 (t, *J*=6.45 Hz, 2 H) 7.38 - 7.53 (m, 5 H)

### Preparation 40

### Ethyl 6,6-dimethyl-1-phenyl-4,5,6,7-tetrahydro-1H-indazole-3-carboxylate

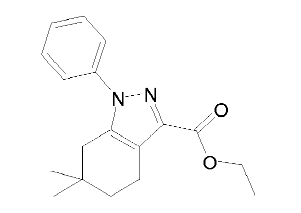

Obtained (15% yield) from Preparation 12 and phenylhidrazine following the experimental procedure described for Preparation 13.
LRMS: m/z 299 (M+1)⁺
Retention time: 7.13 min (Method B)
¹H NMR (300 MHz, CHLOROFORM-*d*) d ppm 1.00 (s, 6 H) 1.42 (m, 3 H) 1.60 (m, 2 H) 2.46 (s, 2 H) 2.84 (t, *J*=5.91 Hz, 2 H) 4.43 (m, 2 H) 7.34 - 7.55 (m, 5 H)

### Preparation 41

### 6,6-Dimethyl-1-(2,2,2-trifluoroethyl)-4,5,6,7-tetrahydro-1H-indazole-3-carboxylic acid

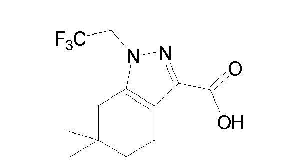

Obtained (95% yield) from Preparation 42 following the General Method 3.
LRMS: m/z 277 (M+1)⁺
Retention time: 5.92 min (Method B)
¹H NMR (300 MHz, CHLOROFORM-*d*) d ppm 1.05 (s, 6 H) 1.56 (t, *J*=6.45 Hz, 2 H) 2.37 (s, 2 H) 2.78 (t, *J*=5.91 Hz, 2 H) 4.66 (q, *J*=8.33 Hz, 2 H)

### Preparation 42

### Ethyl 6,6-dimethyl-1-(2,2,2-trifluoroethyl)-4,5,6,7-tetrahydro-1H-indazole-3-carboxylate

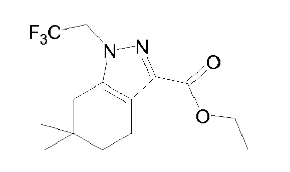

Obtained (28% yield) from Preparation 12 and (2,2,2-trifluoroethyl)hydrazine following the experimental procedure described for Preparation 13.
LRMS: m/z 305 (M+1)⁺
Retention time: 6.72 min (Method B)
¹H NMR (300 MHz, CHLOROFORM-*d*) d ppm 1.08 (s, 6 H) 1.32 (m, 3 H) 1.61 (t, *J*=6.45 Hz, 2 H) 2.42 (s, 2 H) 2.82 (t, *J*=6.45 Hz, 2 H) 4.18 (m, 2 H) 4.72 (q, *J*=8.24 Hz, 2 H)

### Preparation 43

### 4-[5-(1-tert-Butyl-6,6-dimethyl-4,5,6,7-tetrahydro-1H-indazol-3-yl)-1,2,4-oxadiazol-3-yl]benzoic acid

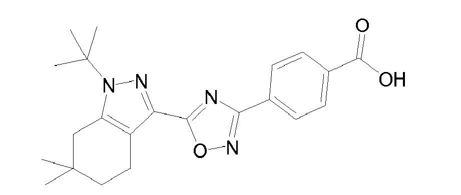

Obtained (100% yield) from Preparation 44 following the General Method 3.
LRMS: m/z 395 (M+1)⁺
Retention time: 7.70 min (Method B)

### Preparation 44

### Ethyl 4-[5-(1-tert-butyl-6,6-dimethyl-4,5,6,7-tetrahydro-1H-indazol-3-yl)-1 ,2,4-oxadiazol-3-yl]benzoate

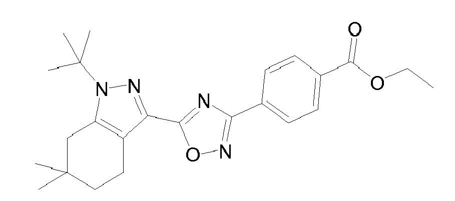

Obtained (14% yield) from Preparation 19 and Preparation 37 following the General Method 2.
LRMS: m/z 423 (M+1)⁺
Retention time: 8.11 min (Method B)
¹H NMR (300 MHz, CHLOROFORM-*d*) d ppm 1.11 (s, 6 H) 1.48 (t, *J*=7.14 Hz, 3 H) 1.66 (t, *J*=6.45 Hz, 2 H) 1.75 (s, 9 H) 2.71 (s, 2 H) 2.99 (t, *J*=6.45 Hz, 2 H) 4.47 (q, *J*=7.14 Hz, 2 H) 8.22 (m, 2 H) 8.33 (d, *J*=8.24 Hz, 2 H)

### Preparation 45

### 4-[5-(6,6-Dimethyl-1-phenyl-4,5,6,7-tetrahydro-1H-indazol-3-yl)-1,2,4-oxadiazol-3-yl]benzoic acid

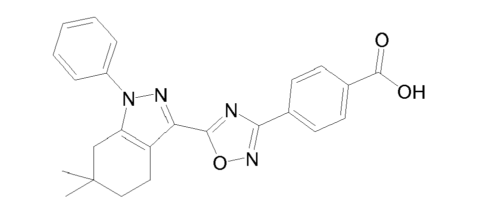

Obtained (100% yield) from Preparation 46 following the General Method 3.
LRMS: m/z 415 (M+1)⁺
Retention time: 7.63 min (Method B)

### Preparation 46

### Ethyl 4-[5-(6,6-dimethyl-1-phenyl-4,5,6,7-tetrahydro-1H-indazol-3-yl)-1,2,4-oxadiazol-3-yl]benzoate

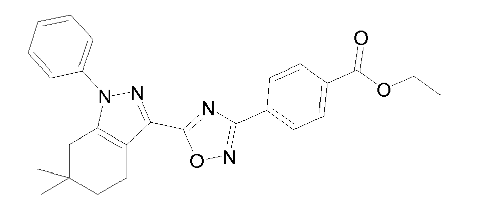

Obtained (24% yield) from Preparation 19 and Preparation 39 following the General Method 2.
LRMS: m/z 443 (M+1)⁺
Retention time: 8.08 min (Method B)
¹H NMR (300 MHz, CHLOROFORM-*d*) d ppm 1.05 (s, 6 H) 1.43 (t, *J*=6.45 Hz, 3 H) 1.69 (s, 2 H) 2.55 (s, 2 H) 3.01 (s, 2 H) 4.42 (m, 2 H) 7.39 - 7.61 (m, 5 H) 8.17 (d, *J*=7.42 Hz, 2 H) 8.30 (m, 2 H)

### Preparation 47

### 1-Ethyl-3-[3-(5-ethyl-6-methoxy-2-methylpyridin-3-yl)-1,2,4-oxadiazol-5-yl]-6,6-dimethyl-4,5,6,7-tetrahydro-1H-indazole

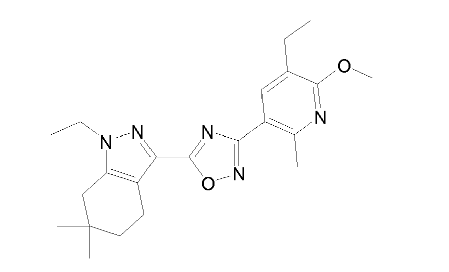

Obtained (21% yield) from Preparation 48 and Preparation 14 following the General Method 2.
LRMS: m/z 396 (M+1)⁺
Retention time: 8.15 min (Method B)

### Preparation 48

### 5-Ethyl-N'-hydroxy-6-methoxy-2-methylpyridine-3-carboximidamide

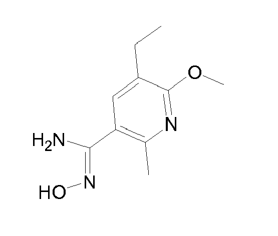

Obtained (32% yield) from Preparation 49 following the General Method 1.
LRMS: m/z 210 (M+1)⁺
Retention time: 4.2 min (Method B)

### Preparation 49

### 5-Ethyl-6-methoxy-2-methylnicotinonitrile

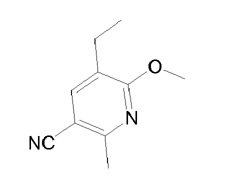

In a microwave oven vessel Preparation 50 (2.3g, 10mmol) was dissolved in DMF (30ml) and dicyanozinc (1.18g, 10.05mmol) and Pd(PPh₃)₄ (1.73g, 1.5mmol) were added. The mixture was heated under nitrogen atmosphere in a Biotage Initiator device at 180°C and normal absorvance for 30 min. The mixture was poured over ethyl acetate/water and the organic layer washed with water, brine, dried over magnesium sulphate and concentrated. The residue obtained was purified by column chromatography with a mixture of ethyl acetate in hexane (from 0 to 5%). Yield=88%.
LRMS: m/z 177 (M+1)⁺
Retention time: 7.12 min (Method B)
¹H NMR (300 MHz, CHLOROFORM-*d*) d ppm 1.18 (t, *J*=7.55 Hz, 3 H) 2.56 (q,
*J*=7.60 Hz, 2 H) 2.62 (s, 3 H) 3.99 (s, 3 H) 7.50 (s, 1 H)

### Preparation 50

### 3-Bromo-5-ethyl-6-methoxy-2-methylpyridine

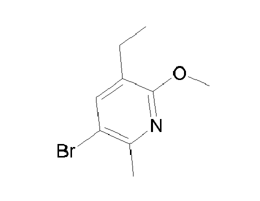

To a mixture of Preparation 51 (2.89g, 13.37mmol) and Ag₂CO₃ (4.98g, 18.05mmol) in DCM (100m1) under nitrogen atmosphere was added methyl iodide (4.83ml, 77.5mmol) and the mixture stirred at r.t. overnight. The reaction mixture was then filtered over Celite and concentrated under reduced pressure to yield the final compound (75% yield).
LRMS: m/z 232 (M+1)⁺
Retention time: 7.50 min (Method B)
¹H NMR (300 MHz, CHLOROFORM-*d*) d ppm 1.17 (m, 3 H) 2.50 (m, 5 H) 3.91 (s, 3 H) 7.44 (s, 1 H)

### Preparation 51

### 5-Bromo-3-ethyl-6-methylpyridin-2(1H)-one

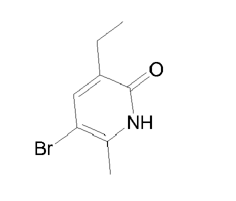

To a solution of Preparation 52 (3.22g, 23.47mmol) in methanol (85ml) at 0°C was added N-bromosuccinimide (4.39g, 24.67mmol) and the reaction mixture was stirred at r.t. overnight. Water was added and the solid obtained filtered. The filtrate was concentrated partially, cooled and the solid formed also filtered and mixed with the previous one. Yield=78%
LRMS: m/z 218 (M+1)⁺
Retention time: 6.07 min (Method B)
¹H NMR (300 MHz, CHLOROFORM-*d*) d ppm 1.19 (m, 3 H) 2.40 (s, 3 H) 2.53 (m, 2 H) 7.32 (s, 1 H)

### Preparation 52

### 3-Ethyl-6-methylpyridin-2(1H)-one

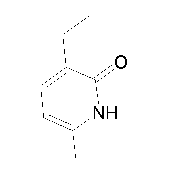

To a solution of 3-ethyl-6-methylpyridin-2-amine (5g, 36.7mmol) in sulfuric acid (78ml, 1.4mol) at 0°C was added dropwise a solution of sodium nitrite (2.18g, 31.6mmol) in water (20ml). The reaction mixture was stirred overnight at r.t. The pH was adjusted to 9 by addition of solid sodium hydroxide and the product extracted twice with ethyl acetate. The organic layer was dried and concentrated to give a solid which was recrystallized in cyclohexane to afford the desired compound (yield=64%).
LRMS: m/z 138 (M+1)⁺
Retention time: 4.93 min (Method B)
¹H NMR (300 MHz, CHLOROFORM-*d*) d ppm 1.2 (m, 3 H) 2.30 (s, 3 H) 2.54 (q,
*J*=7.23 Hz, 2 H) 5.98 (d, *J*=6.87 Hz, 1 H) 7.18 (d, *J*=6.87 Hz, 1 H)

### Preparation 53

### 1-Ethyl-3-[3-(6-methoxy-5-methylpyridin-3-yl)-1,2,4-oxadiazol-5-yl]-6,6-dimethyl-4,5,6,7-tetrahydro-1H-indazole

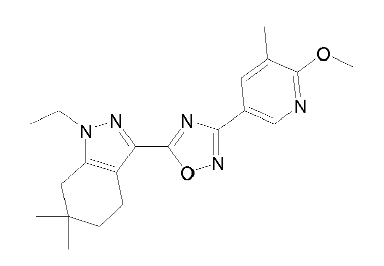

Obtained (21% yield) from Preparation 54 and Preparation 14 following the General Method 2.
LRMS: m/z 368 (M+1)⁺
Retention time: 6.63 min (Method B)
¹H NMR (300 MHz, METHANOL-*d*₄) d ppm 1.14 (s, 6 H) 1.5 (m, 3 H) 1.70 (t, *J*=5.49 Hz, 2 H) 2.33 (s, 3 H) 2.55 (s, 2 H) 2.9 (m, 2 H) 4.08 (s, 3 H) 4.25 (m, 2 H) 8.21 (s, 1 H) 8.78 (s, 1 H)

### Preparation 54

### N'-hydroxy-6-methoxy-5-methylpyridine-3-carboximidamide

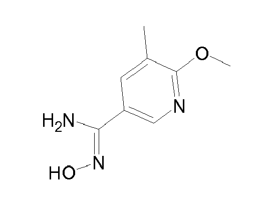

Obtained (100% yield) from Preparation 55 following the General Method 1.
LRMS: m/z 182 (M+1)⁺
Retention time: 1.37min (Method B)
¹H NMR (300 MHz, DMSO-*d*₆) d ppm 2.14 (s, 3 H) 3.88 (s, 3 H) 5.84 (s, 2 H) 7.77 (s, 1 H) 8.25 (m, 1 H) 9.58 (s, 1 H)

### Preparation 55

### 6-Methoxy-5-methylnicotinonitrile

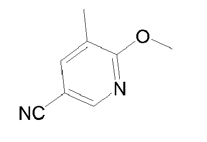

Obtained (57% yield) from Preparation 56 following the experimental procedure described for Preparation 49.
LRMS: m/z 149 (M+1)⁺
Retention time: 3.61 min (Method B)
¹H NMR (300 MHz, CHLOROFORM-*d*) d ppm 2.22 (s, 3 H) 4.02 (s, 3 H) 7.59 (s, 1 H) 8.34 (s, 1 H)

### Preparation 56

### 5-Bromo-2-methoxy-3-methylpyridine

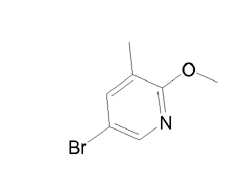

Obtained (83% yield) from 5-bromo-3-methylpyridin-2(1H)-one following the experimental procedure described for Preparation 50.
LRMS: no signal
Retention time: 7.25 min (Method B)
¹H NMR (300 MHz, CHLOROFORM-*d*) d ppm 2.17 (s, 3 H) 3.93 (s, 3 H) 7.48 (m, 1 H) 8.05 (m, 1 H)

### Preparation 57

### 4-[5-(1-Ethyl-6,6-dimethyl -4,5,6,7-tetrahyd ro-1H-indazol-3-yl)-1,2,4-oxadiazol-3-yl]-3-methylbenzoic acid

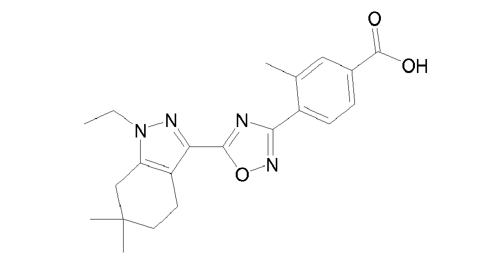

Obtained (69 % yield) from Preparation 58 following the General Method 3.
LRMS: m/z 381 (M+1)⁺
Retention time: 6.19min (Method B)

### Preparation 58

### Methyl 4-[5-(1-ethyl-6,6-dimethyl-4,5,6,7-tetrahydro-1H-indazol-3-yl)-1,2,4-oxadiazol-3-yl]-3-methylbenzoate

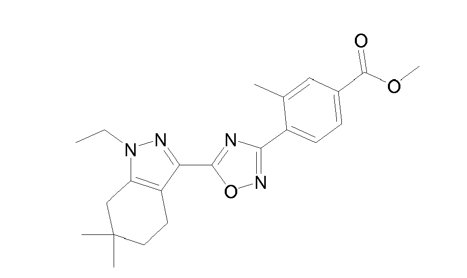

Obtained (23% yield) from Preparation 59 and Preparation 14 following the General Method 2.
LRMS: m/z 395 (M+1)⁺
Retention time: 7.80min (Method B)

### Preparation 59

### Methyl 4-[(Z)-amino(hydroxyimino)methyl]-3-methylbenzoate

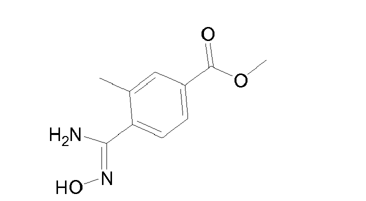

Obtained (35% yield) from Preparation 60 following the General Method 1.
LRMS: m/z 209 (M+1)⁺
Retention time: 2.98min (Method B)

### Preparation 60

### Methyl 4-cyano-3-methylbenzoate

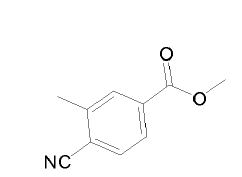

Obtained (69% yield) from methyl 4-bromo-3-methylbenzoate following the experimental procedure described for Preparation 49.
LRMS: no signal
Retention time: 6.32min (Method B)
¹H NMR (300 MHz, CHLOROFORM-*d*) d ppm 2.62 (s, 3 H) 3.96 (s, 3 H) 7.69 (d, *J*=7.97 Hz, 1 H) 7.93 (d, *J*=9.61 Hz, 1 H) 8.00 (s, 1 H)

### Preparation 61

### N'-hydroxy-3-methylpyridine-4-carboximidamide

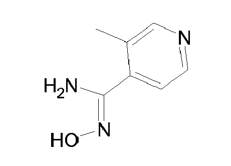

Obtained (88% yield) from Preparation 62 following the General Method 1.
LRMS: m/z 152 (M+1)⁺
Retention time: 0.65min (Method B)
¹H NMR (300 MHz, CHLOROFORM-*d)* d ppm 2.48 (s, 3 H) 7*.*33 (m, 1 H) 8.52 (m, 2 H)

### Preparation 62

### 3-Methylisonicotinonitrile

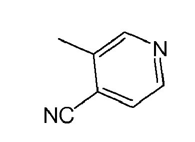

To a solution of 3-methylpyridine 1-oxide (10,59g, 0.1 mol) in ACN (22ml) was added iodoethane (17.5m1, 0.22mol) dropwise and the mixture stirred at r.t. for 2h. The solid formed was filtered off, redissolved in water (48ml) and warm up to 55°C. At this temperature KCN (12.3g, 0.1 9mol) in water (32ml) was added dropwise over 3.5h. Then the reaction mixture was stirred at this temperature for 2h and at r.t. overnight. The desired product was extracted with ether, washed with brine and concentrated. The solid obtained was recrystallized in diisopropyl ether. Yield=49%
LRMS: m/z 119 (M+1)⁺
Retention time: 3.77 min (Method B)

### Preparation 63

### 4-{5-[6,6-Dimethyl-1-(2,2,2-trifluoroethyl)-4,5,6,7-tetrahydro-1H-indazol-3-yl]-1,2,4-oxadiazol-3-yl)benzoic acid

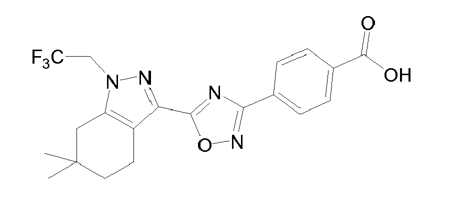

Obtained (50%yield) from Preparation 64 following the General Method 3.
LRMS: m/z 421 (M+1)⁺
Retention time: 7.22min (Method B)
¹H NMR (300 MHz, DMSO-*d*₆) d ppm 1.01 (s, 6 H) 1.59 (s, 2 H) 2.53 (m, 2 H) 2.82 - 2.86 (m, 2 H) 5.23 - 5.33 (m, 2 H) 8.11 - 8.24 (m, 4 H)

### Preparation 64

### Ethyl 4-{5-[6,6-dimethyl-1-(2,2,2-trifluoroethyl)-4,5,6,7-tetrahydro-1H-indazol-3-yl]-1,2,4-oxadiazol-3-yl}benzoate

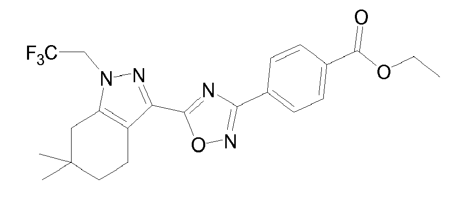

Obtained (31% yield) from Preparation 19 and Preparation 41 following the General Method 2.
LRMS: m/z 449 (M+1)⁺
Retention time: 7.82min (Method B)
¹H NMR (300 MHz, CHLOROFORM-*d*) d ppm 1.09 (s, 6 H) 1.43 (t, *J*=7.14 Hz, 3 H) 1.66 (t, *J*=6.45 Hz, 2 H) 2.45 (s, 2 H) 2.94 (t, *J*=6.45 Hz, 2 H) 4.43 (q, *J*=7.14 Hz, 2 H) 4.75 (q, *J*=8.24 Hz, 2 H) 8.18 (m, 2 H) 8.29 (m, 2 H)

### Preparation 65

### N'-hydroxy-3-methylpyridine-2-carboximidamide

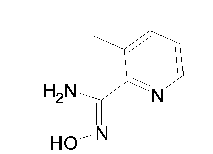

Obtained (100% yield) from 3-methylpicolinonitrile following the General Method 1.
LRMS: m/z 152 (M+1)⁺
Retention time: 0.72min (Method B)
¹H NMR (300 MHz, CHLOROFORM-*d*) d ppm 2.59 (s, 3 H) 5.67 (s, 2 H) 7.22 (dd, *J*=7.69, 4.94 Hz, 1 H) 7.56 (d, *J*=7.69 Hz, 1 H) 8.43 - 8.47 (m, 1 H)

### Preparation 66

### 3-{3-[6-(Benzyloxy)-4-methylpyridin-3-yl]-1,2,4-oxadiazol-5-yl}-1-ethyl-6,6-dimethyl-4,5,6,7-tetrahydro-1H-indazole

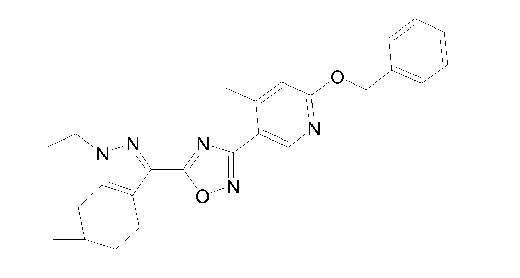

Obtained (57% yield) from Preparation 14 and Preparation 67 following the General Method 2.
LRMS: m/z 444 (M+1)⁺
Retention time: 8.05min (Method B)

### Preparation 67

### 6-(Benzyloxy)-N'-hydroxy-4-methylpyridine-3-carboximidamide

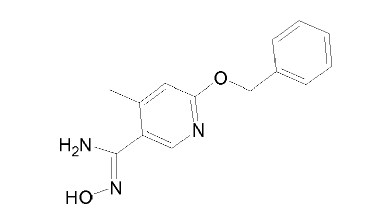

Obtained (20% yield) from Preparation 68 following the General Method 1.
LRMS: m/z 258 (M+1)⁺
Retention time: 4.23min (Method B)

### Preparation 68

### 6-(Benzyloxy)-4-methylnicotinonitrile

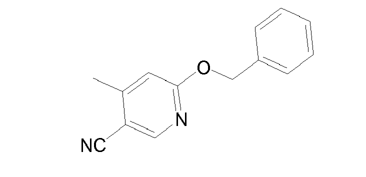

Obtained (68% yield) from Preparation 69 following the experimental procedure described for Preparation 49.
LRMS: m/z 225 (M+1)⁺
Retention time: 6.50min (Method B)
¹H NMR (300 MHz, CHLOROFORM-*d*) d ppm 2.48 (s, 3 H) 5.42 (s, 2 H) 6.73 (s, 1 H) 7.33 - 7.47 (m, 5 H) 8.42 (s, 1 H)

### Preparation 69

### 2-(Benzyloxy)-5-bromo-4-methylpyridine

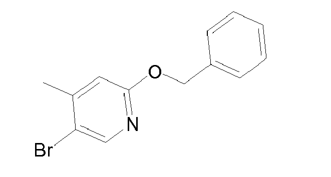

Obtaiend (95% yield) from 5-bromo-4-methylpyridin-2(1 H)-one and benzylbromide following the experimental procedure described for Preparation 50.
LRMS: m/z 278-280 (M+1)⁺

### Preparation 70

### 5-(1-Ethyl-6,6-dimethyl-4,5,6,7-tetrahydro-1H-indazol-3-yl)-3-(6-methoxy-2-methylpyridin-3-yl)-1,2,4-oxadiazole

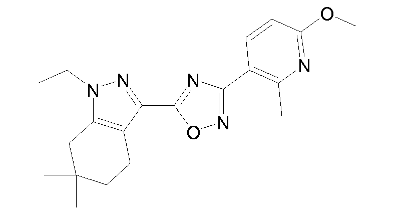

Obtained (66% yield) from Preparation 14 and Preparation 71 following the General Method 2.
LRMS: m/z 368 (M+1)⁺
Retention time: 7.78min (Method B)

### Preparation 71

### N'-Hydroxy-6-methoxy-2-methylnicotinimidamide

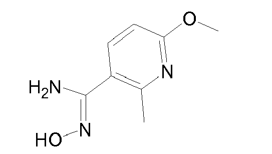

Obtained (22% yield) from Preparation 72 following the General Method 1.
LRMS: m/z 182 (M+1)⁺
Retention time: 0.67min (Method B)

### Preparation 72

### 6-Methoxy-2-methylnicotinonitrile

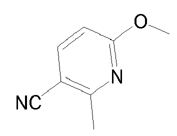

Obtained (68% yield) from Preparation 73 following the experimental procedure described for Preparation 49.
LRMS: no signal
Retention time: 5.08min (Method B)

### Preparation 73

### 3-Bromo-6-methoxy-2-methylpyridine

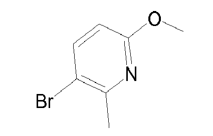

Obtained (68% yield) from 5-bromo-6-methylpyridin-2-ol following the experimental procedure described for Preparation 50.
LRMS: m/z 204 (M+1)⁺
Retention time: 6.32min (Method B)

### Preparation 74

### N'-Hydroxy-4-methylpyridine-3-carboximidamide

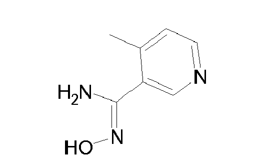

Obtained (33% yield) from Preparation 4-methylnicotinonitrile following the General Method 1.
LRMS: m/z 152 (M+1)⁺
Retention time: 0.62min (Method B)

### Preparation 75

### N'-Hydroxy-4-(trifluoromethyl)pyridine-3-carboximidamide

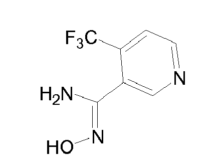

Obtained (31% yield) from 4-(trifluoromethyl)nicotinonitrile following the General Method 1.
LRMS: m/z 206 (M+1)⁺
Retention time: 1.60min (Method B)

### Preparation 76

### N'-hydroxyimidazo[1,2-a]pyridine-6-carboximidamide

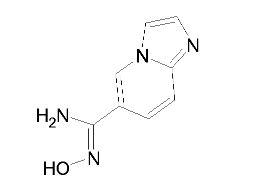

Obtained (100% yield) from Preparation 77 following the General Method 1.
LRMS: m/z 177 (M+1)⁺
Retention time: 0.57min (Method B)

### Preparation 77

### Imidazo[1,2-a]pyridine-6-carbonitrile

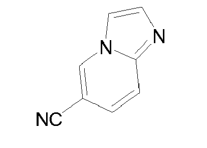

A solution of 6-aminonicotinonitrile (10g, 0.08mol) in CH₃CN (300 mL) was treated with the 2-chloroacetaldehyde solution (26.4ml, 0.21mol) and the mixture warm to reflux for 6h. The mixture was cooled down to r.t. overnight. The crystalline precipitate was filtered and washed with a little CH₃CN. The solid was treated with aq. NaHCO₃ solution until pH = 7 then extracted with DCM. The organic layer was dried (MgSO₄) and evaporated to give a pale yellow solid. Yield=95%
m/z 144 (M+1)⁺
Retention time: 0.65min (Method B)

### Preparation 78

### Methyl 4-[5-(1-ethyl-6,6-dimethyl-4,5,6,7-tetrahydro-1-indazol-3-yl)-1,2,4-oxadiazol-3-yl]-3-(trifluoromethyl)benzoate

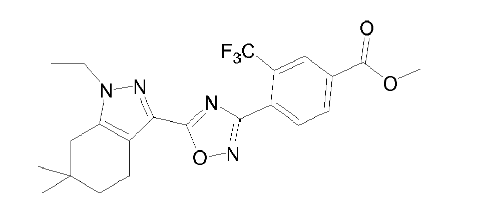

Obtained (36% yield) from Preparation 14 and Preparation 79 following the General Method 2.
LRMS: m/z 449 (M+1)⁺
Retention time: 7.68min (Method B)
¹H NMR (300 MHz, CHLOROFORM-*d*) d ppm 1.07 (s, 6 H) 1.47 (t, *J*=7.28 Hz, 3 H) 1.61 (m, 2 H) 2.41 (s, 2 H) 2.89 (t, *J*=6.32 Hz, 2 H) 4.01 (s, 3 H) 4.17 (m, 2 H) 8.05 (d, 1 H) 8.31 (d, 1 H) 8.52 (s, 1 H)

### Preparation 79

### Methyl 4-[(Z)-amino(hydroxyimino)methyl]-3-(trifluoromethyl)benzoate

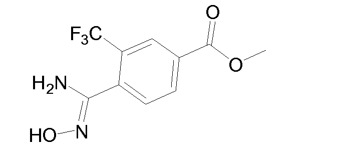

Obtained (30% yield) from Preparation 80 following the General Method 1.
LRMS: m/z 263 (M+1)⁺
Retention time: 3.75min (Method B)
¹H NMR (300 MHz, CHLOROFORM-*d*) d ppm 3.98 (s, 3 H) 4.89 (s, 2 H) 7.22 (s, 1 H) 7.70 (d, *J*=7.97 Hz, 1 H) 8.25 (d, *J*=9.06 Hz, 1 H) 8.41 (m, *J*=1.65 Hz, 1 H)

### Preparation 80

### Methyl 4-cyano-3-(trifluoromethyl)benzoate

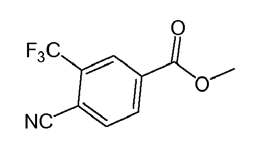

Obtained (55% yield) from Preparation 81 following the experimental procedure described for Preparation 49.
LRMS: m/z 247 (M+1)⁺
Retention time: 5.88min (Method B)
¹H NMR (300 MHz, CHLOROFORM-*d*) d ppm 4.01 (s, 3 H) 7.96 (d, *J*=7.97 Hz, 1 H) 8.34 (d, *J*=7.97 Hz, 1 H) 8.46 (s, 1 H)

### Preparation 81

### Methyl 3-(trifluoromethyl)-4-{[(trifluoromethyl)sulfonyl]oxy}benzoate

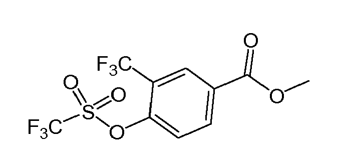

Obtained (94% yield) from Preparation 82 following the experimental procedure described for Preparation 5.
LRMS: m/z 370 (M+1)⁺
Retention time: 6.93min (Method B)
1 H NMR (300 MHz, CHLOROFORM-*d*) d ppm 3.99 (s, 3 H) 7.63 (d, *J*=8.51 Hz, 1 H) 8.35 (dd, *J*=8.52, 1.92 Hz, 1 H) 8.45 (d, 1 H)

### Preparation 82

### Methyl 4-hydroxy-3-(trifluoromethyl)benzoate

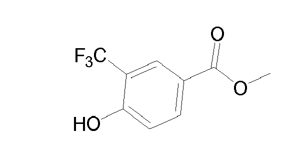

To a solution of Preparation 83 (2.43g, 11.8mmol) in methanol (50ml) was added acetyl chloride (1.26m1, 17.7mmol) and the mixture was stirred overnight at 60°C. Solvent was concentrated and the residue redissolved in ethyl acetate/water. The organic layer was separated, washed with brine, dried and evaporated under reduced pressure to give the title compound as a white solid (yield=92).
LRMS: m/z 219 (M-1)⁻
Retention time: 5.63min (Method B)
¹H NMR (300 MHz, CHLOROFORM-*d*) d ppm 3.95 (s, 3 H) 6.10 (s, 1 H) 7.02 (d, *J*=8.51 Hz, 1 H) 8.13 (dd, *J*=8.52, 1.65 Hz, 1 H) 8.26 (d, *J*=1.92 Hz, 1 H)

### Preparation 83

### 4-hydroxy-3-(trifluoromethyl)benzoic acid

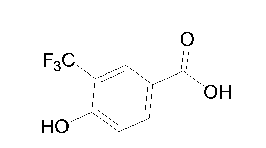

A mixture of 4-methoxy-3-(trifluoromethyl)benzoic acid (2.36g,10.72mmol) and pyridine hydrochloride (12.39g, 107mmol) was heated at 180°C for 5h. After cooling down to room temperature the reaction crude was poured over a 10% solution of citric acid and extracted with ethyl acetate. The organic layer was washed with brine, dried over magnesium sulphate and evaporated under reduced pressure. The oil obtained was purified by column chromatography using a mixture of DCM/MeOH (95/5) to give the title compound as a white solid (yield=84%).
LRMS: m/z 205 (M-1)⁻
Retention time: 4.82min (Method B)
¹H NMR (300 MHz, CHLOROFORM-*d*) d ppm 7.05 (d, *J*=8.51 Hz, 1 H) 8.19 (dd, *J*=8.52, 2.20 Hz, 1 H) 8.33 (d, *J*=1.92 Hz, 1 H)

### Preparation 84

### N'-hydroxy-2-methylpyridine-3-carboximidamide

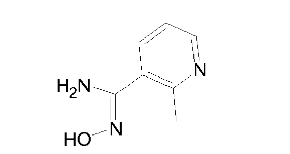

Obtained (93% yield) from Preparation 85 following the General Method 1.
LRMS: m/z 152 (M+1)⁺
Retention time: 0.58min (Method B)

### Preparation 85

### 2-methylnicotinonitrile

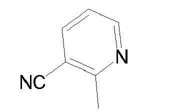

Obtained (83% yield) from 3-bromo-2-methylpyridine following the experimental procedure described for Preparation 49.
LRMS: no signal
Retention time: 3.67min (Method B)
¹H NMR (300 MHz, CHLOROFORM-*d*) d ppm 2.81 (s, 3 H) 7.23 - 7.30 (m, 1 H) 7.91 (d, *J*=7.69 Hz, 1 H) 8.70 (d, *J*=4.67 Hz, 1 H)

### Preparation 86

### 6-amino-N'-hydroxy-2-(trifluoromethyl)pyridine-3-carboximidamide

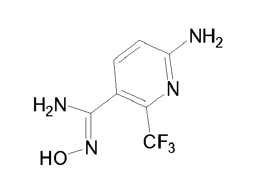

Obtained (54% yield) from Preparation 87 following the General Method 1.
LRMS: m/z 221 (M+1)⁺
Retention time: 0.52min (Method B)

### Preparation 87

### 6-amino-2-(trifluoromethyl)nicotinonitrile

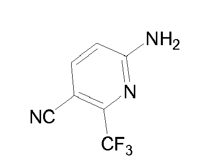

Obtained (100% yield) from Preparation 88 following the experimental procedure described for Preparation 49.
LRMS: m/z 186 (M-1)⁻
Retention time: 4.77min (Method B)
¹H NMR (300 MHz, CHLOROFORM-*d*) d ppm 5.25 (s, 2 H) 6.68 (d, *J*=9.06 Hz, 1 H) 7.78 (d, *J*=8.79 Hz, 1 H)

### Preparation 88

### 5-bromo-6-(trifluoromethyl)pyridin-2-amine

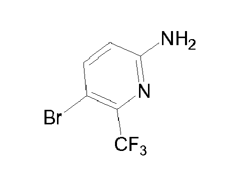

Obtained (81% yield) from 6-(trifluoromethyl)pyridin-2-amine following the experimental procedure described for Preparation 51.
LRMS: m/z 241/243 (M+1)⁺
Retention time: 5.62min (Method B)
¹H NMR (300 MHz, CHLOROFORM-*d*) d ppm 4.76 (s, 2 H) 6.54 (d, *J*=8.79 Hz, 1 H) 7.69 (d, *J*=8.51 Hz, 1 H)

### Preparation 89

### 3-Cyclopropyl-4-[5-(1-ethyl-6,6-dimethyl-4,5,6,7-tetrahydro-1H-indazol-3-yl)-1,2,4-oxadiazol-3-yl]benzoic acid

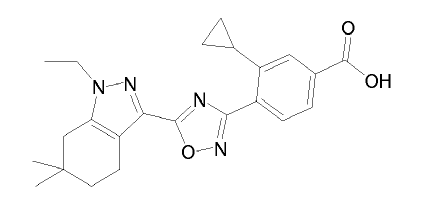

Obtained (90% yield) from Preparation 90 following the General Method 3.
LRMS: m/z 407 (M+1)⁺
Retention time: 7.42min (Method B)
¹H NMR (300 MHz, CHLOROFORM-*d*) d ppm 0.78 - 0.82 (m, 4 H) 1.08 (s, 6 H) 1.47 (t, *J*=7.30 Hz, 3 H) 1.62 (t, *J*=6.19 Hz, 2 H) 2.42 (s, 2 H) 2.74 (m, 1 H) 2.91 (t, *J*=6.19 Hz, 2 H) 4.19 (q, *J*=7.41 Hz, 2 H) 7.80 (s, 1 H) 7.97 (d, *J*=8.25 Hz, 1 H) 8.13 (d, *J*=7.94 Hz, 1 H)

### Preparation 90

### Methyl 3-cyclopropyl-4-[5-(1-ethyl-6,6-dimethyl-4,5,6,7-tetrahydro-1H-indazol-3-yl)-1,2,4-oxadiazol-3-yl]benzoate

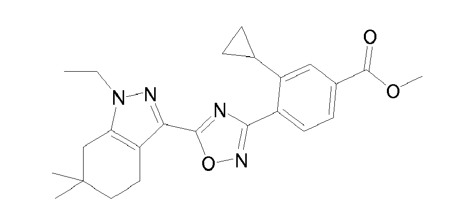

To a solution of Preparation 91 (1114mg, 0.25mmol), K₃PO₄ (179mg, 0.84mmol), cyclopropilboronic acid (56mg, 0.65mmol) and tricyclohexylphosphine (14mg, 0.05mmol) in toluene/water (1.5m1/0.1ml) under nitrogen atmosphere was added Pd(OAc)₂ (6mg, 0.02mmol). The mixture was heated at 100°C overnight under nitrogen atmosphere. The reaction mixture was then cooled to room temperature and concentrated in vacuum. Ethyl acetate was added to the residue and this organic layer was washed with water, brine, dried over MgSO₄, filtered and the solvent evaporated under vacuum. The residue was purified by column chromatography using a mixture of hexane/ethyl acetate (from 7/1 to 5/1). Yield=23%
LRMS: m/z 421 (M+1)⁺
Retention time: 7.85min (Method B)
¹H NMR (300 MHz, CHLOROFORM-*d*) d ppm 0.76 - 0.84 (m, 4 H) 1.09 (s, 6 H) 1.47 (t, *J*=7.28 Hz, 3 H) 1.63 (m, 2 H) 2.42 (s, 2 H) 2.74 (m, 1 H) 2.91 (m, 2 H) 3.95 (m, 3 H) 4.19 (q, *J*=7.42 Hz, 2 H) 7.75 (d, *J*=1.65 Hz, 1 H) 7.93 (dd,
*J*=8.10, 1.79 Hz, 1 H) 8.12 (d, *J*=7.97 Hz, 1 H)

### Preparation 91

### Methyl 3-bromo-4-[5-(1-ethyl-6,6-dimethyl-4,5,6,7-tetrahydro-1H-indazol-3-yl)-1,2,4-oxadiazol-3-yl]benzoate

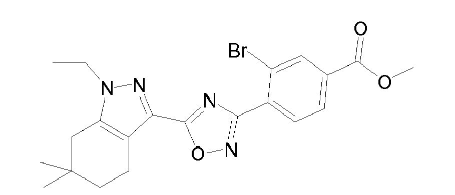

Obtained (38% yield) from Preparation 14 and Preparation 92 following the General Method 2.
LRMS: m/z 459/461 (M+1)⁺
Retention time: 7.77min (Method B)
¹H NMR (300 MHz, CHLOROFORM-*d*) d ppm 1.08 (s, 6 H) 1.47 (t, *J*=7.28 Hz, 3 H) 1.62 (t, *J*=6.45 Hz, 2 H) 2.42 (s, 2 H) 2.91 (t, *J*=6.32 Hz, 2 H) 3.97 (s, 3 H) 4.19 (q, *J*=7.42 Hz, 2 H) 8.09 (m, 2 H) 8.42 (s, 1 H)

### Preparation 92

### Methyl 4-[(Z)-amino(hydroxyimino)methyl]-3-bromobenzoate

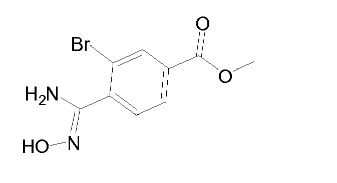

Obtained (25% yield) from Preparation 93 following the General Method 1.
LRMS: m/z 273 (M+1)⁺
Retention time: 3.30min (Method B)
¹H NMR (300 MHz, CHLOROFORM-*d*) d ppm 3.95 (s, 3 H) 4.92 (s, 2 H) 7.57 (d, *J*=7.97 Hz, 1 H) 8.01 (dd, *J*=7.97, 1.65 Hz, 1 H) 8.29 (d, *J*=1.65 Hz, 1 H)

### Preparation 93

### Methyl 3-bromo-4-cyanobenzoate

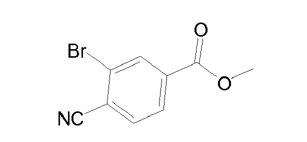

Obtained (55% yield) from Preparation 94 following the experimental procedure described for Preparation 49.
LRMS: no signal
Retention time: 4.93min (Method B)
¹H NMR (300 MHz, CHLOROFORM-*d*) d ppm 4.02 (s, 3 H) 7.93 (d, *J*=8.24 Hz, 1 H) 8.39 (d, *J*=8.10, 1 H) 8.46 (s, 1 H)

### Preparation 94

### Methyl 3-bromo-4-{[(trifluoromethyl)sulfonyl]oxy}benzoate

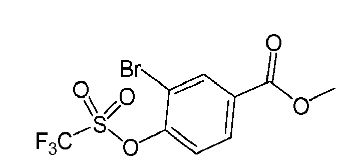

Obtained (81% yield) from Preparation 95 following the experimental procedure of Preparation 5.
LRMS: m/z 380, 382 (M+17)⁺
Retention time: 6.90min (Method B)
¹H NMR (300 MHz, CHLOROFORM-*d*) d ppm 3.96 (s, 3 H) 7.45 (m, 1 H) 8.07 (dd, *J*=8.52, 1.92 Hz, 1 H) 8.37 (d, *J*=2.20 Hz, 1 H)

### Preparation 95

### Methyl 3-bromo-4-hydroxybenzoate

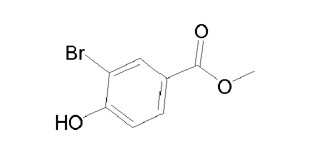

Obtained (100% yield) from 3-bromo-4-hydroxybenzoic acid following the experimental procedure described for Preparation 82.
LRMS: m/z 229, 231 (M-1)⁻
Retention time: 5.30min (Method B)
¹H NMR (300 MHz, CHLOROFORM-*d*) d ppm 3.90 (s, 3 H) 5.94 (s, 1 H) 7.07 (m, 1 H) 7.93 (m, 1 H) 8.20 (d, *J*=1.65 Hz, 1 H)

### Preparation 96

### 3-[3-(5-Ethyl-6-methoxy-2-methylpyridin-3-yl)-1,2,4-oxadiazol-5-yl]-6,6-dimethyl-1-(2,2,2-trifluoroethyl)-4,5,6,7-tetrahydro-1H-indazole

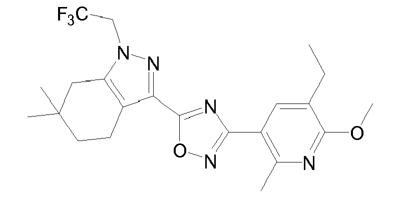

Obtained (37% yield) from Preparation 41 and Preparation 48 following the General Method 2.
LRMS: m/z 449 (M+1)⁺
Retention time: 8.21min (Method B)
¹H NMR (300 MHz, CHLOROFORM-*d*) d ppm 1.08 (s, 6H), 1.19-1.24 (t, 3H), 1.62-1.66 (t, 2H), 2.44 (s, 2H), 2.58-2.65 (q, 2H), 2.80 (s, 3H), 2.90-2.94 (t, 2H), 4.01 (s, 3H), 4.70-4.78 (m, 2H), 8.12 (s, 1 H)

### Preparation 97

### 1-Ethyl-3-(3-(5-ethyl-6-methoxy-2-methylpyridin-3-yl)-1,2,4-oxadiazol-5-yl)-6,6-dimethyl-1,4,6,7-tetrahydropyrano[4,3-c]pyrazole

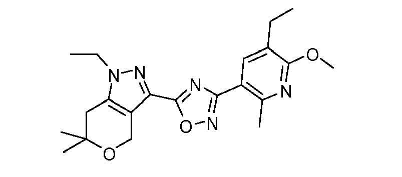

Obtained (17% yield) from Preparation 98 and Preparation 48 following the General Method 2.
¹H NMR (300 MHz, CHLOROFORM-d) d ppm: 1.19-1.24 (t, 3H), 1.38 (s, 6H), 1.47-1.52 (t, 3H), 2.60-2.64 (m, 4H), 2.80 (s, 3H), 4.01 (s, 3H), 4.18-4.25 (q, 2H), 5.00 (s, 2H), 8.14 (s, 1 H)

### Preparation 98

### 1-Ethyl-6,6-dimethyl-1,4,6,7-tetrahydropyrano[4,3-c]pyrazole-3-carboxylic acid

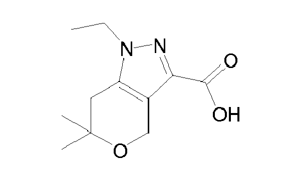

Obtained (80% yield) from Preparation 99 following the General Method 3.
LRMS: m/z 225 (M+1)⁺
Retention time: 4.32min (Method B)
¹H NMR (300 MHz, CHLOROFORM-*d*) d ppm 1.33(m, 6 H) 1.45 (t, *J*=7.28 Hz, 3 H) 2.58 (s, 2 H) 4.13 (q, *J*=7.14 Hz, 2 H) 4.85 (s, 2 H)

### Preparation 99

### Ethyl 1-ethyl-6,6-dimethyl-1,4,6,7-tetrahydropyrano[4,3-c]pyrazole-3-carboxylate

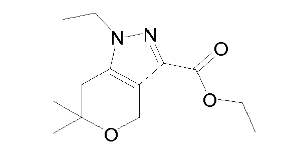

Obtained (14% yield) from Preparation 100 and ethylhydrazine hydrate following the experimental procedure described for Preparation 13.
LRMS: m/z 253 (M+1)⁺
Retention time: 5.27min (Method B)
¹H NMR (300 MHz, CHLOROFORM-*d*) d ppm 1.33 (s, 6 H) 1.35 - 1.47 (m, 6 H) 2.57 (s, 2 H) 4.13 (m, 2 H) 4.39 (q, *J*=7.05 Hz, 2 H) 4.84 (d, *J*=1.10 Hz, 2 H)

### Preparation 100

### Ethyl (6,6-dimethyl-4-oxotetrahydro-2H-pyran-3-yl)(oxo)acetate

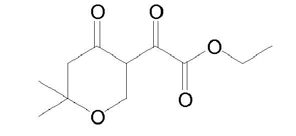

Obtained (45% yield) from 2,2-dimethyldihydro-2H-pyran-4(3H)-one following the experimental procedure described for Preparation 12.
LRMS: m/z 229 (M+1)⁺
Retention time: 5.20min (Method B)
¹H NMR (300 MHz, CHLOROFORM-*d*) d ppm 1.31 (s, 6 H) 1.40 (m, 3 H) 1.69 - 2.50 (m, 5 H) 4.36 (q, 2 H)

### Preparation 101

### (Z)-Ethyl 3-(4-(N'-hydroxycarbamimidoyl)-3-methylphenyl)propanoate

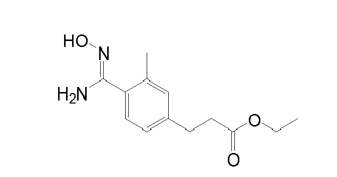

Obtained (56% yield) from Preparation 102 following the General Method 1.
LRMS: m/z 251 (M+1)⁺
Retention time: 3.56 min (Method B)

### Preparation 102

### Ethyl 3-(4-cyano-3-methylphenyl)propanoate

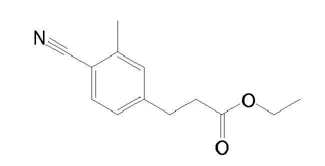

Preparation 103 (7.7g, 33.63mmol) was dissolved in methanol (200ml) and under nitrogen atmosphere was added Pd/C (0.07g, 0.07mmol). The mixture was hydrogenated at 1 psi for 1 h. The catalyst was filtered off and the filtrate concentrated to give the title compound (yield=97%).
LRMS: m/z 218 (M+1)⁺
Retention time: 6.06 min (Method B)
¹H NMR (200 MHz, CHLOROFORM-*d*) δ ppm 1.2 (t, J=7.2Hz, 3 H) 2.5 (s, 3 H) 2.6 (t, J=7.6Hz, 2H) 2.9 (t, J=7.6Hz, 2H) 4.1 (q, J=7.2Hz, 2H) 7.1 (m, 2H) 7.5 (d, J=7.8Hz, 1H)

### Preparation 103

### (E)-Ethyl 3-(4-cyano-3-methylphenyl)acrylate

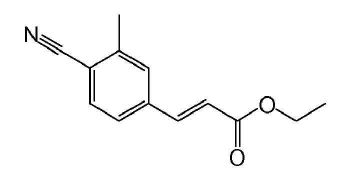

Obtained (66% yield) from 4-bromo-2-methylbenzonitrile and ethyl acrylate following the experimental procedure described for Preparation 6.
LRMS: m/z 216 (M+1)⁺
Retention time: 6.29 min (Method B)
¹H NMR (200 MHz, CHLOROFORM-*d*) δ ppm 1.3 (t, J=7.2Hz, 3 H) 2.6 (s, 3 H) 4.3 (q, J=7.2Hz, 2H) 6.5 (d, J=16Hz, 1 H) 7.5 (m, 4H)

### Preparation 104

### (Z)-N'-hydroxyisonicotinimidamide

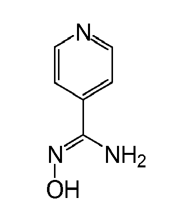

Obtained (56% yield) from isonicotinonitrile following the General Method 1.
LRMS: m/z 138 (M+1)⁺
Retention time: 0.64min (Method B)

### Preparation 105

### Methyl 3-(4-(5-(1-ethyl-6,6-dimethyl-4,5,6,7-tetrahydro-1H-indazol-3-yl)-1,2,4-oxadiazol-3-yl)-2,6-dimethylphenyl)propanoate

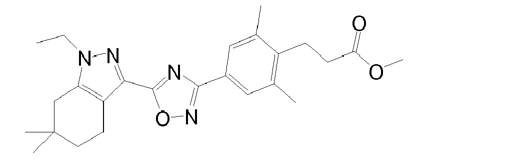

Obtained (38% yield) from Preparation 8 and Preparation 14 following the General Method 2.
LRMS: m/z 437 (M+1)⁺
Retention time: 8.44 min (Method B)

### Preparation 106

### Ethyl 3-(4-(4-(5-(1-ethyl-6,6-dimethyl-4,5,6,7-tetrahydro-1H-indazol-3-yl)-1,2,4-oxadiazol-3-yl)phenyl)piperazin-1-yl)propanoate

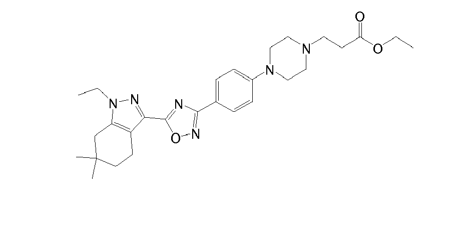

Obtained (6% yield) from Preparation 4 and Preparation 14 following the General Method 2.
LRMS: m/z 507 (M+1)⁺
Retention time: 5.89min (Method B)

### Preparation 107

### Ethyl 1-(4-(5-(1-ethyl-6,6-dimethyl-4,5,6,7-tetrahydro-1H-indazol-3-yl)-1,2,4-oxadiazol-3-yl)phenyl)piperidine-4-carboxylate

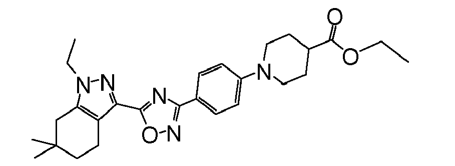

Obtained from Preparation 108 and Preparation 14 following the General Method 2. The title compound was used without further purification.
LRMS: m/z 478 (M+1)⁺
Retention time: 7.83 min (Method B)

### Preparation 108

### (E)-Ethyl 1-(4-(N'-hydroxycarbamimidoyl)phenyl)piperidine-4-carboxylate

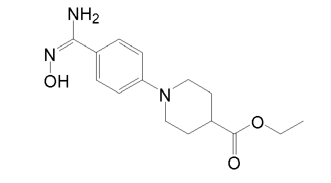

Obtained (84% yield) from Preparation 109 following the General Method 1.
LRMS: m/z 292 (M+1)⁺
Retention time: 3.85 min (Method B)

### Preparation 109

### Ethyl 4-(4-cyanophenyl)piperidine-1-carboxylate

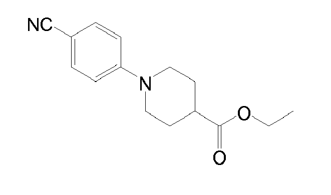

To a solution of 4-fluorobenzonitrile (1.16g, 9.55mmol) in DMSO (20ml) was added potassium carbonate (1.45g, 10.5mmol) and ethyl piperidine-4-carboxylate (1.5g, 9.55mol) and the mixture stirred at 120°C for 4h. The reaction mixture was poured over ethyl acetate and washed twice with 0.1 N HCl, twice with saturated NaHCO₃ and once with brine. The organic layer was dried and concentrated and the residue obtained purified with a mixture of hexane/ethyl acetate (from 95/5 to 1/1) to give the title compound. Yield=73%.
LRMS: m/z 259 (M+1)⁺
Retention time: 6.13 min (Method B)

### Preparation 110

### Ethyl 3-(4-(5-(1-ethyl-6,6-dimethyl-4,5,6,7-tetrahydro-1H-indazol-3-yl)-1,2,4-oxadiazol-3-yl)-3-methylphenyl)propanoate

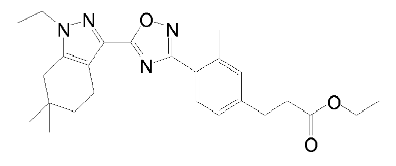

Obtained (14% yield) from Preparation 101 and Preparation 14 following the General Method 2.
LRMS: m/z 479 (M+1)⁺
Retention time: 7.86min (Method B)

### Preparation 111

### Tert-butyl 3-(4-(5-(1-ethyl-6,6-dimethyl-4,5,6,7-tetrahydro-1H-indazol-3-yl)-1,2,4-oxadiazol-3-yl)-3-(trifluoromethyl)phenyl)propanoate

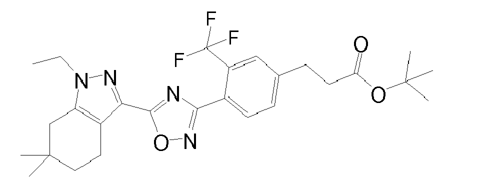

Obtained (38% yield) from Preparation 112 and Preparation 14 following the General Method 2.
LRMS: m/z 519 (M+1)⁺
Retention time: 7.92min (Method B)

### Preparation 112

### (Z)-Tert-butyl 3-(4-(N'-hydroxycarbamimidoyl)-3-(trifluoromethyl)phenyl) propanoate

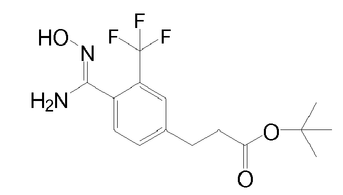

Obtained (31% yield) from Preparation 113 following the General Method 1.
LRMS: m/z 333 (M+1)⁺
Retention time: 5.07min (Method B)

### Preparation 113

### Tert-butyl 3-(4-cyano-3-(trifluoromethyl)phenyl)propanoate

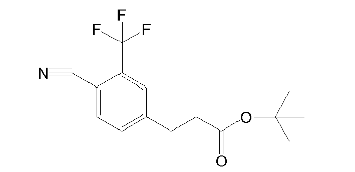

Obtained (93% yield) from Preparation 114 following the experimental procedure described for Preparation 102.
LRMS: m/z 317 (M+17)⁺
Retention time: 6.82min (Method B)

### Preparation 114

### (E)-tert-butyl 3-(4-cyano-3-(trifluoromethyl)phenyl)acrylate

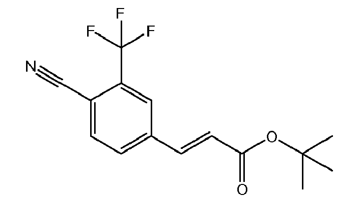

Obtained (55% yield) from Preparation 115 and tert-butylacrylate following the experimental procedure described for Preparation 6.
LRMS: m/z 298 (M+1)⁺
Retention time: 7.03min (Method B)
¹H NMR (200 MHz, CHLOROFORM-*d*) δppm 1.5 (s, 9 H) 6.5 (d, J=16Hz, 1 H) 7.6 (d, J=16Hz, 1H) 7.8 (m, 3H)

### Preparation 115

### 4-cyano-3-(trifluoromethyl)phenyl trifluoromethanesulfonate

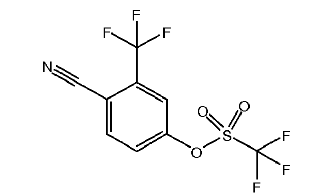

Obtained (86% yield) from 4-hydroxy-2-(trifluoromethyl)benzonitrile following the experimental procedure described for Preparation 5.
LRMS: no signal
Retention time: 6.61 min (Method B)
¹H NMR (200 MHz, CHLOROFORM-*d*) δppm 7.7 (m, 2H) 8.0 (d, J=8.6Hz, 1 H)

### Preparation 116

### tert-butyl 6-(5-(1-ethyl-6,6-dimethyl-4,5,6,7-tetrahydro-1H-indazol-3-yl)-1,2,4-oxadiazol-3-yl)-1,2,3,4-tetrahydronaphthalen-2-ylcarbamate

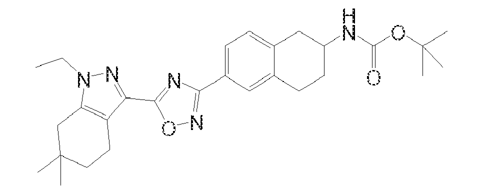

Obtained (94% yield) from Preparation 117 and Preparation 14 following the General Method 2.
LRMS: m/z 492 (M+1)⁺
Retention time: 7.88min (Method B)

### Preparation 117

### (Z)-Tert-butyl 6-(N'-hydroxycarbamimidoyl)-1,2,3,4-tetrahydronaphthalen-2-ylcarbamate

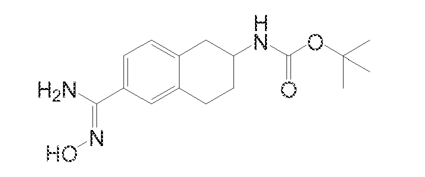

Obtained (100% yield) from Preparation 118 following the General Method 1.
LRMS: m/z 306 (M+1)⁺
Retention time: 4.09min (Method B)

### Preparation 118

### Tert-butyl 6-cyano-1,2,3,4-tetrahydronaphthalen-2-ylcarbamate

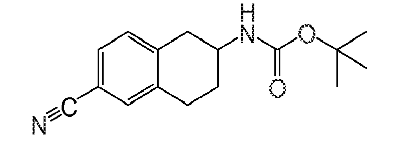

Obtained (79% yield) from Preparation 119 following the experimental procedure described for Preparation 49.
LRMS: m/z 273 (M+1)⁺
Retention time: 6.31min (Method B)

### Preparation 119

### Tert-butyl 6-bromo-1,2,3,4-tetrahydronaphthalen-2-ylcarbamate

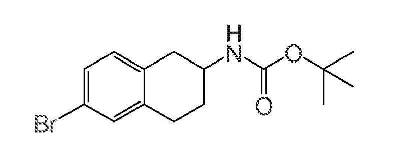

Obtained (99% yield) from Preparation 120 following the experimental procedure described for Preparation 23.
LRMS: m/z 326, 328 (M+1)⁺
Retention time: 7.14min (Method B)

### Preparation 120

### 6-Bromo-1,2,3,4-tetrahydronaphthalen-2-amine

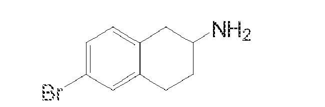

To a solution of the 6-bromo 2-tetralone (2g, 8.89mmol) and NH₄OAc (5.52g, 71.61mmol) in MeOH (100 mL) was added NaCNBH₃ (0.67g, 10.66mmol) at room temperature. The resulting yellow solution was stirred at that temperature for 20 hours. The reaction mixture was acidified with 2 M HCl, stirred for 10min and methanol evaporated. The mixture was extracted with CH₂Cl₂ twice. The aqueous layer was basified with 1.0 N NaOH to pH 10 then extracted with CH₂Cl₂ two times. The extracts are dried over anhydrous MgSO₄ and concentrated in vacuo to afford 1.31 g (65percent yield) of the desired product as a yellow oil which is used without further purification.
LRMS: m/z 226.1 (M+H)⁺, 209 (M+H- NH3)⁺.
Retention time: 3.84min (Method B)
¹H NMR (300 MHz, CD₃OD) δ 7.27-7.35 (m, 8H), 7.05 (d, J = 8.4 Hz, 4H), 3.56 (m, 1H), 3.17 (dd, J = 3.9, 16.2 Hz, 1H), 2.95 (m, 2H), 2.81 (dd, J = 9.9, 16.2 Hz, 1H), 2.19-2.29 (m, 1H), 1.79-1.92 (m, 1H)

### Preparation 121

### tert-butyl 3-(2,6-dimethyl-4-(5-(1,6,6-trimethyl-4,5,6,7-tetrahydro-1H-indazol-3-yl)-1,2,4-oxadiazol-3-yl)phenyl)propanoate

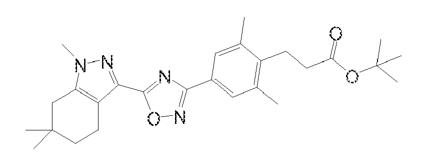

Obtained (25% yield) from Preparation 11 and Preparation 18 following the General Method 2.
LRMS: m/z 479 (M+H)⁺
Retention time: 8.09min (Method B)

### Preparation 122

### Tert-butyl 3-(4-(5-(6,6-dimethyl-4,5,6,7-tetrahydrobenzo[d]isoxazol-3-yl)-1,2,4-oxadiazol-3-yl)-2,6-dimethylphenyl)propanoate

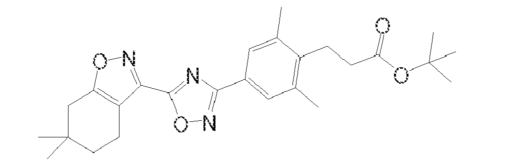

Obtained (59% yield) from Preparation 11 and Preparation 123 following the General Method 2.
LRMS: m/z 452 (M+H)⁺
Retention time: 8.19min (Method B)

### Preparation 123

### 6,6-Dimethyl-4,5,6,7-tetrahydrobenzo[d]isoxazole-3-carboxylic acid

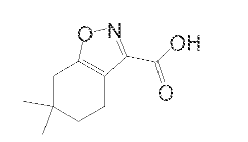

Obtained (71% yield) from Preparation 124 following the General Method 3.
LRMS: m/z 196 (M+H)⁺
Retention time: 5.53min (Method B)
¹H NMR (200 MHz, DMSO-D6) δ ppm 1.0 (s, 6 H) 1.5 (t, *J*=6.4 Hz, 2 H) 2.5 (s, 2 H)2.5(t,*J*=1.6Hz,2H)

### Preparation 124

### Ethyl 6,6-dimethyl-4,5,6,7-tetrahydrobenzo[d]isoxazole-3-carboxylate

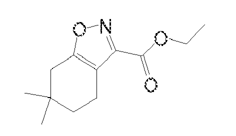

Obtained (90% yield) from Preparation 12 and hydroxylamine hydrochloride following the experimental procedure described for Preparation 13.
LRMS: m/z 224 (M+H)⁺
Retention time: 6.55min (Method B)

### Preparation 125

### 5-(6,6-dimethyl-1-(pyridin-3-ylmethyl)-4,5,6,7-tetrahydro-1H-indazol-3-yl)-3-(5-ethyl-6-methoxy-2-methylpyridin-3-yl)-1,2,4-oxadiazole

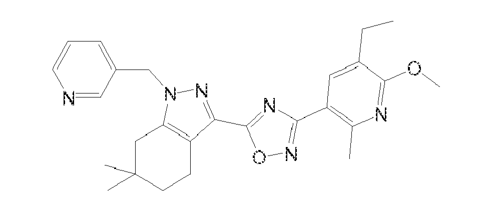

Obtained (21% yield) from Preparation 126 and Preparation 48 following the General Method 2.
LRMS: m/z 459 (M+H)⁺
Retention time: 7.90 min (Method B)

### Preparation 126

### 6,6-dimethyl-1-(pyridin-3-ylmethyl)-4,5,6,7-tetrahydro-1H-indazole-3-carboxylic acid

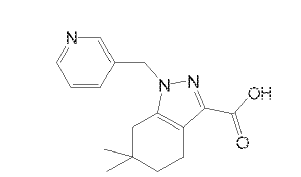

Obtained (85% yield) from Preparation 127 following the General Method 3.
LRMS: m/z 286 (M+H)⁺
Retention time: 4.90 min (Method B)

### Preparation 127

### Ethyl 6,6-dimethyl-1-(pyridin-3-ylmethyl)-4,5,6,7-tetrahydro-1H-indazole-3-carboxylate

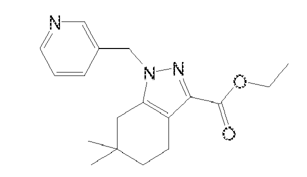

Obtained (76% yield) from Preparation 12 and 3-(hydrazinylmethyl)pyridine following the experimental procedure described for Preparation 13.
LRMS: m/z 314 (M+H)⁺
Retention time: 6.02 min (Method B)

### Preparation 128

### Ethyl 2-(4-(5-(6,6-dimethyl-4,5,6,7-tetrahydrobenzo[d]isoxazol-3-yl)-1,2,4-oxadiazol-3-yl)-2,6-dimethylphenethylamino)acetate

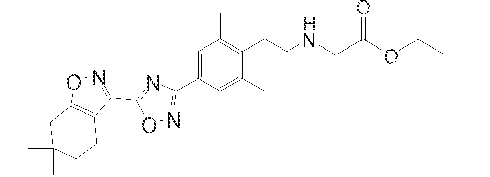

Obtained (10% yield) from Example 72 and ethyl 2-oxoacetate following the General Method 8.
LRMS: m/z 453 (M+H)⁺
Retention time: 6.05min (Method B)

### Preparation 129

### Ethyl 2-(3-(4-(5-(6,6-dimethyl-4,5,6,7-tetrahydrobenzo[d]isoxazol-3-yl)-1,2,4-oxadiazol-3-yl)-2,6-dimethylphenyl)propylamino)acetate

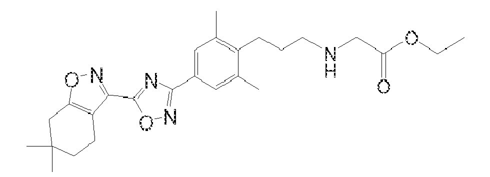

Obtained (41% yield) from Example 70 and ethyl 2-oxoacetate following the General Method 8.
LRMS: m/z 467 (M+H)⁺
Retention time: 6.36min (Method B)

### Preparation 130

### Ethyl 3-(4-(5-(6,6-dimethyl-4,5,6,7-tetrahydrobenzo[d]isoxazol-3-yl)-1,2,4-oxadiazol-3-yl)-2,6-dimethylphenethylamino)propanoate

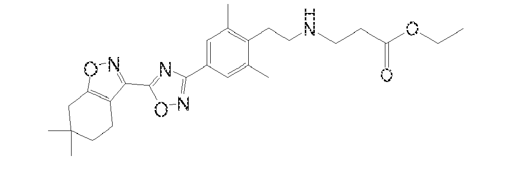

To a solution of Example 72 (100mg, 0.26mmol) in ethanol (5ml) was added ethyl acrylate (30 1, 300mmol) and the reaction mixture stirred overnight at r.t.. The crude was evaporated and purified according the General Purification Method (64% yield).
LRMS: m/z 467 (M+H)⁺
Retention time: 6.07min (Method B)

### Preparation 131

### 5-(1-ethyl-4,5,6,7-tetrahydro-1H-indazol-3-yl)-3-(5-ethyl-6-methoxy-2-methylpyridin-3-yl)-1,2,4-oxadiazole

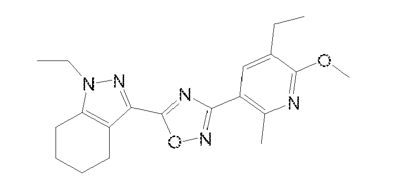

Obtained (20% yield) from Preparation 132 and Preparation 48 following the General Method 2.
LRMS: m/z 368 (M+H)⁺
Retention time: 7.92 min (Method B)

### Preparation 132

### 1-Ethyl-4,5,6,7-tetrahydro-1H-indazole-3-carboxylic acid

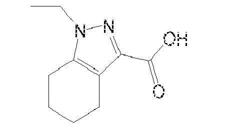

Obtained (70% yield) from Preparation 133 following the General Method 3.
LRMS: m/z 195 (M+H)⁺
Retention time: 4.78 min (Method B)

### Preparation 133

### Ethyl 1-ethyl-4,5,6,7-tetrahydro-1H-indazole-3-carboxylate

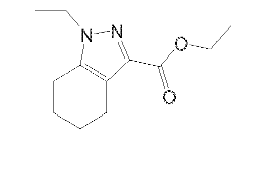

Obtained (70% yield) from Preparation 134 and the oxalic salt of ethylhydrazine following the experimental procedure described for Preparation 13.
LRMS: m/z 223(M+H)⁺
Retention time: 5.75 min (Method B)

### Preparation 134

### Ethyl 2-oxo-2-(2-oxocyclohexyl)acetate

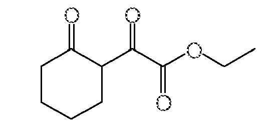

Obtained (85% yield) from cyclohexanone and diethyl oxalate following the experimental procedure described for Preparation 12.
LRMS: m/z 199 (M+H)⁺
Retention time: 5.57 min (Method B)

### Preparation 135

### 3-(5-Ethyl-6-methoxy-2-methylpyridin-3-yl)-5-(1,6,6-trimethyl-4,5,6,7-tetrahydro-1H-indazol-3-yl)-1,2,4-oxadiazole

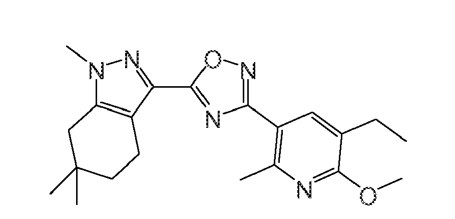

Obtained (10% yield) from Preparation 18 and Preparation 48 following the General Method 2.
LRMS: m/z 382 (M+H)⁺
Retention time: 6.47min (Method B)

### Preparation 136

### 3-(3-(5-Ethyl-6-methoxy-2-methylpyridin-3-yl)-1,2,4-oxadiazol-5-yl)-6,6-dimethyl-4,5,6,7-tetrahydrobenzo[d]isoxazole

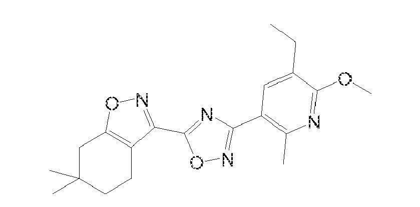

Obtained (40% yield) from Preparation 123 and Preparation 48 following the General Method 2.
LRMS: m/z 369 (M+H)+
Retention time: 8.12 min (Method B)

### Preparation 137

### Tert-butyl 2-(3-(3-(5-ethyl-6-methoxy-2-methylpyridin-3-yl)-1,2,4-oxadiazol-5-yl)-6,6-dimethyl-4,5,6,7-tetrahydro-1H-indazol-1-yl)ethylcarbamate

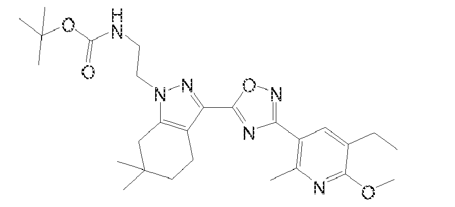

Obtained (14% yield) from Preparation 138 and Preparation 48 following the General Method 2.
LRMS: m/z 512 (M+H)+
Retention time:4.18 min (Method A)

### Preparation 138

### 1-(2-(tert-Butoxycarbonylamino)ethyl)-6,6-dimethyl-4,5,6,7-tetrahydro-1H-indazole-3-carboxylic acid

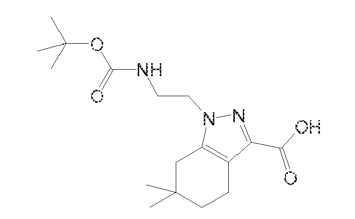

Obtained (77% yield) from Preparation 139 following the General Method 3.
LRMS: m/z 338 (M+H)+
Retention time: 6.08 min (Method B)

### Preparation 139

### Ethyl 1-(2-(tert-butoxycarbonylamino)ethyl)-6,6-dimethyl-4,5,6,7-tetrahydro-1H-indazole-3-carboxylate

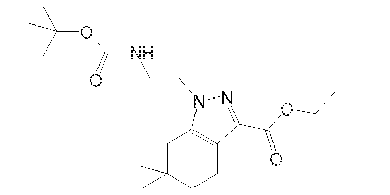

To a suspension of NaH (60%) (130mg, 3.24mmol) in DMF (2ml) under nitrogen atmosphere was added a solution of Preparation 15 (600mg, 2.70mmol) in DMF (2ml). The mixture was stirred at room temperature for 30min and then tert-butyl 2-bromoethylcarbamate (665mg, 2.97mmol) in DMF (0.5ml) was added and the reaction mixture stirred overnight at r.t..
Solvent was concentrated. The residue was dissolved in ethyl acetate, washed with water and brine, dried over magnesium sulphate and concentrated. The oil obtained was purified by column chromatography using a mixture of hexane/ethyl acetate (from 3/1 to 2/1) as eluent to give the desired compound as the main isomer (51 % yield).
LRMS: m/z 366 (M+H)+
Retention time: 6.82 min (Method B)

### Preparation 140

### 3-Ethyl-5-(5-(1-ethyl-6,6-dimethyl-4,5,6,7-tetrahydro-1H-indazol-3-yl)-1,2,4-oxadiazol-3-yl)-N-(4-methoxybenzyl)-6-methylpyridin-2-amine

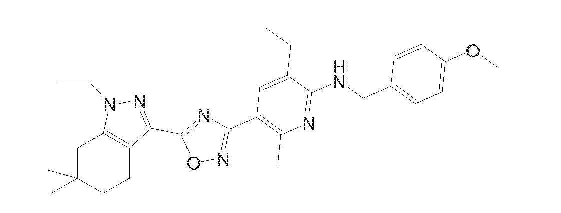

Obtained (4% yield) from Preparation 14 and Preparation 141 following the General Method 2.
LRMS: m/z 502 (M+H)+
Retention time: 7.90 min (Method B)

### Preparation 141

### (Z)-5-Ethyl-N'-hydroxy-6-(4-methoxybenzylamino)-2-methylnicotinimidamide

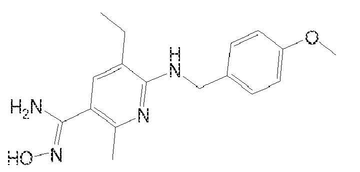

Obtained (21% yield) from Preparation 142 following the General Method 1.
LRMS: m/z 315 (M+H)+
Retention time:3.32 min (Method B)

### Preparation 142

### 5-Ethyl-6-(4-methoxybenzylamino)-2-methylnicotinonitrile

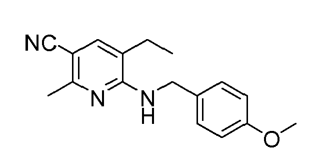

Obtained (47% yield) from Preparation 143 and 1-(chloromethyl)-4-methoxybenzene following the experimental procedure described for Preparation 139.
LRMS: m/z 282 (M+H)+
Retention time: 6.58 min (Method B)

### Preparation 143

### 6-Amino-5-ethyl-2-methylnicotinonitrile

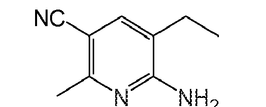

To a solution of Preparation 144 (2.97g, 13.81 mmol) in DMF (30ml) under nitrogen atmosphere was added CuCN (1.85g, 20.71mmol) and the mixture stirred at 150°C overnight. The reaction mixture was poured over water and the solid formed, filtered and redissolved in a mixture of ethyl acetate and aqueous ammonia. The organic layer was separated, washed with water, dried and concentrated to give the title compound (67% yield).
) LRMS: m/z 162 (M+H)+
Retention time: 3.37 min (Method B)

### Preparation 144

### 5-Bromo-3-ethyl-6-methylpyridin-2-amine

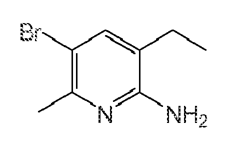

Obtained (94% yield) from 3-ethyl-6-methylpyridin-2-amine following the experimental procedure described for Preparation 51.
LRMS: m/z 215 (M+H)+, 217 (M+H)+
Retention time: 3.48 min (Method B)

### Preparation 145

### (Z)-4-Allyl-N'-hydroxy-3,5-dimethylbenzimidamide

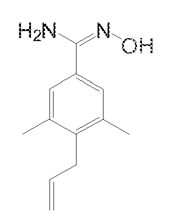

Obtained (69% yield) from Preparation 146 following the General Method 1.
LRMS: m/z 205 (M+H)⁺
Retention time: 4.07min (Method B)

### Preparation 146

### 4-Allyl-3,5-dimethylbenzonitrile

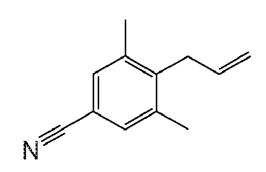

To a solution of Preparation 5 (5g, 14.74mmol) in DMF (175ml) under nitrogen atmosphere, was added allyltributylstannane (5.50ml, 17.74mmol) and Pd(PPh3)4 (1.71 g, 1.48mmol) and the mixture stirred overnight at 90°C. The reaction mixture was poured over ice/water and ethyl acetate was added. The mixture was filtered over Decalite and the organic layer separated, washed with water and brine, dried over magnesium sulphate and concentrated. The crude obtained was used without further purification (80% yield).
LRMS: no signal
Retention time: 6.69min (Method B)

### Preparation 147

### Ethyl 3-(4-(4-(5-(6,6-dimethyl-4,5,6,7-tetrahydrobenzo[d]isoxazol-3-yl)-1,2,4-oxadiazol-3-yl)phenyl)piperazin-1-yl)propanoate

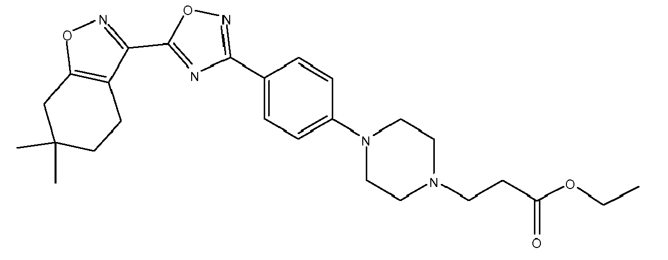

Obtained (12% yield) from Preparation 4 and Preparation 48 following the General Method 2.
LRMS: m/z 480 (M+1)⁺
Retention time: 4.38 min (Method B)

### Preparation 148

### 5-(1-Ethyl-6,6-difluoro-4,5,6,7-tetrahydro-1H-indazol-3-yl)-3-(5-ethyl-6-methoxy-2-methylpyridin-3-yl)-1,2,4-oxadiazole

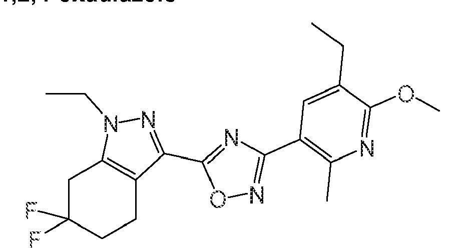

Obtained (7% yield) from Preparation 149 and Preparation 123 following the General Method 2.
LRMS: m/z 404 (M+1)⁺
Retention time: 7.72 min (Method B)

### Preparation 149

### 1-Ethyl-6,6-difluoro-4,5,6,7-tetrahydro-1H-indazole-3-carboxylic acid

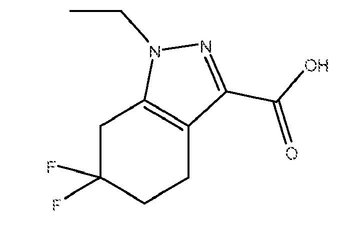

Obtained (7% yield) from Preparation 150 following the General Method 3.
LRMS: m/z 231 (M+1)⁺
Retention time: 4.73 min (Method B)

### Preparation 150

### Ethyl 1-ethyl-6,6-difluoro-4,5,6,7-tetrahydro-1H-indazole-3-carboxylate

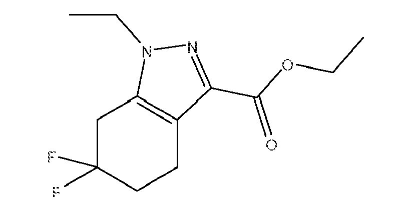

To a solution of Preparation 151 (150mg, 0,63mmol) in DCM (6 ml), trifluoride was added at-78°C (Diethylamino)sulfur trifluoride (415µl, 3,17mmol). It was left 10 min at - 78°C and 2h at room temperature. More DCM was added and then washed with NaHCO3 4%, water and brine.The organic layer was dried over magnesium sulphate and concentrated to yield 163mg of the desired compound as a solid (58% yield).
LRMS: m/z 259 (M+1)⁺
Retention time: 5.65 min (Method B)

### Preparation 151

### Ethyl 1-ethyl-6-oxo-4,5,6,7-tetrahydro-1H-indazole-3-carboxylate

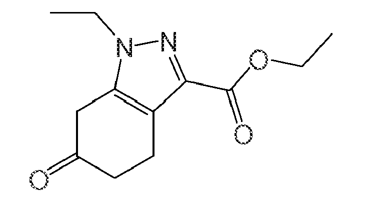

Preparation 152 (371 mg, 1,4 mmol) was dissolved in HCl 1M (10ml, 10mmol) and the resulting solution was stirred at room temperature for 1,5h. NaHCO3 solid was then added (ph 7) and stirred for 15min. CHCl3 was added and then washed with water and brine. The organic layer was dried over magnesium sulphate and concentrated to yield 282mg of the desired compound as a solid (85% yield).
LRMS: m/z 237 (M+1)⁺
Retention time: 4.67 min (Method B)

### Preparation 152

### Ethyl 6-ethoxy-1-ethyl-4,5-dihydro-1H-indazole-3-carboxylate

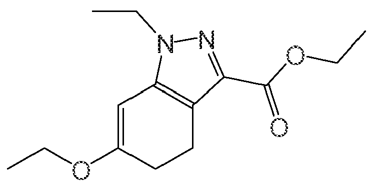

To a suspension of ethylhydrazine oxalic acid (0,75g, 4,99mmol) in EtOH (6ml) was added triethylamine (754µl, 5,4mmol). The resulting solution was slowly added to a solution of Preparation 153 in EtOH (6 ml). the resulting reaction mixture was stirred for 1h at room temperature. Then it was concentrated and Et20 was added and then washed with water and brine. The organic layer was dried over magnesium sulphate and concentrated and the resulting oil was purified by column chromatography with a mixture of hexane/AcOEt (4:1). The title compound was obtained as a solid (34% yield).
LRMS: m/z 265 (M+1)⁺
Retention time: 6.22 min (Method B)

### Preparation 153

### Ethyl 2-(4-ethoxy-2-oxocyclohex-3-enyl)-2-oxoacetate

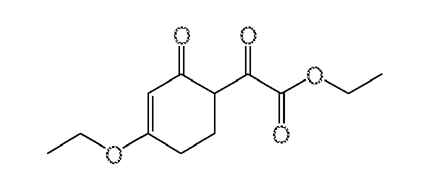

To a solution of sodium (1,23g, 53mmol) in EtOH (60ml) was added 3-ethoxycyclohex-2-enone (5g, 36mmol) in EtOH (10ml). The resulting solution was stirred at room temperature for 1h and then diethyl oxalate (4,83ml, 36mmol) in EtOH (10m1) was slowly added. The reaction mixture was stirred overnight at room temperature. It was concentrated and ethyl acetate was added and then washed with water and brine. The organic layer was dried over magnesium sulphate and concentrated to yield 2,71 g of the desired compound as a solid (32% yield).
LRMS: m/z 211 (M+1)⁺
Retention time: 5.83 min (10 min)

### Preparation 154

### Methyl 3-(3-ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoate

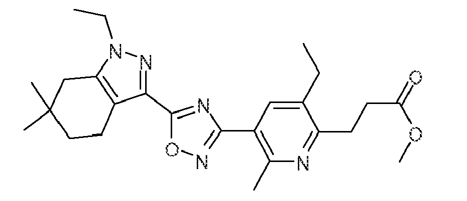

Obtained (44% yield) from Preparation 14 and Preparation 155 following the General Method 2.
LRMS: m/z 453 (M+1)⁺
Retention time: 7.70 min (9 min)

### Preparation 155

### (Z)-Methyl 3-(3-ethyl-5-(N'-hydroxycarbamimidoyl)-6-methylpyridin-2-yl)propanoate

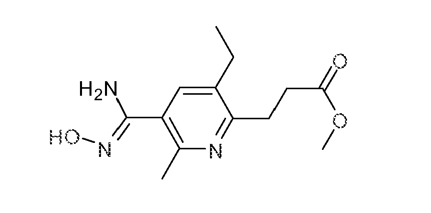

Obtained (51% yield) from Preparation 156 following the General Method 1.
LRMS: m/z 266 (M+1)⁺
Retention time: 2.90 min ( 9 min)

### Preparation 156

### Methyl 3-(5-cyano-3-ethyl-6-methylpyridin-2-yl)propanoate

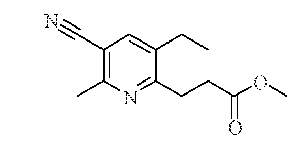

To a solution of Preparation 157 (100mg, 0,43mmol) in MeOH (4ml) was added Pd/C in catalytic quantity and submitted under hydrogen atmosphere at atmospheric pressure for 30min. After filtration of the catalyst and concentration 94 mg of the desired compound were obtained as a solid (86% yield).
LRMS: m/z 233 (M+1)⁺
Retention time: 5.73 min ( 9 min)

### Preparation 157

### (E)-Methyl 3-(5-cyano-3-ethyl-6-methylpyridin-2-yl)acrylate

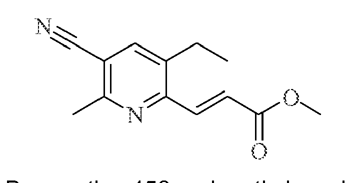

Obtained (44% yield) from Preparation 158 and methyl acrylate following the experimental procedure described for Preparation 6.
LRMS: m/z 231 (M+1)⁺
Retention time: 6.20 min ( 9 min)

### Preparation 158

### 6-Bromo-5-ethyl-2-methylnicotinonitrile

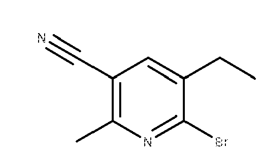

A mixture of 5-ethyl-6-hydroxy-2-methylnicotinonitrile (1,1g, 6,8mmol), phosphoryl tribromide (2g, 7mmol) and tribromophosphine (0,7ml, 7,4mmol) was stirred at 120°C for 3h. The resulting mixture was slowly added to a mixture of ice and water. DCM was added and then washed with water and brine. The organic layer was dried over magnesium sulphate and concentrated to yield 1,53g of the desired compound as a solid (94% yield).
LRMS: m/z 225, 227 (M+1)⁺
Retention time: 3.08 min (5 min)

### Preparation 159

### 5-(1-(Cyclopropylmethyl)-6,6-dimethyl-4,5,6,7-tetrahydro-1H-indazol-3-yl)-3-(5-ethyl-6-methoxy-2-methylpyridin-3-yl)-1,2,4-oxadiazole

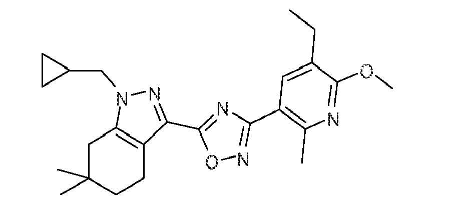

Obtained (44% yield) from Preparation 149 and Preparation 158 following the General Method 2.
LRMS: m/z 423 (M+1)⁺
Retention time: 8.25 min (10 min)

### Preparation 158

### 1-(Cyclopropylmethyl)-6,6-dimethyl-4,5,6,7-tetrahydro-1H-indazole-3-carboxylic acid

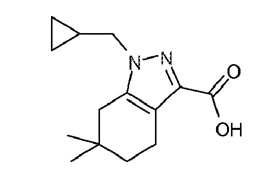

Obtained (100% yield) from Preparation 159 following the General Method 3.
LRMS: m/z 249 (M+1)⁺
Retention time: 4.67 min (Method B)

### Preparation 159

### Ethyl 1-(cyclopropylmethyl)-6,6-dimethyl-4,5,6,7-tetrahydro-1H-indazole-3-carboxylate

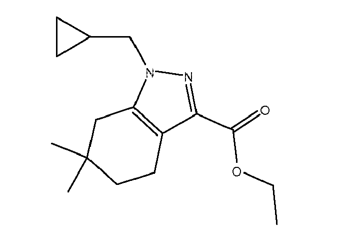

To a suspension of NaH (130mg, 3,2mmol) in DMF (2ml) was added Preparation 15 (600mg, 2,7mmol) in DMF (2ml) under nitrogen atmosphere and was stirred for 30 min. Then (bromomethyl)cyclopropane (400mg, 3mmol) in DMF was added and the rection mixture was stirred for 16h. Ethyl acetate was added and then washed with water and brine. The organic layer was dried over magnesium sulphate, concentrated and purified by column chromatography with a mixture of hexane/AcOEt (8:2) to yield 746 mg of the desired compound as a solid (50% yield).
LRMS: m/z 277 (M+1)⁺
Retention time: 5.78 min (Method B)

### Preparation 162

### 3-(4-Allyl-3,5-dimethylphenyl)-5-(1,6,6-trimethyl-4,5,6,7-tetrahydro-1H-indazol-3-yl)-1,2,4-oxadiazole

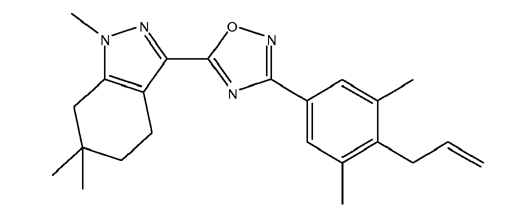

Obtained (33% yield) from Preparation 145 and Preparation 18 following the General Method 2.
LRMS: m/z 377 (M+H)⁺
Retention time: 8,08min (Method B)

### Preparation 163

### 2-(2,6-dimethyl-4-(5-(1,6,6-trimethyl-4,5,6,7-tetrahydro-1H-indazol-3-yl)-1,2,4-oxadiazol-3-yl)phenyl)acetaldehyde

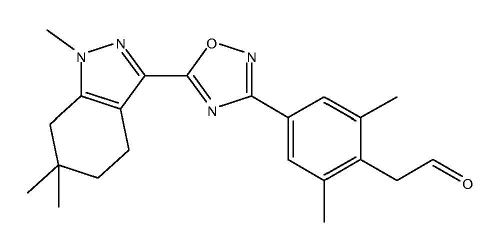

To a solution of the compound described in Example 87 (1,72g, 2,39mmol) in methanol (15ml) and water (2ml) was added NalO₄ (1,46g, 6,8mmol) and the mixture stirred overnight at room temperature. Methanol was concentrated and the residue dissolved in ethyl acetate and water. Organic layer was separated, washed with water and brine, dried over magnesium sulphate and concentrated to give 1,52g of the title compound (85% yield).
LRMS: m/z 379 (M+H)⁺
Retention time: 7,33min (Method B)

### Preparation 164

### Ethyl 3-(2-methyl-4-(5-(1,6,6-trimethyl-4,5,6,7-tetrahydro-1H-indazol-3-yl)-1,2,4-oxadiazol-3-yl)phenyl)propanoate

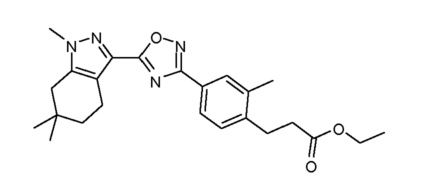

Obtained (54% yield) from Preparation 165 and Preparation 18 following the General Method 2.
LRMS: m/z 423 (M+H)⁺
Retention time: 7,76min (Method B)

### Preparation 165

### (Z)-Ethyl 3-(4-(N'-hydroxycarbamimidoyl)-2-methylphenyl)propanoate

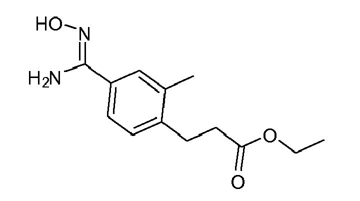

Obtained (27% yield) from Preparation 166 following the experimental procedure described for Preparation 8.
LRMS: m/z 251 (M+H)⁺
Retention time: 3,71min (Method B)

### Preparation 166

### (E)-Ethyl 3-(4-((Z)-N'-hydroxycarbamimidoyl)-2-methylphenyl)acrylate

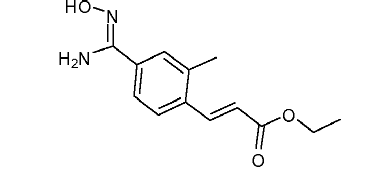

Obtained (89% yield) from Preparation 167 following the General Method 1.
LRMS: m/z 249 (M+H)⁺
Retention time: 4,04min (Method B)

### Preparation 167

### (E)-Ethyl 3-(4-cyano-2-methylphenyl)acrylate

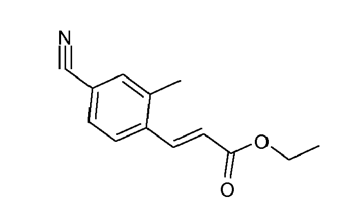

Obtained (79% yield) from 4-bromo-3-methylbenzonitrile and ethyl acrylate following the experimental procedure described for Preparation 6.
LRMS: m/z 216 (M+H)⁺
Retention time: 6,24min (Method B)

### Preparation 168

### 3-(4-Allyl-3,5-dimethylphenyl)-5-(6,6-dimethyl-4,5,6,7-tetrahydro-1H-indazol-3-yl)-1,2,4-oxadiazole

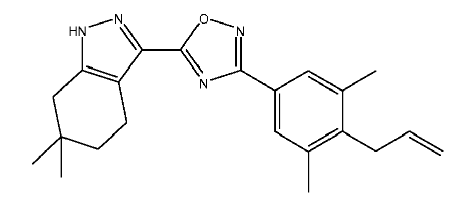

Obtained (56% yield) from Preparation 145 and Preparation 16 following the General Method 2.
LRMS: m/z 377 (M+H)⁺
Retention time: 8,08min (Method B)

### Preparation 169

### 2-(4-(5-(6,6-Dimethyl-4,5,6,7-tetrahydro-1H-indazol-3-yl)-1,2,4-oxadiazol-3-yl)-2,6-dimethylphenyl)acetaldehyde

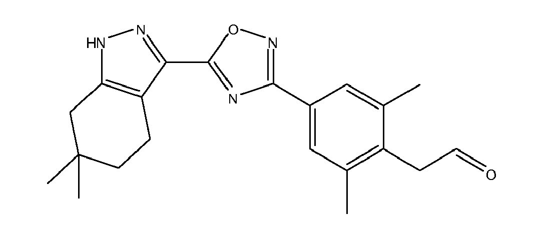

Obtained from example 94 following the procedure derscibed for Praparation 163. (95% yield).

### Preparation 170

### Ethyl 3-(4-(5-(1-ethyl-6,6-dimethyl-4,5,6,7-tetrahydro-1H-indazol-3-yl)-1,2,4-oxadiazol-3-yl)benzamido)propanoate

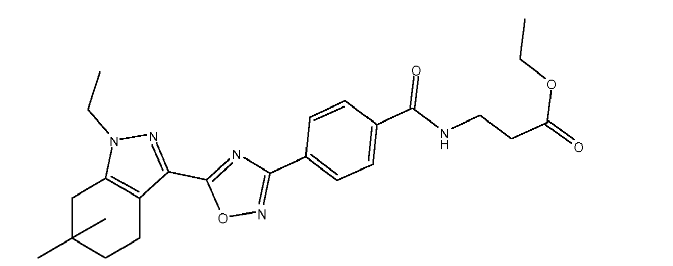

Obtained (77% yield) from the title compound in Example 10 and tert-butyl 3-aminopropanoate. HCl following the General Method 5.
LRMS: m/z 495 (M+H)⁺
Retention time:7.47 (Method B)

### Preparation 171

### 3-(4-Allyl-3,5-dimethylphenyl)-5-(6,6-dimethyl-1-(pyridin-3-ylmethyl)-4,5,6,7-tetrahydro-1H-indazol-3-yl)-1,2,4-oxadiazole

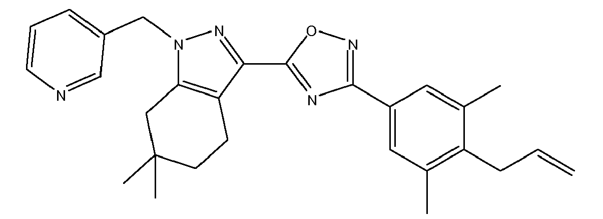

Obtained (54% yield) from Preparation 145 and Preparation 35 following the General Method 2.
LRMS: m/z 377 (M+H)⁺
Retention time: 8,08min (Method B)

### Preparation 172

### 2-(4-(5-(6,6-dimethyl-1-(pyridin-3-ylmethyl)-4,5,6,7-tetrahydro-1H-indazol-3-yl)-1,2,4-oxadiazol-3-yl)-2,6-dimethylphenyl)acetaldehyde

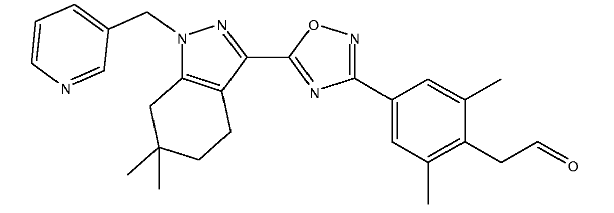

Obtained from example 96 following the procedure described for Praparation 163. (98% yield).

### Preparation 173

### tert-butyl 3-(2-chloro-4-(5-(1-ethyl-6,6-dimethyl-4,5,6,7-tetrahydro-1H-indazol-3-yl)-1,2,4-oxadiazol-3-yl)phenylsulfonamido)propanoate

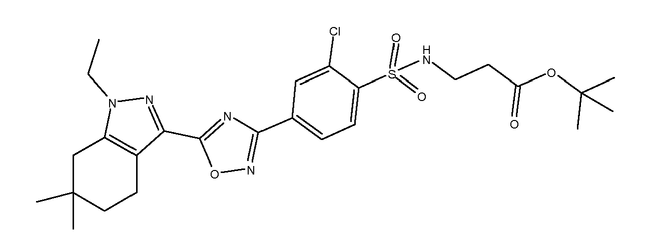

Obtained (29% yield) from Preparation 14 and Preparation 174 following the General Method 2.
LRMS: m/z 566 (M+H)⁺
Retention time: 7,72min (Method B)

### Preparation 174

### (Z)-Tert-butyl 3-(2-chloro-4-(N'-hydroxycarbamimidoyl)phenylsulfonamido) propanoate

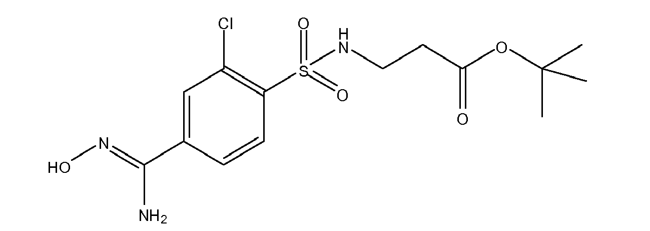

Obtained (98% yield) from Preparation 175 following the General Method 1.
LRMS: m/z 378 (M+H)⁺
Retention time: 3,07 (Method B)

### Preparation 175

### (Tert-butyl 3-(2-chloro-4-cyanophenylsulfonamido)propanoate

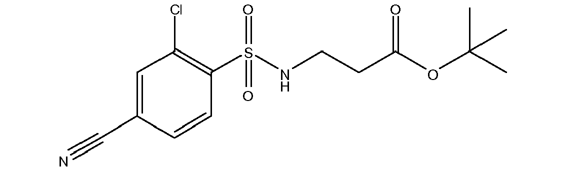

To a solution of tert-butyl 3-aminopropanoate.HCl (1,54g, 8,5mmol) in DCM (25ml) was added triethylamine (2,48ml, 17,8mmol) and stirred for 10 min. 2-chloro-4-cyanobenzene-1-sulfonyl chloride (2g, 8,47mmol) was slowly added as a solid and the resulting reaction mixture was stirred for 3h at room temperature. Ethyl acetate and water were added. Organic layer was separated, washed with water and brine, dried over magnesium sulphate and concentrated to give 2,92g of the title compound (100% yield).
LRMS: m/z 345 (M+H)⁺
Retention time: 6,10 (Method B)

### Preparation 176

### 3-(3-(4-Allyl-3,5-dimethylphenyl)-1,2,4-oxadiazol-5-yl)-6,6-dimethyl-4,5,6,7-tetrahydrobenzo[d]isoxazole

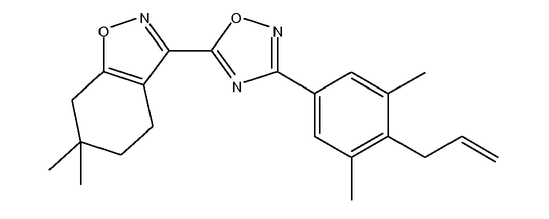

Obtained (63% yield) from Preparation 145 and Preparation 123 following the General Method 2.
LRMS: m/z 377 (M+H)⁺
Retention time: 8,08min (Method B)

### Preparation 177

### 2-(4-(5-(6,6-dimethyl-4,5,6,7-tetrahydrobenzo[d]isoxazol-3-yl)-1,2,4-oxadiazol-3-yl)-2,6-dimethylphenyl)acetaldehyde

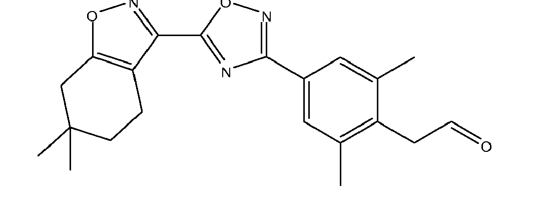

Obtained from example 98 following the procedure described for Praparation 163. (98% yield).

### Preparation 178

### (Z)-1-Ethyl-N'-hydroxy-6,6-dimethyl-4,5,6,7-tetrahydro-1H-indazole-3-carboximidamide

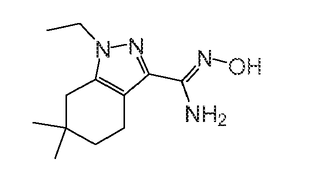

Obtained (100% yield) from Preparation 179 following the General Method 1.
LRMS: m/z 237 (M+H)+
Retention time: 4.22min (Method B)

### Preparation 179

### 1-ethyl-6,6-dimethyl-4,5,6,7-tetrahydro-1H-indazole-3-carbonitrile

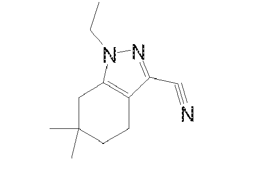

POCl₃ (14ml, 0.15mol) was added dropwise to a solution of Preparation 180 (30.20g, 0.14mol) in pyridine (200ml) at 0°C and the reaction mixture stirred at room temperatura for 1 h. The solvent was evaporated and the residue redissolved in ether and water. The organic layer was washed with water, brine and concentrated. The solid obtained was recristallyzed in hexane to yield the final compound as a white solid (88% yield).
¹H NMR (300 MHz, CHLOROFORM-D) δ ppm 1.0 (s, 6 H) 1.4 (t, *J*=7.4 Hz, 3 H) 1.5 (t, *J*=6.5 Hz, 2 H) 2.3 (s, 2 H) 2.6 (t, *J*=6.5 Hz, 2 H) 4.1 (q, *J*=7.4 Hz, 2 H)

### Preparation 180

### 1-Ethyl-6,6-dimethyl-4,5,6,7-tetrahydro-1H-indazole-3-carboxamide

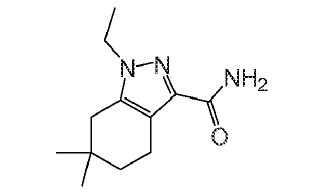

Obtained (71% yield) from Preparation 14 following the General Method 5.
¹H NMR (300 MHz, CHLOROFORM-D) δ ppm 1.0 (s, 6 H) 1.4 (m, 3 H) 1.5 (m, 2 H) 2.3 (s, 2 H) 2.8 (t, *J*=6.3 Hz, 2 H) 4.0 (m, 2 H) 5.3 (m, 1 H) 6.7 (s, 1 H)

### Preparation 181

### 3-(4-Bromo-2-methylphenyl)-5-(1-ethyl-6,6-dimethyl-4,5,6,7-tetrahydro-1Hindazol-3-yl)-1,2,4-oxadiazole

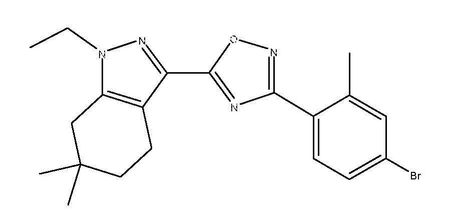

Obtained (35% yield) from Preparation 182 and Preparation 14 following the General Method 2.
LRMS: m/z 415-417 (M+H)⁺
Retention time: 8,33min (Method B)

### Preparation 182

### (Z)-4-Bromo-N'-hydroxy-2-methylbenzimidamide

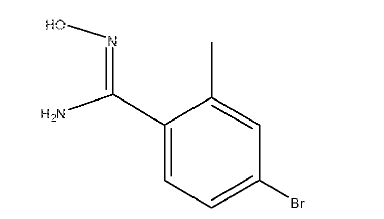

Obtained (60% yield) from 4-bromo-2-methylbenzonitrile following the General Method 1.
LRMS: m/z 229-231 (M+H)⁺
Retention time: 3,17min (Method B)

### Preparation 183

### Ethyl 3-(5-(5-(1-ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoate

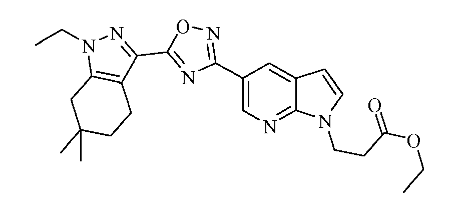

Obtained (70% yield) from Preparation 184 and Preparation 14 following the General Method 2.
LRMS: m/z 464 (M+H)⁺
Retention time: 7.63 (Method B)

### Preparation 184

### (Z)-Ethyl 3-(5-(N'-hydroxycarbamimidoyl)-1H-pyrrolo[2,3-b]pyridin-1yl)propanoate

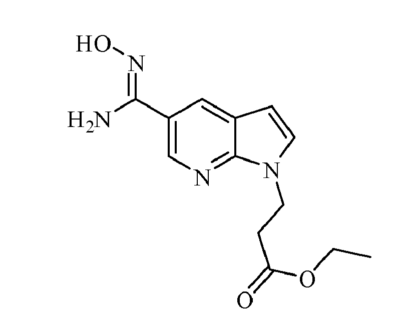

Obtained (76% yield) from Preparation 185 following the General Method 1.
LRMS: m/z 277 (M+H)⁺
Retention time: 3.33 (Method B)

### Preparation 185

### Ethyl 3-(5-cyano-1H-pyrrolo[2,3-b]pyridin-1-yl)propanoate

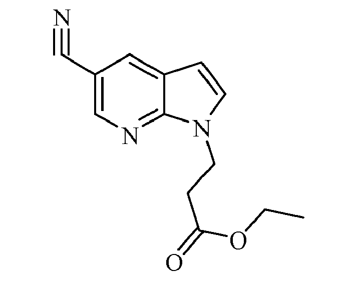

To a solution of 1H-pyrrolo[2,3-b]pyridine-5-carbonitrile (435mg, 3,04mmol) in DMF (15ml) under nitrogen atmosphere was added Cs₂CO₃ (2g, 6,14mmol) and stirred for 30 min. Ethyl 3-bromopropanoate (585µl, 4,56mmol) was then slowly added and the resulting reaction mixture was strirred at 80°C for 2h. Ethyl acetate and water were added. The organic layer was washed with water, brine and concentrated. It was purified by column chromatography with a mixture of hexane/AcOEt (3:1) to yield 739 mg of the desired compound as a solid (93% yield).
LRMS: m/z 244 (M+H)⁺
Retention time: 2.82 (Method A)

### Preparation 186

### Ethyl 3-(5-(5-(6,6-dimethyl-4,5,6,7-tetrahydrobenzo[d]isoxazol-3-yl)-1,2,4-oxadiazol-3-yl)-1H-pyrrolo[2,3-b]pyridin-1-yl)propanoate

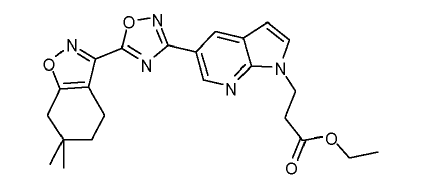

Obtained (33% yield) from Preparation 178 and Preparation 123 following the General Method 2.
LRMS: m/z 437 (M+H)⁺
Retention time: 7.62 (Method B)

### EXAMPLES

### EXAMPLE 1

### 4-(5-(6,6-Dimethyl-4,5,6,7-tetrahydro-2H-indazol-3-yl)-1,2,4-oxadiazol-3-yl)benzenesulfonamide

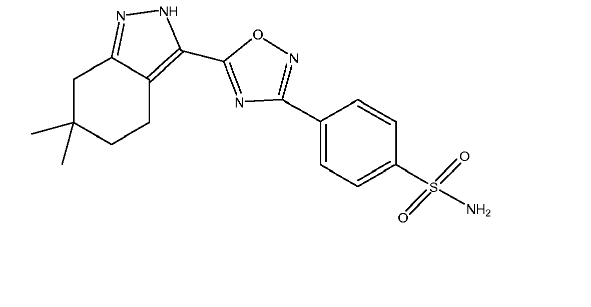

Obtained (10% yield) from Preparation 16 and Preparation 2 following the General Method 2.
LRMS: m/z 374 (M+1)⁺
Retention time: 15.33 min (Method C)
¹H NMR (200 MHz, DMSO-D6) δ ppm 1.0 (s, 6 H) 1.6 (t, *J*=6.2 Hz, 2 H) 2.5 (s, 2 H) 2.5 (s, 2 H) 2.8 (t, *J*=6.1 Hz, 2 H) 8.0 (d, J=8.6 Hz, 2 H) 8.3 (d, *J*=8.6 Hz, 2 H) 8.4 (s, 1 H)

### EXAMPLE 2

### (4-(5-(6,6-dimethyl-4,5,6,7-tetrahydro-2H-indazol-3-yl)-1,2,4-oxadiazol-3-yl)phenyl)metanol

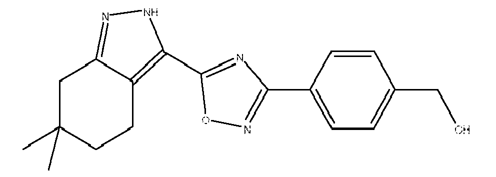

Obtained (19% yield) from Preparation 16 and Preparation 1 following the General Method 2.
LRMS: m/z 325 (M+1)⁺
Retention time: 16.04 min (Method C)
¹H NMR (200 MHz, DMSO-D6) δ ppm 1.0 (s, 6 H) 1.5 (t, J=6.1 Hz, 2 H) 2.4 (s, 2 H) 2.8 (t, *J*=6.1 Hz, 2 H) 4.6 (s, 2 H) 7.5 (d, *J*=8.2 Hz, 2 H) 8.0 (d, *J*=8.2 Hz, 2 H)

### EXAMPLE 3 (4-(5-(1,6,6-Trimethyl-4,5,6,7-tetrahydro-1H-indazol-3-yl)-1,2,4-oxadiazol-3-yl)phenyl)methanol

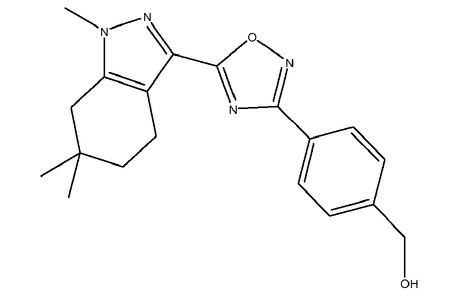

Obtained (19% yield) from Preparation 18 and Preparation 1 following the General Method 2.
LRMS: m/z 339 (M+1)⁺
Retention time: 16.83 min (Method C)
¹H NMR (400 MHz, CDCl₃) δ ppm 1.07 (s, 6 H) 1.61 (t, 2 H) 2.40 (t, 2 H) 2.91 (t, *J*=6.26 Hz, 2 H) 3.87 (s, 3 H) 4.78 (s, 2 H) 7.49 (d, *J*=8.61 Hz, 2 H) 8.21 (d, *J*=8.22 Hz, 2 H)

### EXAMPLE 4

### 4-(5-(1,6,6-Trimethyl-4,5,6,7-tetrahydro-1H-indazol-3-yl)-1,2,4-oxadiazol-3-yl)benzenesulfonamide

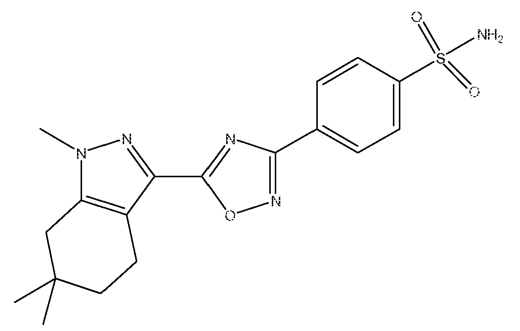

A mixture of example 1 (25mg, 0.07mmol), potassium carbonate (5mg, 0.04mmol) in and methyl iodide (5 I, 0.08mmol) DMF (1ml) was heated at 110°C for 1h. The reaction mixture was concentrated and the crude purified according to General Purification Method. Yield=11%.
LRMS: m/z 388 (M+1)⁺
Retention time: 16.10 min (Method C)
¹H NMR (400 MHz, CDCl₃) δ ppm 1.08 (s, 6 H) 1.61 (t, 2 H) 2.41 (s, 2 H) 2.91 (t, 2 H) 3.88 (s, 3 H) 8.04 (d, *J*=8.22 Hz, 2 H) 8.37 (d, *J*=8.61 Hz, 2 H)

### EXAMPLE 5

### 4-(5-(1-Ethyl-6,6-dimethyl-4,5,6,7-tetrahydro-1H-indazol-3-yl)-1,2,4-oxadiazol-3-yl)benzenesulfonamide

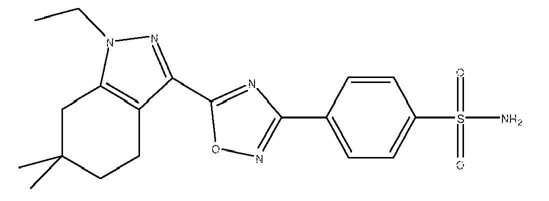

Obtained (41% yield) from Preparation 14 and Preparation 2 following the General Method 2.
LRMS: m/z 402 (M+1)⁺
Retention time: 17.15 min (Method C)
¹H NMR (200 MHz, DMSO-*d*₆) δ ppm 1.03 (s, 6 H) 1.36 (t, *J*=7.22 Hz, 3 H) 1.57 (t, 2 H) 2.50 (s, 2 H) 2.83 (t, 2 H) 4.17 (q, 2 H) 8.03 (d, *J*=8.59 Hz, 2 H) 8.27 (d, *J*=8.20 Hz, 2 H)

### EXAMPLE 6

### 5-(1-Ethyl-6,6-dimethyl-4,5,6,7-tetrahydro-1H-indazol-3-yl)-3-(pyridin-4-yl)-1,2,4-oxadiazole

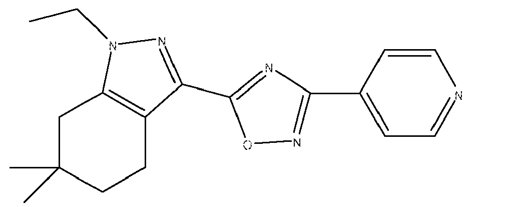

Obtained (55% yield) from Preparation 14 and Preparation 104 following the General Method 2.
LRMS: m/z 324 (M+1)⁺
Retention time: 18.29 min (Method C)
¹H NMR (200 MHz, CDCl₃) δ ppm 1.1 (s, 6 H) 1.5 (t, *J*=7.3 Hz, 3 H) 1.6 (m, 2 H) 2.4 (s, 2 H) 2.9 (t, *J*=6.4 Hz, 2 H) 4.2 (q, *J*=7.3 Hz, 2 H) 8.1 (d, *J*=5.9 Hz, 2 H) 8.8 (d, *J*=5.9 Hz, 2 H).

### EXAMPLE 7

### 3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid

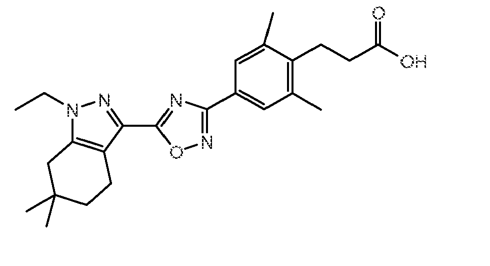

Obtained (83% yield) from Preparation 105 following the General Method 3. The desired acid product was redissolved in dioxane and 2N NaOH was added and the mixture stirred at 40°C overnight. Dioxane was concentrated and diethyl ether was added and the solid formed filtered to give the title compound as a sodium salt (83% yield).
LRMS: m/z 423 (M+1)⁺
Retention time: 19.85 min (Method C)
¹H NMR (200 MHz, DMSO-D6) d ppm 1.0 (s, 6 H) 1.4 (t, *J*=7.2 Hz, 3 H) 1.6 (t, *J*=5.9 Hz, 2 H) 2.4 (m, 6 H) 2.5 (m, *J*=2.0 Hz, 4 H) 2.9 (m, 4 H) 4.2 (q, *J*=7.3 Hz, 2 H) 7.7 (s,1 H) 12.3 (s, 1 H)

### EXAMPLE 8

### 3-(4-(4-(5-(1-ethyl-6,6-dimethyl-4,5,6,7-tetrahydro-1H-indazol-3-yl)-1,2,4-oxadiazol-3-yl)phenyl)piperazin-1-yl)propanoic acid

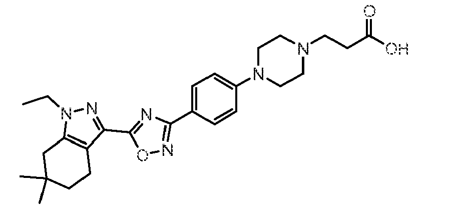

Obtained (83% yield) from Preparation 106 following the General Method 3.
LRMS: m/z 479 (M+1)⁺
Retention time: 14.34 min (Method C)
¹H NMR (200 MHz, DMSO-D6) δ ppm 1.0 (m, *J*=3.9 Hz, 6 H) 1.2 (t, *J*=7.4 Hz, 3 H) 1.6 (q, 2 H) 2.4 (t, *J*=7.0 Hz, 2 H) 2.6 (m, 2 H) 2.6 (t, *J*=7.0 Hz, 2 H) 2.8 (q, *J*=7.4 Hz, 2 H) 3.2 (m, *J*=6.5, 6.5 Hz, 2 H) 3.3 (m, 8 H) 7.1 (d, *J*=8.6 Hz, 2 H) 7.9 (d, *J*=8.6 Hz, 2 H)

### EXAMPLE 9

### 4-(5-(1-Ethyl-6,6-dimethyl-4,5,6,7-tetrahydro-1H-indazol-3-yl)-1,2,4-oxadiazol-3-yl)benzoic acid

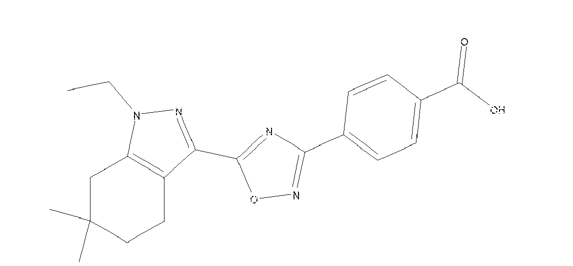

Obtained (66% yield) from Preparation 20 following the General Method 3.
LRMS: m/z 367 (M+1)⁺
Retention time: 18.70 min (Method C)
¹H NMR (300 MHz, DMSO-*d*₆) δ ppm: 1.01 (s, 6H), 1.32-1.37 (t, 3H), 1.54-1.58 (t, 2H), 2.79-2.83 (t, 2H), 4.12-4.18 (q, 2H), 8.11-8.14 (d, 2H), 8.17-8.20 (d, 2H)

### EXAMPLE 10

### 4-(5-(1-Ethyl-6,6-dimethyl-4,5,6,7-tetrahydro-1H-indazol-3-yl)-1,2,4-oxadiazol-3-yl)benzamide

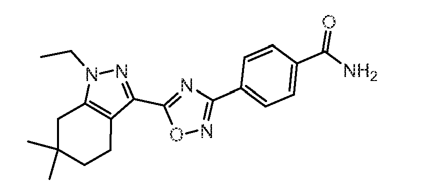

Obtained (72% yield) from Example 9 following the General Method 5.
LRMS: m/z 366 (M+1)⁺
Retention time: 16.82 min (Method C)

### EXAMPLE 11

### 5-(1-Ethyl-6,6-dimethyl-4,5,6,7-tetrahydro-1H-indazol-3-yl)-3-(4-(piperazin-1-yl)phenyl)-1,2,4-oxadiazole

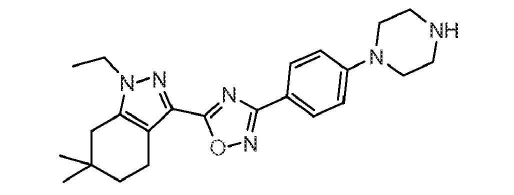

Preparation 21 (20mg, 0.04mmol) was dissolved in DCM (2ml) and trifluoroacetic acid was added (148 I, 1.91mmol). The reaction mixture was stirred at r.t. for 2h. Solvents were concentrated and the residue crystallized in diethyl ether. The title compound was obtained as a trifluoroacetic acid salt (yield=86%).
LRMS: m/z 407 (M+1)⁺
Retention time: 12.68 min (Method C)
¹H NMR (300 MHz, DMSO-D6) d ppm 1.0 (s, 6 H) 1.3-1.37 (t, 3 H) 1.51-1.6 (m, 2 H) 2.73-2.84 (m, 2 H) 3.15-3.24 (m, 6 H) 3.44-3.55 (m, 4 H) 4.11-4.24 (q, 2 H) 7.1 (d, 2 H) 7.9 (d, 2 H)

### EXAMPLE 12

### 2-(4-(5-(1-Ethyl-6,6-dimethyl-4,5,6,7-tetrahydro-1H-indazol-3-yl)-1,2,4-oxadiazol-3-yl)phenyl)acetic acid

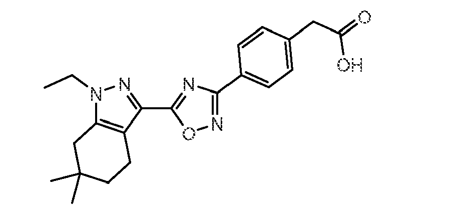

Obtained (77% yield) from Preparation 24 following the General Method 3.
LRMS: m/z 381 (M+1)⁺
Retention time: 18.11 min (Method C)
¹H NMR (300 MHz, CD₃OD) d ppm 1.09 (s, 6 H) 1.43 (m, 3 H) 1.65 (m, 2 H) 2.50 (m, 2 H) 2.91 (m, 2 H) 3.58 (m, 2 H) 4.19 (m, 2 H) 7.48 (m, 2 H) 8.04 (m, 2 H)

### EXAMPLE 13

### 1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid

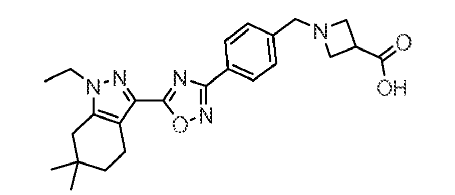

A mixture of Preparation 27 (34mg, 0.1 mmol), azetidine-3-carboxylic acid (12mg, 0.12mmol) and acetic acid (0.9mmol) in methanol (3ml) was stirred at room temperature for 30min and then NaBH3CN (6mg, 0.08mmol) was added. The reaction mixture was stirred overnight at r.t.. Solvent was removed under reduced pressure and the residue purified according to General Purification Method. Yield=47%.
LRMS: m/z 436 (M+1)⁺
Retention time: 14.21 min (Method C)
¹H NMR (300 MHz, CD₃OD) d ppm 1.09 (s, 6 H) 1.24 - 1.34 (m, 2 H) 1.43 (t, 3 H) 1.58 - 1.71 (m, 2 H) 2.50 (s, 2 H) 2.86 - 2.98 (m, 2 H) 4.06 - 4.25 (m, 4 H) 4.35 (s,2H) 7.63 (d,2H) 8.21 (d, 2 H)

### EXAMPLE 14

### 2-(4-(5-(1-Ethyl-6,6-dimethyl-4,5,6,7-tetrahydro-1H-indazol-3-yl)-1,2,4-oxadiazol-3-yl)phenyl)acetamide

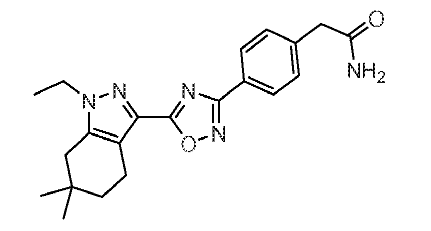

Obtained (57% yield) from Example 12 following the General Method 5.
LRMS: m/z 380 (M+1)⁺
Retention time: 16.76 min (Method C)
¹H NMR (300 MHz, CD₃OD) δ ppm 1.08 (s, 6 H) 1.47 (t, 2 H) 1.54 - 1.68 (m, 2 H) 2.42 (s, 2 H) 2.91 (t, 2 H) 3.67 (s, 2 H) 4.19 (q, 3 H) 7.43 (d, 2 H) 8.23 (d, 2 H)

### EXAMPLE 15

### 4-(5-(1-Ethyl-6,6-dimethyl-4,5,6,7-tetrahydro-1H-indazol-3-yl)-1,2,4-oxadiazol-3-yl)pyridine 1-oxide

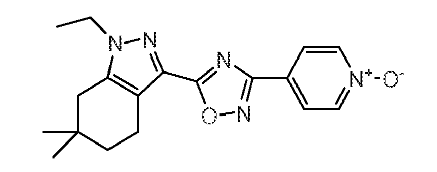

To a solution of example 6 (260mg, 0.8mmol) in DCM (10ml) at 0°C was added portionwise mCPBA (350mg, 2.03mmol). The reaction mixture was then stirred at r.t. overnight. The organic layer was washed twice with water, twice with 4% solution of NaHCO₃, once with brine and dried over magnesium sulphate. Solvent was removed under reduced pressure and the oil obtained mixed with diethyl ether. A solid precipitated as the title compound. Yield=66%.
LRMS: m/z 339 (M+1)⁺
Retention time: 15.50 min (Method C)
¹H NMR (300 MHz, DMSO-d6) d ppm 1.01 (s, 6H) 1.35 (t, J=7.14 Hz, 3H) 1.56 (t, J=6.18 Hz, 2H) 2.44 - 2.56 (m, 2H) 2.80 (t, J=6.32 Hz, 2H) 4.16 (d, J=7.14 Hz, 2H) 8.01 (d, J=7.14 Hz, 2H) 8.38 (d, J=7.14 Hz, 2H)

### EXAMPLE 16

### N-(4-(5-(1-Ethyl-6,6-dimethyl-4,5,6,7-tetrahydro-1H-indazol-3-yl)-1,2,4-oxadiazol-3-yl)phenyl)piperidin-4-amine

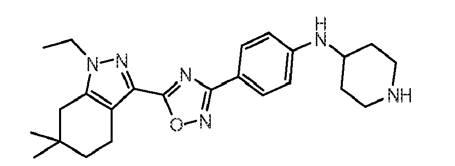

Obtained from Preparation 29 following the experimental procedure described for example 11.
LRMS: m/z 421 (M+1)⁺
Retention time: 13.19 min (Method C)
¹H NMR (300 MHz, CDCl₃) d ppm 1.1 (s, 6 H) 1.5 (q, 3 H) 1.6 (t, 2 H) 1.66-1.68 (m, 4 H) 2.05-2.19 (m, 2 H) 2.4 (s, 1 H) 2.73-2.81 (m, 2 H) 2.86-2.92 (m, 2 H) 3.2 (d, 2 H) 3.44-3.52 (m, 1 H) 3.86-3.96 (m, 1 H) 4.2 (t, 2 H) 6.6 (d, 2 H) 8.0 (d, 2 H)

### EXAMPLE 17

### 5-(1-Ethyl-6,6-dimethyl-4,5,6,7-tetrahydro-1H-indazol-3-yl)-3-(6-methoxypyridin-3-yl)-1,2,4-oxadiazole

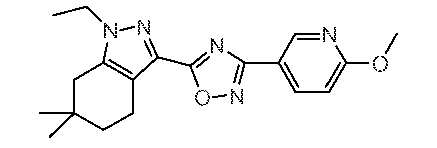

Obtained (41% yield) from Preparation 14 and Preparation 32 following the General Method 2.
LRMS: m/z 354 (M+1)⁺
Retention time: 20.10 min (Method C)
¹H NMR (300 MHz, CDCl₃) d ppm 1.00 - 1.13 (m, 6H) 1.45 (t, 3 H) 1.69 (m, 2 H) 2.34 - 2.47 (s, 2 H) 2.89 (t, 2 H) 4.08 (s, 3 H) 4.18 (q, 2 H) 6.84 (d, 1 H) 8.34 (d, 1 H) 8.97 - 9.06 (m, 1 H)

### EXAMPLE 18

### 5-(5-(1-Ethyl-6,6-dimethyl-4,5,6,7-tetrahydro-1H-indazol-3-yl)-1,2,4-oxadiazol-3-yl)pyridin-2-ol

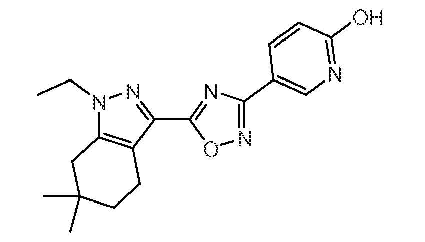

To a mixture of example 17 (100mg, 0.28mmol) and sodium iodide (127mg, 0.85mmol) in acetonitrile (4ml) under nitrogen atmosphere, was added TMSCI (107 I, 0.85mmol) dropwise. The reaction mixture was stirred overnight at 65°C. Water was added and extracted twice with DCM. The organic layer was dried and concentrated. The residue was purified by column chromatography using a mixture of MeOH in DCM (from 0 to 5%) to give the title compound as a white solid. Yield=52%.
LRMS: m/z 340 (M+1)⁺
Retention time: 15.95 min (Method C)
¹H NMR (300 MHz, CDCl₃) d ppm 1.07 (s, 6 H) 1.46 (t, J=7.3 Hz, 3 H) 1.65 (m, 2 H) 2.41 (s, 2 H) 2.87 (t, 2 H) 4.18 (q, J=7.3 Hz, 2 H) 6.70 (d, J=9.61 Hz, 1 H) 8.17 (dd, J=9.61, 2.47 Hz, 1 H) 8.34 (d, J=2.47 Hz, 1 H)

### EXAMPLE 19

### 4-(5-(1-Ethyl-6,6-dimethyl-4,5,6,7-tetrahydro-1H-indazol-3-yl)-1,2,4-oxadiazol-3-yl)pyridin-2-ol

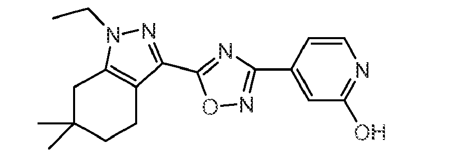

To a solution of example 15 (130mg, 0.38mmol) in THF (2ml) at 0°C was added triethylamine (0.16ml, 1.15mmol) and trifluoroacetic anhydride (0.53ml, 5.75mmol). The reaction mixture was stirred for 30min and then poured over a 4% solution of NaHCO₃, ice and ethyl acetate. The organic layer was separated, dried and concentrated. The crude was purified according to General Purification Method to give the title compound (yield=27%).
LRMS: m/z 340 (M+1)⁺
Retention time: 15.92 min (Method C)
¹H NMR (300 MHz, CDCl₃) δ ppm 1.10 (m, 6 H) 1.47 (t, J=7.2 Hz, 3 H) 1.62 - 1.68 (m, 2 H) 2.42 (s, 2 H) 2.89 (t, 2 H) 4.20 (q, J=7.2 Hz, 2 H) 7.01 (m, 1 H) 7.43 - 7.54 (m, 2 H)

### EXAMPLE 20

### 3-(4-(5-(1-ethyl-6,6-dimethyl-4,5,6,7-tetrahydro-1H-indazol-3-yl)-1,2,4-oxadiazol-3-yl)-2,6-dimethylphenyl)propanamide

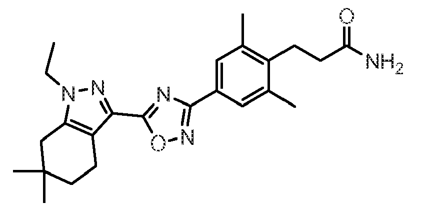

Obtained (77% yield) from Example 7 following the General Method 5.
LRMS: m/z 422 (M+1)⁺
Retention time: 18.52 min (Method C)
¹H NMR (300 MHz, CDCl₃) δ ppm: 1.07 (s, 6H), 1.43-1.47 (t, 3H), 1.60-1.64 (t, 2H), 2.27-2.44 (m, 2H), 2.41 (s, 6H), 2.89-2.93 (t, 2H), 3.03-3.09 (m, 4H), 4.15-4.22 (q, 2H), 7.88 (s, 1 H).

### EXAMPLE 21

### 4-(5-(1-Benzyl-6,6-dimethyl-4,5,6,7-tetrahydro-1H-indazol-3-yl)-1,2,4-oxadiazol-3-yl)benzamide

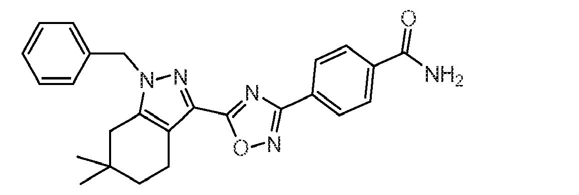

Obtained (50% yield) from Preparation 33 following the General Method 5.
LRMS: m/z 428 (M+1)⁺
Retention time: 18.61 min (Method C)
¹H NMR (300 MHz, CDCl₃) δ ppm 1.0 (s, 6 H) 1.6 (m, 2 H) 2.3 (s, 2 H) 2.9 (t, 2 H) 5.4 (s, 2 H) 5.61-5.76 (m, 1 H) 6.07-6.22 (m, 1 H) 7.1 (d, 2 H) 7.31-7.36 (m, 3 H) 7.9 (d,2H) 8.3 (d, 2 H)

### EXAMPLE 22

### 4-(5-(1-tert-butyl-6,6-dimethyl-4,5,6,7-tetrahydro-1H-indazol-3-yl)-1,2,4-oxadiazol-3-yl)benzamide

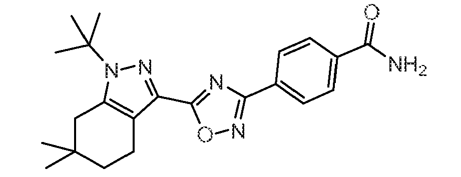

Obtained (67% yield) from Preparation 43 following the General Method 5.
LRMS: m/z 394 (M+1)⁺
Retention time: 18.33 min (Method C)
¹H NMR (300 MHz, CDCl₃) δ ppm 1.1 (s, 6 H) 1.6 (s, 2 H) 1.69-1.79 (s, 9 H) 2.66-2.74 (m, 2 H) 2.92-2.99 (m, 2 H) 5.68-5.75 (m, 1 H) 6.07-6.22 (m, 1 H) 7.9 (d,2 H) 8.3 (d, 2 H)

### EXAMPLE 23

### 5-(1-Ethyl-6,6-dimethyl-4,5,6,7-tetrahydro-1H-indazol-3-yl)-3-(2-methoxypyridin-4-yl)-1,2,4-oxadiazole

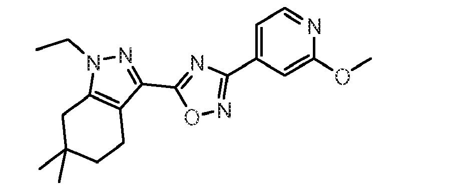

Obtained (51% yield) from example 19 following the experimental procedure described for Preparation 50.
LRMS: m/z 354 (M+1)⁺
Retention time: 19.77 min (Method C)
¹H NMR (300 MHz, CD₃OD) d ppm 1.09 (s, 6 H) 1.44 (t, J=7.2 Hz, 3 H) 1.60 (m, 2 H) 2.50 (s, 2 H) 2.84 (m, 2 H) 3.99 (s, 3 H) 4.19 (q, J=7.2 Hz, 2 H) 7.5 (m, 1 H) 7.6 (m, 1 H) 8.3 (m, 1 H)

### EXAMPLE 24

### 4-(5-(1-Ethyl-6,6-dimethyl-4,5,6,7-tetrahydro-1H-indazol-3-yl)-1,2,4-oxadiazol-3-yl)-1-methylpyridin-2(1H)-one

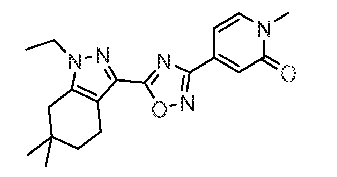

Obtained (19% yield) from example 19 following the experimental procedure described for Preparation 50.
LRMS: m/z 354 (M+1)⁺
Retention time: 15.51 min (Method C)
¹H NMR (300 MHz, CD₃OD) δppm 1.08 (s, 6 H) 1.45 (m, 3 H) 1.65 (m, 2 H) 2.50 (s, 2 H) 2.85 (m, 2 H) 3.64 (s, 3 H) 4.2 (m, 2 H) 7.01 (d, 1 H) 7.3 (m, 1 H) 7.82 (d, 1 H)

### EXAMPLE 25

### 1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid

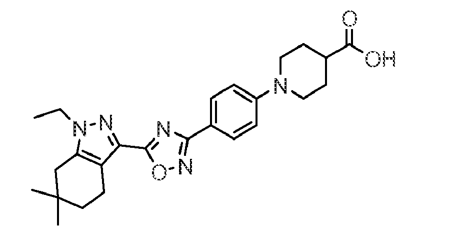

Obtained (81%) from Preparation 107 following the General Method 3.
LRMS: m/z 450 (M+1)⁺
Retention time: 19.15 min (Method C)

### EXAMPLE 26

### (4-(5-(1-ethyl-6,6-dimethyl-4,5,6,7-tetrahydro-1H-indazol-3-yl)-1,2,4-oxadiazol-3-yl)phenyl)methanol

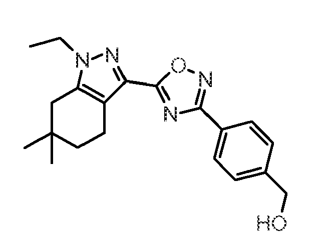

Obtained (63% yield) from Preparation 14 and Preparation 1 following the General Method 2.
LRMS: m/z 353 (M+1)⁺
Retention time: 18.02 min (Method C)
¹H NMR (200 MHz, DMSO-D6) δ ppm 1.0 (s, 6 H) 1.4 (t, *J*=7.3 Hz, 3 H) 1.6 (t, *J*=6.2 Hz, 2 H) 2.5 (d, *J*=1.6 Hz, 2 H) 2.8 (t, *J*=6.1 Hz, 2 H) 4.2 (q, *J*=7.3 Hz, 2 H) 4.6 (s, 2 H) 5.4 (s, 1 H) 7.5 (d, *J*=8.2 Hz, 2 H) 8.0 (d, *J*=8.2 Hz, 2 H)

### EXAMPLE 27

### 4-(5-(6,6-Dimethyl-1-phenyl-4,5,6,7-tetrahydro-1H-indazol-3-yl)-1,2,4-oxadiazol-3-yl)benzamide

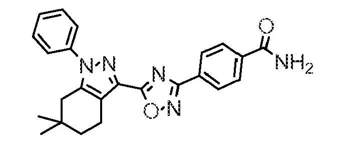

Obtained (61% yield) from Preparation 45 following the General Method 5.
LRMS: m/z 414 (M+1)⁺
Retention time: 19.14 min (Method C)
¹H NMR (300 MHz, CDCl₃) δ ppm 1.08 (s, 6H) 1.70 (m, 2H) 2.55 (m, 2H) 3.02 (s, 2H) 7.36-7.57 (m, 5H) 7.94-7.96 (m, 2H), 8.31-8.34 (m, 2H)

### EXAMPLE 28

### 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol

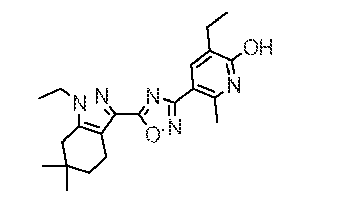

Obtained (75% yield) from Preparation 47 following the experimental procedure described for example 18.
LRMS: m/z 382 (M+1)⁺
Retention time: 18.69 min (Method C)
¹H NMR (300 MHz, CD₃OD) δ ppm 1.08 (s, 6 H) 1.23 (t, 3 H) 1.43 (t, 3 H) 1.65 (t, 2 H) 2.47 - 2.53 (m, 2 H) 2.58 (q, 2 H) 2.65 - 2.74 (m, 3 H) 2.88 (t, 2 H) 4.19 (q, 2 H) 7.98 - 8.09 (m, 1 H)

### EXAMPLE 29

### 5-(5-(1-Ethyl-6,6-dimethyl-4,5,6,7-tetrahydro-1H-indazol-3-yl)-1,2,4-oxadiazol-3-yl)-3-methylpyridin-2-ol

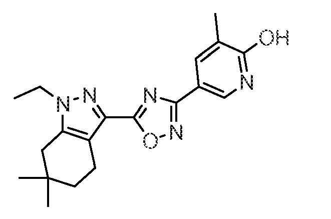

Obtained (62% yield) from Preparation 53 following the experimental procedure described for example 18.
LRMS: m/z 354 (M+1)⁺
Retention time: 17.28 min (Method C)
¹H NMR (300 MHz, CDCl₃) δ ppm 1.08 (s, 6 H) 1.46 (t, J=7.28 Hz, 3 H) 1.6 (m, 2 H) 2.25 (s, 3 H) 2.41 (s, 2 H) 2.88 (t, 2 H) 4.18 (q, J=7.28 Hz, 2 H) 8.05 (s, 1 H) 8.25 (s, 1 H)

### EXAMPLE 30

### 4-(5-(1-Ethyl-6,6-dimethyl-4,5,6,7-tetrahydro-1H-indazol-3-yl)-1,2,4-oxadiazol-3-yl)-3-methylbenzamide

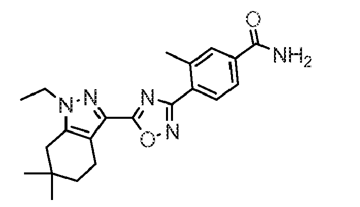

Obtained (75% yield) from Preparation 57 following the General Method 5.
LRMS: m/z 380 (M+1)⁺
Retention time: 17.55 min (Method C)
¹H NMR (300 MHz, CDCl₃) δ ppm 1.08 (s, 6 H) 1.41 - 1.52 (m, 2 H) 1.54 - 1.69 (m, 2.06 Hz, 3 H) 2.42 (s, 2 H) 2.75 (s, 3 H) 2.84 - 2.97 (m, 2 H) 4.11 - 4.27 (m, 2 H) 7.66 - 7.76 (m, 1 H) 7.77 - 7.86 (m, 1 H) 8.20 - 8.31 (m, 1 H)

### EXAMPLE 31

### 4-(4-(5-(1-Ethyl-6,6-dimethyl-4,5,6,7-tetrahydro-1H-indazol-3-yl)-1,2,4-oxadiazol-3-yl)benzyl)morpholine

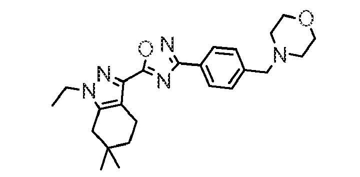

Obtained (37% yield) from Preparation 27 and morpholine following the experimental procedure described for example 13.
LRMS: m/z 422 (M+1)⁺
Retention time: 12.77 min (Method C)
¹H NMR (200 MHz, DMSO-D6) δ ppm 1.0 (s, 6 H) 1.4 (t, *J*=7.1 Hz, 3 H) 1.6 (m, 2 H) 2.4 (m, 4 H) 2.5 (s, 2 H) 2.8 (s, 2 H) 3.3 (s, 2 H) 3.6 (m, 4 H) 4.2 (q, *J*=7.1 Hz, 2 H) 7.5 (d, *J*=8.0 Hz, 2 H) 8.0 (d, *J*=8.0 Hz, 2 H)

### EXAMPLE 32

### 3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one

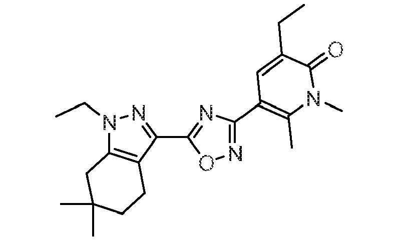

Obtained (87% yield) from example 28 following the experimental procedure described for Preparation 50 using Cs₂CO₃ as a base and DMF as a solvent.
LRMS: m/z 396 (M+1)⁺
Retention time: 19.06 min (Method C)
¹H NMR (300 MHz, CDCl₃) δ ppm 1.08 (s, 6 H) 1.19 - 1.26 (m, 3 H) 1.47 (t, J=7.28 Hz, 2 H) 1.62 (t, J=6.45 Hz, 3 H) 2.42 (s, 3 H) 2.61 (q, J=7.42 Hz, 2 H) 2.77 (s, 2 H) 2.88 (t, J=6.32 Hz, 2 H) 3.67 (s, 3 H) 4.19 (q, J=7.14 Hz, 2 H) 7.84 (s, 1 H)

### EXAMPLE 33

### 5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one

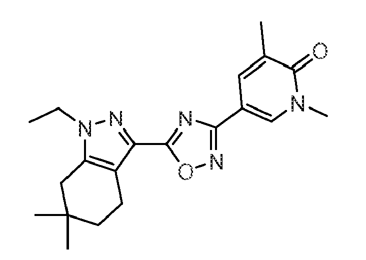

Obtained (76% yield) from example 29 following the experimental procedure described for Preparation 50 using Cs₂CO₃ as a base and DMF as a solvent.
LRMS: m/z 368 (M+1)⁺
Retention time: 18.06 min (Method C)
¹H NMR (300 MHz, CDCl₃) δ ppm 1.08 (s, 6 H) 1.46 (t, J=7.28 Hz, 3 H) 1.62 (t, J=6.32 Hz, 2 H) 2.23 (s, 3 H) 2.42 (s, 2 H) 2.89 (t, J=6.45 Hz, 2 H) 3.64 (s, 3 H) 4.19 (q, J=7.42 Hz, 2 H) 7.91 - 7.96 (m, 1 H) 8.24 - 8.28 (m, 1 H)

### EXAMPLE 34

### N-Cyclopropyl-4-(5-(1-ethyl-6,6-dimethyl-4,5,6,7-tetrahydro-1H-indazol-3-yl)-1,2,4-oxadiazol-3-yl)benzamide

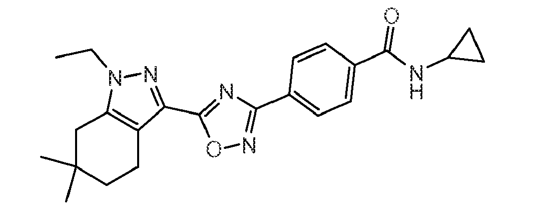

Obtained (45% yield) from Example 9 and cyclopropylamine following the General Method 5.
LRMS: m/z 406 (M+1)⁺
Retention time: 18.43 min (Method C)
¹H NMR (300 MHz, CDC1₃) δ ppm 0.66 (m, 2 H) 0.92 (m, 2 H) 1.08 (s, 6 H) 1.43 - 1.50 (m, 3 H) 1.61 - 1.67 (m, 2 H) 2.42 (s, 2 H) 2.85 - 2.97 (m, 3 H) 4.15 - 4.25 (m, 2 H) 7.86 (d, J=8.24 Hz, 2 H) 8.28 (d, J=8.24 Hz, 2 H)

### EXAMPLE 35

### 1-(4-(5-(1-Ethyl-6,6-dimethyl-4,5,6,7-tetrahydro-1H-indazol-3-yl)-1,2,4-oxadiazol-3-yl)phenyl)-N-methylmethanamine

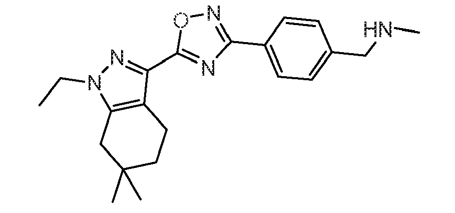

Obtained (34% yield) from Preparation 27 and methylamine following the experimental procedure described for example 13.
LRMS: m/z 366 (M+1)⁺
Retention time: 12.21 min (Method C)
¹H NMR (200 MHz, DMSO-D6) δ ppm 1.0 (s, 6 H) 1.4 (m, 3 H) 1.6 (t, *J*=6.1 Hz, 2 H) 2.6 (m, 2 H) 2.8 (m, 2H) 3.4 (s, 3 H) 4.2 (m, 4 H) 7.7 (d, *J*=8.2 Hz, 2 H) 8.1 (d, *J*=8.2 Hz, 2 H) 9.2 (s, 2 H)

### EXAMPLE 36

### 5-(1-Ethyl-6,6-dimethyl-4,5,6,7-tetrahydro-1H-indazol-3-yl)-3-(3-methylpyridin-4-yl)-1,2,4-oxadiazole

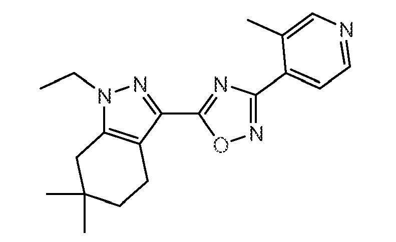

Obtained (9% yield) from Preparation 14 and Preparation 61 following the General Method 2.
LRMS: m/z 338 (M+1)⁺
Retention time: 19.09 min (Method C)
¹H NMR (300 MHz, CDCl₃) δ ppm 1.08 (s, 6 H) 1.47 (t, J=7.28 Hz, 3 H) 1.63 (t, J=6.32 Hz, 2 H) 2.42 (s, 2 H) 2.69 (s, 3 H) 2.91 (t, J=6.18 Hz, 2 H) 4.18 (q, J=7.14 Hz, 2 H) 8.06 (d, J=4.94 Hz, 1 H) 8.58 - 8.65 (m, 2 H)

### EXAMPLE 37

### 4-(5-(6,6-Dimethyl-1-(2,2,2-trifluoroethyl)-4,5,6,7-tetrahydro-1H-indazol-3-yl)-1,2,4-oxadiazol-3-yl)benzamide

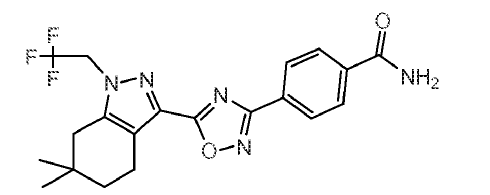

Obtained (9% yield) from Preparation 63 following the General Method 5.
LRMS: m/z 420 (M+1)⁺
Retention time: 17.51 min (Method C)
¹H NMR (300 MHz, CDCl₃) δ ppm 1.08 (s, 6H) 1.65 (m, 2H) 2.45 (s, 2H) 2.92-2.95 (m, 2H) 4.73-4.76 (m, 2H) 7.93-7.96 (m, 2H) 8.30-8.33 (m, 3H)

### EXAMPLE 38

### (4-(5-(1-Ethyl-6,6-dimethyl-4,5,6,7-tetrahydro-1H-indazol-3-yl)-1,2,4-oxadiazol-3-yl)phenyl)methanamine

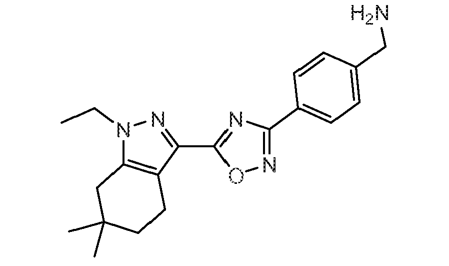

To a solution of Preparation 27 (200mg, 0.57mmol) in methanol (6ml) was added hydroxylamine.hydrochloride (40,g, 0.58mmol) in water (1ml) and the reaction mixture was stirred at r.t. overnight. Solvent was removed and the residue redissolved in ethyl acetate and washed with 0.1N HCl, 4% solution of NaHCO₃ and brine. The organic layer was dried and concentrated. The solid obtained was dissolved in AcOH (6ml) and zinc dust (80mg, 1.2mmol) was added, the mixture was stirred at r.t. overnight under argon atmosphere. Methanol was added and the mixture filtered over Decalite. The filtrate was concentrated and dissolved in ethyl acetate. The organic layer was washed with 2N NaOH, water and brine, then dried and concentrated. The oil obtained was redissolved in 4M HCl in dioxane and stirred at r.t. overnight. The precipitate was filtered and washed with diethyl ether to yield the final compound as hydrochloride salt. Yield=51%.
LRMS: m/z 352 (M+1)⁺
Retention time: 12.58 min (Method C)
¹H NMR (200 MHz, DMSO-D6) δ ppm 1.0 (s, 6 H) 1.4 (t, *J*=7.3 Hz, 3 H) 1.6 (t, *J*=6.2 Hz, 2 H) 2.5 (m, 2 H) 2.8 (m, 2 H) 4.2 (d, *J*=7.3 Hz, 4 H) 7.7 (d, *J*=8.2 Hz, 2 H) 8.1 (d, *J*=8.2 Hz, 2 H) 8.4 (s, 3 H)

### EXAMPLE 39

### 5-(1-Ethyl-6,6-dimethyl-4,5,6,7-tetrahydro-1H-indazol-3-yl)-3-(3-methylpyridin-2-yl)-1,2,4-oxadiazole

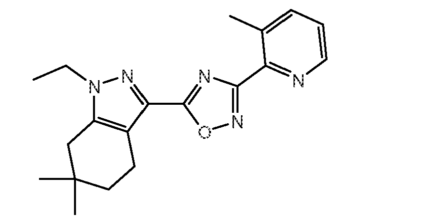

Obtained (33% yield) from Preparation 14 and Preparation 65 following the General Method 2.
LRMS: m/z 338 (M+1)⁺
Retention time: 17.57 min (Method C)
¹H NMR (300 MHz, CD₃OD) d ppm 1.03 - 1.15 (m, 6 H) 1.39 - 1.50 (m, 3H) 1.60 - 1.73 (m, 2 H) 2.48 - 2.58 (m, 2 H) 2.83 - 3.00 (m, 5 H) 4.17 - 4.29 (m, 2 H) 7.98 - 8.12 (m, 1 H) 8.51 - 8.63 (m, 1 H) 8.74 - 8.89 (m, 1 H)

### EXAMPLE 40

### 2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine

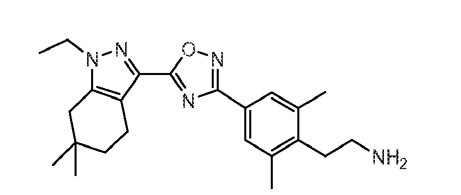

Obtained (35% yield) from Example 7 following the General Method 6.
LRMS: m/z 394 (M+1)⁺
Retention time: 13.38 min (Method C)
¹H NMR (400 MHz, DMSO-D6) d ppm 1.0 (s, 6 H) 1.4 (t, *J*=7.2 Hz, 3 H) 1.6 (t, *J*=6.1 Hz, 2 H) 2.4 (s, 6 H) 2.5 (s, 2 H) 2.8 (m, 4 H) 3.0 (m, 2 H) 4.2 (q, *J*=7.4 Hz, 2 H) 7.7 (s, 2 H) 8.1 (s, 3 H)

### EXAMPLE 41

### 5-(5-(1-Ethyl-6,6-dimethyl-4,5,6,7-tetrahydro-1H-indazol-3-yl)-1,2,4-oxadiazol-3-yl)-4-methylpyridin-2-ol

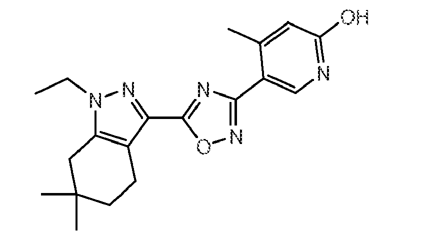

Preparation 66 was disolved in TFA and the mixture stirred at 45°C for 1.5h. Solvent was removed under vacuum and the residue redisolved in 1 N NaOH and extracted with chloroform. The organic layer was dried and concentrated. The residue was purified according to the General Purification Method to give the title compound as a white solid. Yield=89%.
LRMS: m/z 354 (M+1)⁺
Retention time: 16.60 min (Method C)
¹H NMR (300 MHz, DMSO-d6) d ppm 0.99 - 1.03 (s, 6 H) 1.34 (t, 3 H) 1.54 (t, 2 H) 2.45 (s, 3 H) 2.5 (m, 2 H) 2.76 (t, 2 H) 4.15 (q, 2 H) 6.36 (m, 1 H) 8.09 (m, 1 H)

### EXAMPLE 42

### 3-(4-(5-(1-Ethyl-6,6-dimethyl-4,5,6,7-tetrahydro-1H-indazol-3-yl)-1,2,4-oxadiazol-3-yl)-3-methylphenyl)propanoic acid

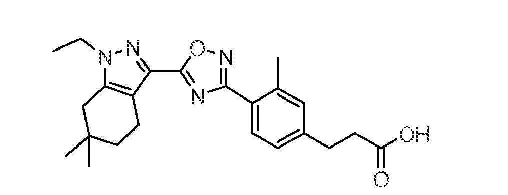

Obtained (92% yield) from Preparation 110 following the General Method 3.
LRMS: m/z 409 (M+1)⁺
Retention time: 19.11 min
¹H NMR (400 MHz, DMSO-D6) d ppm 1.0 (s, J=10.6 Hz, 6 H) 1.4 (t, *J*=7.2 Hz, 3 H) 1.6 (t, *J*=6.1 Hz, 2 H) 2.5 (m, *J*=30.1 Hz, 2 H) 2.6 (m, 4 H) 2.8 (m, 4 H) 3.2 (s, 1 H) 4.2 (q, *J*=6.8 Hz, 2 H) 7.3 (m, 2 H) 7.9 (d, J=7.8 Hz, 1 H) 12.2 (s, 1 H)

### EXAMPLE 43 5-(5-(1-Ethyl-6,6-dimethyl-4,5,6,7-tetrahydro-1H-indazol-3-yl)-1,2,4-oxadiazol-3-yl)-6-methylpyridin-2-ol

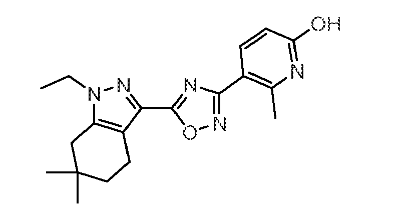

Obtained (65% yield) from Preparation 70 following the experimental procedure described for example 18.
LRMS: m/z 354 (M+1)⁺
Retention time: 16.44 min (Method C)
¹H NMR (300 MHz, CDCl₃) δ ppm 1.08 (s, 6 H) 1.46 (t, J=7.14 Hz, 3 H) 1.62 (t, J=6.32 Hz, 2 H) 2.41 (s, 2 H) 2.81 (s, 3 H) 2.88 (t, J=6.32 Hz, 2 H) 4.18 (q, J=7.42 Hz, 2 H) 6.55 (d, J=9.61 Hz, 1 H) 8.27 (d, J=9.61 Hz, 1 H)

### EXAMPLE 44

### 5-(1-Ethyl-6,6-dimethyl-4,5,6,7-tetrahydro-1H-indazol-3-yl)-3-(4-methylpyridin-3-yl)-1,2,4-oxadiazole

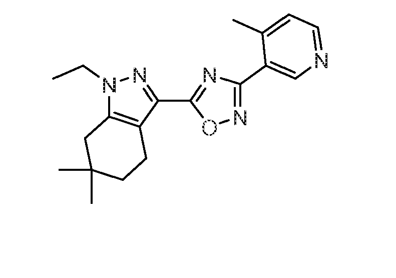

Obtained (7% yield) from Preparation 14 and Preparation 74 following the General Method 2.
LRMS: m/z 338 (M+1)⁺
Retention time: 18.28 min (Method C)
¹H NMR (300 MHz, CDCl₃) δ ppm 1.08 (s, 6 H) 1.43 - 1.51 (m, 3 H) 1.61 - 1.66 (m, 2 H) 2.42 (s, 2 H) 2.71 (s, 3 H) 2.90 - 2.94 (m, 2 H) 4.14 - 4.23 (m, 2 H) 7.27 - 7.28 (m, 1 H) 8.58 (d, J=4.94 Hz, 1 H) 9.31 (s, 1 H)

### EXAMPLE 45

### 5-(1-Ethyl-6,6-dimethyl-4,5,6,7-tetrahydro-1H-indazol-3-yl)-3-(4-(trifluoromethyl)pyridin-3-yl)-1,2,4-oxadiazole

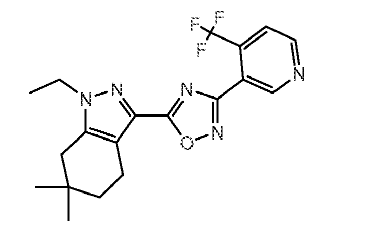

Obtained (33% yield) from Preparation 14 and Preparation 75 following the General Method 2.
LRMS: m/z 392 (M+1)⁺
Retention time: 18.88 min (Method C)
¹H NMR (300 MHz, CDCl₃) δ ppm 1.08 (s, 6 H) 1.43 - 1.51 (m, 3 H) 1.61 - 1.65 (m, 2 H) 2.42 (s, 2 H) 2.88 (t, J=6.18 Hz, 2 H) 4.19 (q, 2 H) 7.74 (d, J=5.22 Hz, 1 H) 8.96 (d, J=5.22 Hz, 1 H) 9.24 (s, 1 H)

### EXAMPLE 46

### 5-(1-Ethyl-6,6-dimethyl-4,5,6,7-tetrahydro-1H-indazol-3-yl)-3-(imidazo[1,2-a]pyridin-6-yl)-1,2,4-oxadiazole

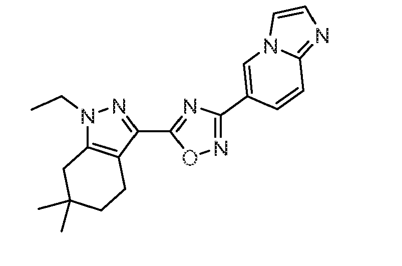

Obtained (33% yield) from Preparation 14 and Preparation 76 following the General Method 2.
LRMS: m/z 363 (M+1)⁺
Retention time: 13.38 min (Method C)
¹H NMR (300 MHz, CDCl₃) δ ppm 1.09 (s, 6 H) 1.48 (t, J=7.28 Hz, 3 H) 1.63 - 1.68 (m, 2 H) 2.43 (s, 2 H) 2.92 (t, 2 H) 4.20 (q, J=7.42 Hz, 2 H) 7.67 - 7.75 (m, 3 H) 7.90 (d, 1 H) 9.15 (s, 1 H)

### ) EXAMPLE 47

### 3-(4-(5-(1-Ethyl-6,6-dimethyl-4,5,6,7-tetrahydro-1H-indazol-3-yl)-1,2,4-oxadiazol-3-yl)-3-methylphenyl)propanamide

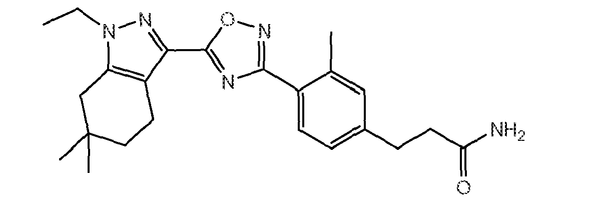

Obtained (73% yield) from Example 42 following the General Method 5.
LRMS: m/z 408 (M+1)⁺
Retention time: 17.86 min (Method C)
¹H'RMN (400 MHz, CDCl₃) δ ppm 1.1 (s, 6 H) 1.5 (t, J=7.2 Hz, 3 H) 1.6 (t, J=6.5 Hz, 2 H) 2.4 (s, 2 H) 2.6 (m, 2 H) 2.7 (s, 3 H) 2.9 (t, J=6.5 Hz, 2 H) 3.0 (t, J=7.8 Hz, 2 H) 4.2 (q, J=7.2 Hz, 2 H) 5.3 (s, 2 H) 7.2 (d, J=7.4 Hz, 2 H) 8.1 (d, J=7.8 Hz, 1 H)

### EXAMPLE 48

### 2-(4-(5-(1-Ethyl-6,6-dimethyl-4,5,6,7-tetrahydro-1H-indazol-3-yl)-1,2,4-oxadiazol-3-yl)-3-methylphenyl)ethanamine

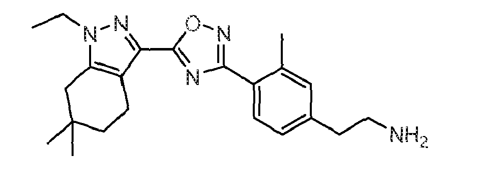

Obtained (26% yield) from Example 42 following the General Method 6.
LRMS: m/z 380 (M+1)⁺
Retention time: 13.16 min (Method C)
¹H NMR (400 MHz, DMSO-D6) δ ppm 1.0 (s, 6 H) 1.3 (t, J=7.2 Hz, 3 H) 1.5 (t, J=6.5 Hz, 2 H) 2.5 (s, J=2.0 Hz, 3 H) 2.6 (s, 2 H) 2.8 (t, 2 H) 2.9 (t, J=8.6 Hz, 2 H) 3.1 (t, J=8.6 Hz, 2 H) 4.1 (q, J=7.0 Hz, 2 H) 7.3 (d, 1 H) 7.3 (s, 1 H) 7.9 (s, 3 H) 7.9 (d, J=7.8 Hz, 1 H)

### EXAMPLE 49

### 3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine

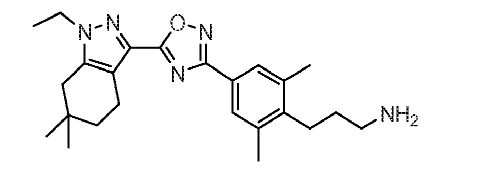

Obtained (65% yield) from example 7 following the General Method 7.
LRMS: m/z 408 (M+1)⁺
Retention time: 14.64 min (Method C)

### EXAMPLE 50

### 4-(5-(1-Ethyl-6,6-dimethyl-4,5,6,7-tetrahydro-1H-indazol-3-yl)-1,2,4-oxadiazol-3-yl)-3-(trifluoromethyl)benzoic acid

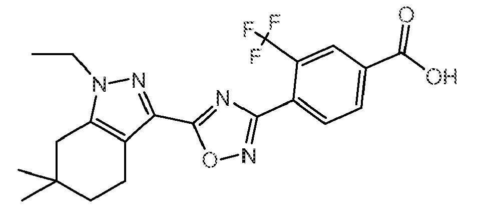

Obtained (93% yield) from Preparation 78 following the General Method 3.
LRMS: m/z 435 (M+1)⁺
Retention time: 19.02min (Method C)
¹H NMR (300 MHz, CHLOROFORM-D) δ ppm 1.1 (s, 6 H) 1.5 (m, 3 H) 1.6 (t, *J*=6.5 Hz, 2 H) 2.4 (s, 2 H) 2.9 (t, *J*=6.3 Hz, 2 H) 4.2 (q, *J*=7.1 Hz, 2 H) 8.1 (d, *J*=8.0 Hz, 1 H) 8.4 (d, *J*=8.0 Hz, 1 H) 8.6 (s, 1 H)

### EXAMPLE 51

### 4-(5-(1-Ethyl-6,6-dimethyl-4,5,6,7-tetrahydro-1H-indazol-3-yl)-1,2,4-oxadiazol-3-yl)-3-(trifluoromethyl)benzamide

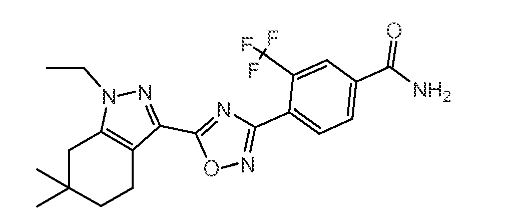

Obtained (60% yield) from Example 50 following the General Method 5.
LRMS: m/z 434 (M+1)⁺
Retention time: 17.54 min (Method C)
¹H NMR (300 MHz, CDCl₃) δ ppm 1.08 (s, 6H) 1.45-1.50 (m, 2H) 1.60-1.65 (t, 3H) 2.42 (s, 2H) 2.87-2.91 (m, 2H) 4.16-4.20 (q, 2H) 8.06-8.10 (m, 2H) 8.32 (s, 1 H)

### EXAMPLE 52

### 5-(1-Ethyl-6,6-dimethyl-4,5,6,7-tetrahydro-1H-indazol-3-yl)-3-(2-methylpyridin-3-yl)-1,2,4-oxadiazole

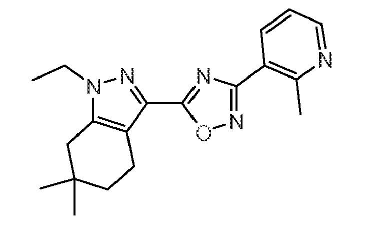

Obtained (49% yield) from Preparation 14 and Preparation 84 following the General Method 2.
LRMS: m/z 338 (M+1)⁺
Retention time: 18.05 min (Method C)
¹H NMR (300 MHz, CD₃OD) δ ppm 1.08 (s, 6 H) 1.39 - 1.48 (m, 3 H) 1.60 - 1.69 (m, 2 H) 2.50 (s, 2 H) 2.85 - 2.94 (m, 5 H) 4.14 - 4.25 (m, 2 H) 7.43 - 7.51 (m, 1 H) 8.49 (d, 1 H) 8.54 - 8.62 (m, 1 H)

### EXAMPLE 53

### 5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine

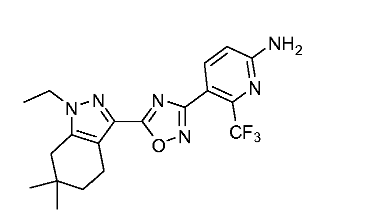

Obtained (21% yield) from Preparation 14 and Preparation 86 following the General Method 2.
LRMS: m/z 407 (M+1)⁺
Retention time: 17.83 min (Method C)
¹H NMR (300 MHz, DMSO-d6) d ppm 1.00 (s, 6 H) 1.30 - 1.37 (m, 3 H) 1.53 (t, J=6.04 Hz, 2 H) 2.45 - 2.49 (m, 2 H) 2.72 (t, J=5.36 Hz, 2 H) 4.14 (q, J=7.23 Hz, 2 H) 6.78 (d, J=8.79 Hz, 1 H) 7.08 (s, 2 H) 7.84 (d, J=8.51 Hz, 1 H)

### EXAMPLE 54

### 3-Cyclopropyl-4-[5-(1-ethyl-6,6-dimethyl-4,5,6,7-tetrahydro-1H-indazol-3-yl)-1,2,4-oxadiazol-3-yl]benzamide

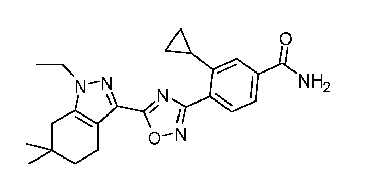

Obtained (13% yield) from Preparation 89 following the General Method 5.
LRMS: m/z 406 (M+1)⁺
Retention time: 17.97 min (Method C)
¹H NMR (300 MHz, CDCl₃) δ ppm: 0.78-0.92 (m, 4H), 1.07 (s, 6H), 1.44-1.49 (t, 3H), 1.60-1.64 (m, 2H), 2.42 (s, 2H), 2.71-2.77 (m, 1 H), 2.88-2.92 (t, 2H), 4.15-4.22 (q, 2H), 7.58 (s, 1 H), 7.63-7.65 (d, 1 H), 8.12-8.15 (d, 1 H)

### EXAMPLE 55

### 5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol

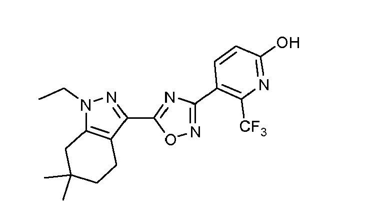

To a solution of Example 53 (50mg, 0.12mmol) in sulphuric acid (262 I, 4.92mmol) at 0°C was added sodium nitrite (7.6mg, 0.11 mol) in water (0.3ml) dropwise. The reaction mixture was stirred overnight at r.t.. Sodium hydroxide was added until neutral pH and the product extracted twice with ethyl acetate. The organic layer was dried and concentrated and the residue obtained purified by column chromatography with a mixture of DCM/MeOH. The title compound was obtained as a white solid. Yield=80%.
LRMS: m/z 407 (M+1)⁺
Retention time: 18.05 min (Method C)
¹H NMR (300 MHz, CDCl₃) δppm 1.08 (s, 6 H) 1.46 (t, 3 H) 1.62 (t, J=6.32 Hz, 2 H) 2.41 (s, 2 H) 2.87 (t, J=6.18 Hz, 2 H) 4.18 (q, J=7.14 Hz, 2 H) 6.99 (d, J=9.06 Hz, 1 H) 8.09 (d, J=9.06 Hz, 1 H)

### EXAMPLE 56

### 5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol

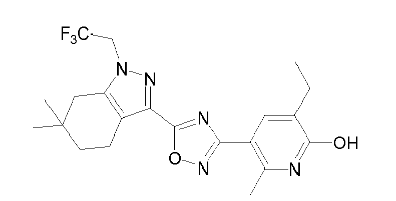

Obtained (70% yield) from Preparation 96 following the experimental procedure described for example 18.
LRMS: m/z 436 (M+1)⁺
Retention time: 18.60 min (Method C)
¹H NMR (300 MHz, CDCl₃) δ ppm: 1.08 (s, 6H), 1.24-1.28 (t, 3H), 1.63-1.67 (t, 2H), 2.44 (s, 2H), 2.58-2.64 (q, 2H), 2.75 (s, 3H), 2.89-2.93 (t, 2H), 4.70-4.78 (m, 2H), 8.04 (s, 1 H)

### EXAMPLE 57

### 3-(4-(5-(1-Ethyl-6,6-dimethyl-4,5,6,7-tetrahydro-1H-indazol-3-yi)-1,2,4-oxadiazol-3-yl)-3-(trifluoromethyl)phenyl)propanoic acid

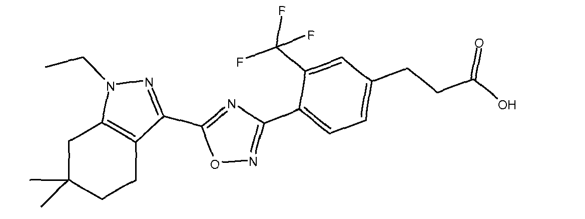

Obtained (100% yield) from Preparation 111 following the General Method 4.
LRMS: m/z 463 (M+1)⁺
Retention time: 18.76 min (Method C)
H'RMN (200 MHz, DMSO-D6) δ ppm 1.1 (s, 6 H) 1.4 (t, J=7.2 Hz, 3 H) 1.5 (t, J=6.2 Hz, 2 H) 2.8 (m, 8 H) 4.2 (q, J=7.2 Hz, 2 H) 7.8 (m, J=19.7, 8.0 Hz, 3 H) 12.2 (s, 1 H)

### EXAMPLE 58

### 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide

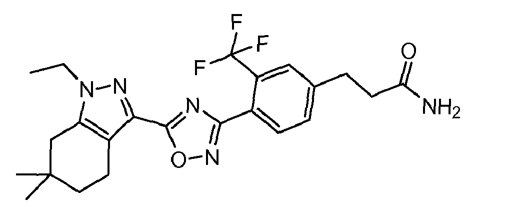

Obtained (79% yield) from example 57 following the General Method 5.
LRMS: m/z 462 (M+1)⁺
Retention time: 17.65 min (Method C)
) H'RMN (200 MHz, CDCl₃); δ ppm 1.1 (s, 6 H) 1.4 (m, 3 H) 1.6 (t, J=6.1 Hz, 2 H) 2.4 (s, 2 H) 2.6 (t, J=7.4 Hz, 2 H) 2.9 (t, J=5.9 Hz, 2 H) 3.1 (t, J=7.4 Hz, 2 H) 4.2 (q, J=7.2 Hz, 2 H) 5.4 (s, 2 H) 7.5 (d, J=8.2 Hz, 1 H) 7.7 (s, 1 H) 7.8 (d, J=7.8 Hz, 1 H)

### EXAMPLE 59

### 3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol

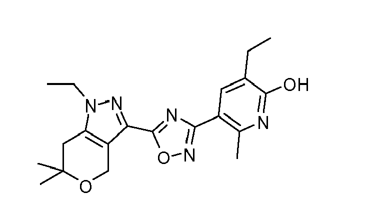

Obtained (8% yield) from Preparation 97 following the experimental procedure described for example 18.
LRMS: m/z 384 (M+1)⁺
Retention time: 15.08 min (Method C)
¹H NMR (300 MHz, CDCl₃)δ ppm 1.19-1.28 (m, 3H), 1.38 (s, 6H), 1.47-1.51 (t, 3H), 2.53-2.64 (m, 4H), 2.74 (s, 3H), 4.17-4.25 (q, 2H), 4.97 (s, 2H), 8.04 (s, 2H)

### EXAMPLE 60

### 3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine

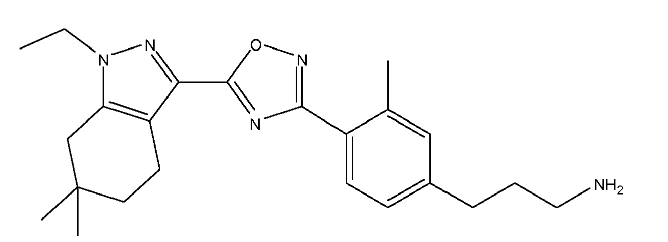

Obtained (59% yield) from Example 47 following the General Method 7.
LRMS: m/z 394 (M+1)⁺
Retention time: 13.78 min (Method C)

### EXAMPLE 61

### 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine

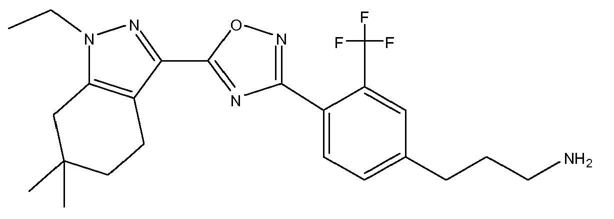

Obtained (50% yield) from Example 58 following the General Method 7.
LRMS: m/z 448 (M+1)⁺
Retention time: 13.79 min (Method C)

### EXAMPLE 62

### 6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine

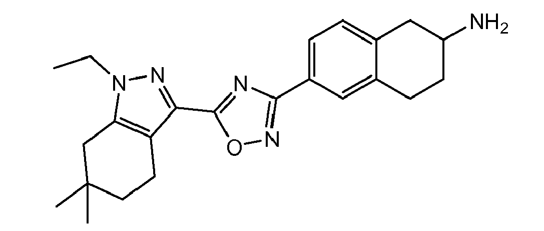

A mixture of Preparation 116 (612mg, 1.06mmol) in 4M HCl in dioxane (4ml) was stirred at r.t. overnight. Water and diethyl ether were added and layers separated. The aqueous layer was adjusted to basic pH and extracted twice with ethyl acetate. The combined organic layer was dried over magnesium sulphate and concentrated to give the desired compound (73% yield).
LRMS: m/z 391 (M+1)⁺
Retention time: 12.62 min (Method C)
¹H NMR (200 MHz, DMSO-D6) δ ppm 1.1 (m, 6 H) 1.4 (t, *J*=7.0 Hz, 3 H) 1.6 (m, 2 H) 2.0 (m, 4 H) 2.8 (m, 2 H) 3.0 (m, 3 H) 3.2 (m, *J*=17.2 Hz, 2 H) 3.5 (m, 2 H) 4.2 (q, *J*=7.0 Hz, 2 H) 7.4 (d, *J*=8.2 Hz, 1 H) 7.8 (m, 2 H) 8.4 (s, 3 H)

### EXAMPLE 63

### 2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine

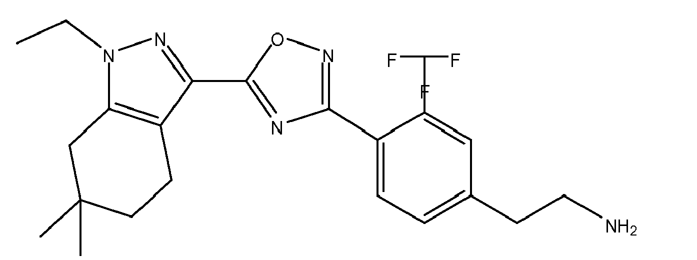

Obtained (11% yield) from Example 57 following the General Method 6.
LRMS: m/z 434 (M+1)⁺
Retention time: 13.18 min (Method C)
¹H NMR (400 MHz, DMSO-D6) δ ppm 1.0 (s, 6 H) 1.3 (t, *J*=7.2 Hz, 3 H) 1.5 (t, *J*=6.3 Hz, 2 H) 2.5 (m, *J*=2.0 Hz, 2 H) 2.7 (t, *J*=6.1 Hz, 2 H) 3.1 (d, *J*=7.4 Hz, 2 H) 3.2 (d, *J*=7.4 Hz, 2 H) 4.2 (m, 2 H) 7.8 (d, *J*=9.0 Hz, 1 H) 7.9 (d, *J*=7.8 Hz, 2 H)

### EXAMPLE 64

### 3-(2,6-Dimethyl-4-(5-(1,6,6-trimethyl-4,5,6,7-tetrahydro-1H-indazol-3-yl)-1,2,4-oxadiazol-3-yl)phenyl)propanoic acid

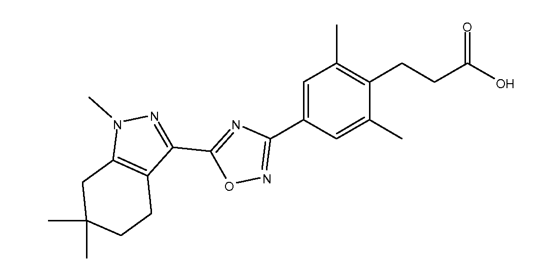

Obtained (76% yield) from Preparation 121 following the General Method 4.
LRMS: m/z 409 (M+1)⁺
Retention time: 19.08 min (Method C)
¹H NMR (400 MHz, DMSO-D6) δ ppm 1.0 (s, 6 H) 1.5 (t, *J*=6.3 Hz, 2 H) 2.4 (s, 6 H) 2.5 (s, 2 H) 2.5 (d, *J*=2.0 Hz, 2 H) 2.8 (t, *J*=6.1 Hz, 2 H) 2.9 (m, 2 H) 3.8 (s, 3 H) 7.7 (s, 2 H)

### EXAMPLE 65

### 3-(2,6-Dimethyl-4-(5-(1,6,6-trimethyl-4,5,6,7-tetrahydro-1H-indazol-3-yl)-1,2,4-oxadiazol-3-yl)phenyl)propanamide

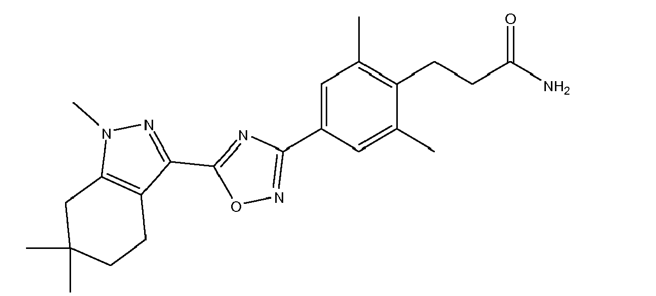

Obtained (100% yield) from Example 64 following the General Method 5.
LRMS: m/z 408 (M+1)⁺
Retention time: 17.66 min (Method C)
¹H NMR (400 MHz, DMSO-D6) δ ppm 1.0 (s, 6 H) 1.5 (t, *J*=6.3 Hz, 2 H) 2.2 (m, 2 H) 2.4 (s, 6 H) 2.4 (s, 2 H) 2.8 (t, *J*=6.1 Hz, 2 H) 2.8 (m, 2 H) 3.8 (s, 3 H) 6.8 (s, 1 H) 7.3 (s, 1 H) 7.7 (s, 2 H)

### EXAMPLE 66

### 3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid

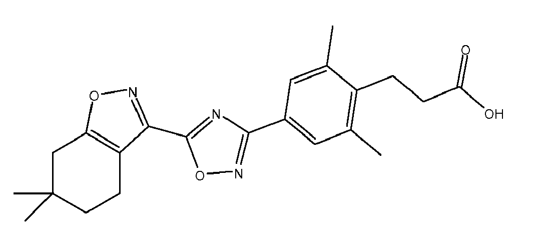

Obtained (69% yield) from Preparation 122 following the General Method 4.
LRMS: m/z 396 (M+1)⁺
Retention time: 19.76 min (Method C)
¹H NMR (200 MHz, DMSO-D6) δ ppm 1.0 (s, 6 H) 1.6 (t, *J*=5.9 Hz, 2 H) 2.5 (s, *J*=1.8 Hz, 2 H) 2.6 (s, 2 H) 2.8 (t, *J*=6.2 Hz, 2 H) 2.9 (m, 2 H) 3.3 (s, 6 H) 7.7 (s, 2 H) 12.3 (s, 1 H)

### EXAMPLE 67

### 3-(4-(5-(6,6-Dimethyl-4,5,6,7-tetrahydrobenzo[d]isoxazol-3-yl)-1,2,4-oxadiazol-3-yl)-2,6-dimethylphenyl)propanamide

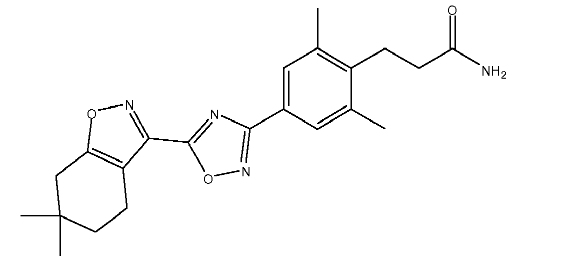

Obtained (97% yield) from Example 66 following the General Method 5.
LRMS: m/z 395 (M+1)⁺
Retention time: 18.55 min (Method C)
¹H NMR (200 MHz, DMSO-D6) δ ppm 1.0 (s, 6 H) 1.6 (t, *J*=6.2 Hz, 2 H) 2.2 (m, 2 H) 2.4 (s, 6 H) 2.6 (s, 2 H) 2.8 (m, 4 H) 6.8 (s, 1 H) 7.4 (s, 1 H) 7.7 (s, 2 H)

### EXAMPLE 68

### 2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid

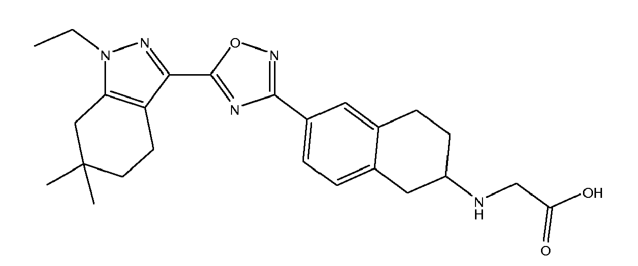

Obtained (10% yield) from Example 62 and 2-oxoacetic acid following the General Method 8.
LRMS: m/z 450 (M+1)⁺
Retention time: 15.61 min (Method C)
¹H NMR (400 MHz, DMSO-D6) δ ppm 0.8 (s, 6 H) 1.1 (t, 3 H) 1.3 (q, *J*=5.7 Hz, 2 H) 1.5 (m, 2 H) 1.9 (m, 2 H) 2.6 (m, 5 H) 2.7 (m, 2 H) 3.0 (s, 2 H) 3.9 (q, *J*=7.0 Hz, 2 H) 7.1 (d, *J*=8.2 Hz, 1 H) 7.6 (m, 2 H)

### EXAMPLE 69

### 3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine

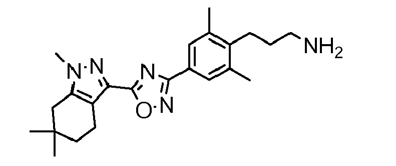

Obtained (27% yield) from Example 65 following the General Method 7.
LRMS: m/z 394 (M+1)⁺
Retention time: 13.45 min (Method C)
¹H NMR (400 MHz, DMSO-D6) d ppm 1.0 (s, 6 H) 1.6 (m, 2 H) 1.7 (m, 2 H) 2.4 (s, 6 H) 2.5 (s, 2 H) 2.7 (m, 2 H) 2.8 (m, 2 H) 2.9 (m, 2 H) 3.8 (s, 3 H) 7.7 (s, 2 H) 7.9 (m, 3 H).

### EXAMPLE 70

### 3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine

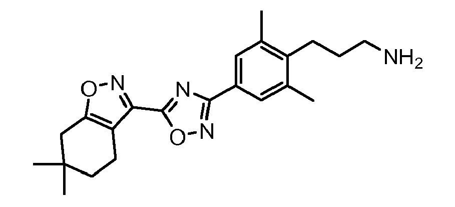

Obtained (21% yield) from Example 67 following the General Method 7.
LRMS: m/z 381 (M+1)⁺
Retention time: 14.28 min (Method C)
¹H NMR (400 MHz, DMSO-D6) δ ppm 1.0 (s, 6 H) 1.6 (t, *J*=6.3 Hz, 2 H) 1.7 (m, 2 H) 2.4 (s, 6 H) 2.7 (s, 2 H) 2.7 (m, 2 H) 2.8 (t, *J*=6.1 Hz, 2 H) 2.9 (m, 2 H) 7.8 (s, 2 H) 8.0 (s, 3 H)

### EXAMPLE 71

### 2-(2,6-Dimethyl-4-(5-(1,6,6-trimethyl-4,5,6,7-tetrahydro-1H-indazol-3-yl)-1,2,4-oxadiazol-3-yl)phenyl)ethanamine

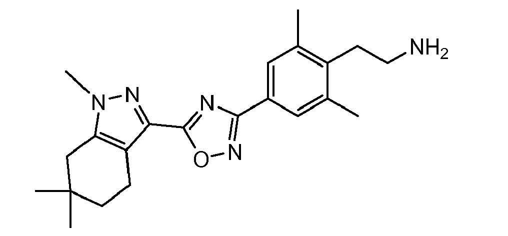

Obtained (23% yield) from Example 64 following the General Method 6.
LRMS: m/z 380 (M+1)⁺
Retention time: 12.75 min (Method C)
¹H NMR (400 MHz, DMSO-D6) δ ppm 1.0 (s, 6 H) 1.5 (t, *J*=6.3 Hz, 2 H) 2.4 (s, 6 H) 2.5 (s, 2 H) 2.8 (m, 4 H) 3.0 (m, 2 H) 3.8 (s, 3 H) 7.7 (s, 2 H) 8.1 (s, 3 H)

### EXAMPLE 72

### 2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine

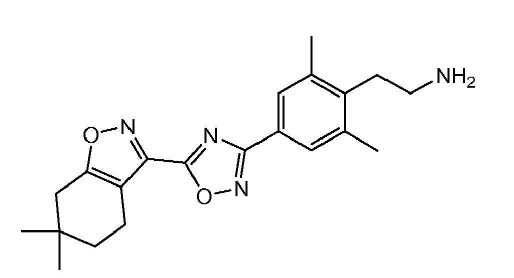

Obtained (43% yield) from Example 66 following the General Method 6.
LRMS: m/z 367 (M+1)⁺
Retention time: 13.54 min (Method C)
¹H NMR (400 MHz, DMSO-D6) δ ppm 1.0 (s, 6 H) 1.6 (t, *J*=6.3 Hz, 2 H) 2.4 (s, *J*=8.2 Hz, 6 H) 2.7 (s, 2 H) 2.8 (t, *J*=6.1 Hz, 2 H) 2.9 (m, 2 H) 3.0 (m, 2 H) 7.8 (s, 2H)8.1 (s, 3 H)

### EXAMPLE 73

### 5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one

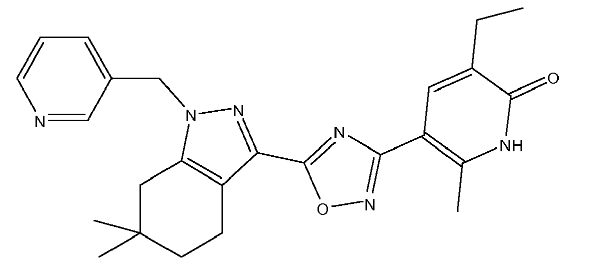

Obtained (8% yield) from Preparation 125 following the experimental procedure described for Example 18.
LRMS: m/z 445 (M+1)⁺
Retention time: 16.64 min (Method C)
¹H NMR (300 MHz, CHLOROFORM-D) δ ppm 1.0 (s, 6 H) 1.3 (t, 3 H) 1.6 (m, 2 H) 2.3 (s, 2 H) 2.6 (q, *J*=7.4 Hz, 2 H) 2.8 (s, 3 H) 2.9 (t, *J*=6.2 Hz, 2 H) 5.4 (s, 2 H) 7.3 (dd, 1 H) 7.5 (dd, *J*=8.0, 1.6 Hz, 1 H) 8.1 (s, 1 H) 8.6 (m, 2 H) 11.9 (s, 1 H)

### EXAMPLE 74

### 2-(Dimethyl-4,5,6,7-tetrahydrobenzo[d]isoxazol-3-yl)-1,2,4-oxadiazol-3-yl)-2,6-dimethylphenethylamino)ethanoic acid

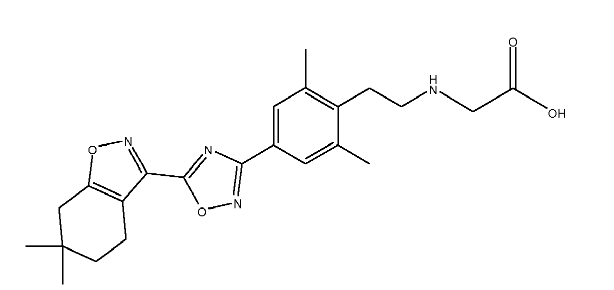

Obtained (20% yield) from Preparation 128 following the General Method 3.
LRMS: m/z 425 (M+1)⁺
Retention time: 16.48 min (Method C)
¹H NMR (400 MHz, DMSO-D6) δ ppm 1.0 (s, 6 H) 1.6 (t, *J*=6.3 Hz, 2 H) 2.4 (s, 6 H) 2.6 (s, 2 H) 2.8 (t, *J*=6.3 Hz, 2 H) 2.8 (d, *J*=16.4 Hz, 2 H) 3.0 (d, *J*=9.8 Hz, 2 H) 3.2 (s, 2 H) 7.7 (s, 2H)

### EXAMPLE 75

### 2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid

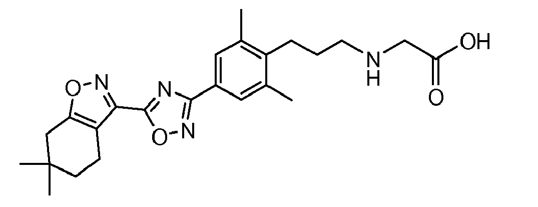

Obtained (15% yield) from Preparation 129 following the General Method 3.
LRMS: m/z 439 (M+1)⁺
Retention time: 17.16 min (Method C)

### EXAMPLE 76

### 3-(Dimethyl-4,5,6,7-tetrahydrobenzo[d]isoxazol-3-yl)-1,2,4-oxadiazol-3-yl)-2,6-dimethylphenethylamino)propanoic acid

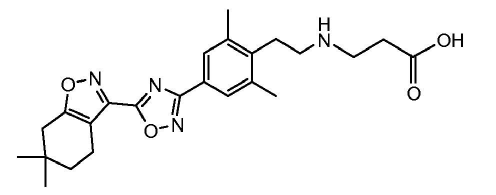

Obtained (42% yield) from Preparation 130 following the General Method 3.
LRMS: m/z 439 (M+1)⁺
Retention time: 14.99 min (Method C)
¹H NMR (400 MHz, DMSO-D6) δ ppm 1.0 (s, 6 H) 1.6 (t, *J*=6.3 Hz, 2 H) 2.4 (s, 6 H) 2.5 (m, *J*=6.7 Hz, 2 H) 2.6 (s, 2 H) 2.8 (t, *J*=6.1 Hz, 2 H) 2.8 (m, 2 H) 2.9 (m, 2 H) 3.0 (t, *J*=6.7 Hz, 2 H) 7.7 (s, 2 H)

### EXAMPLE 77

### 3-Ethyl-5-(5-(1-ethyl-4,5,6,7-tetrahydro-1H-indazol-3-yl)-1,2,4-oxadiazol-3-yl)-6-methylpyridin-2(1H)-one

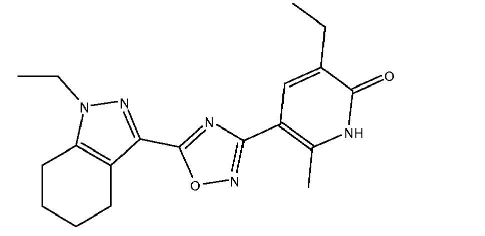

Obtained (63% yield) from Preparation 131 following the experimental procedure described for Example 18.
LRMS: m/z 433 (M+1)⁺
Retention time: 14.98 min (Method C)
¹H NMR (300 MHz, CHLOROFORM-D) δ ppm 1.3 (t, 3 H) 1.5 (t, 3 H) 1.9 (m, 4 H) 2.6 (m, 4 H) 2.8 (m, 3 H) 2.9 (t, *J*=6.0 Hz, 2 H) 4.2 (q, *J*=7.4 Hz, 2 H) 8.1 (s, 1 H) 12.0 (s, 1 H)

### EXAMPLE 78

### 3-Ethyl-6-methyl-5-(5-(1,6,6-trimethyl-4,5,6,7-tetrahydro-1H-indazol-3-yl)-1,2,4-oxadiazol-3-yl)pyridin-2-ol

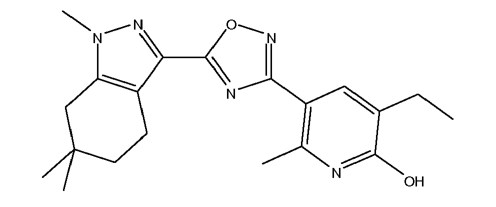

Obtained (26% yield) from Preparation 135 following the experimental procedure described for Example 18.
LRMS: m/z 368 (M+1)⁺
Retention time: 17.40 min (Method C)
¹H NMR (300 MHz, CHLOROFORM-D) δ ppm 1.1 (s, 6 H) 1.2 (m, 5 H) 2.4 (s, 2 H) 2.6 (m, 2 H) 2.7 (s, 3 H) 2.9 (m, 2 H) 3.9 (s, 3 H) 8.0 (s, 1 H) 10.4 (s, 1 H)

### EXAMPLE 79

### 5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one

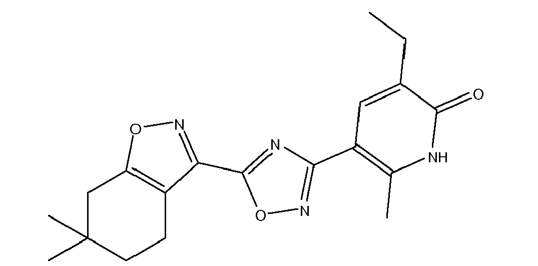

Obtained (80% yield) from Preparation 136 following the experimental procedure described for Example 18.
LRMS: m/z 355 (M+1)⁺
Retention time: 18.34 min (Method C)
¹H NMR (300 MHz, CHLOROFORM-D) δ ppm 1.1 (s, 6 H) 1.3 (t, *J*=7.5 Hz, 3 H) 1.7 (m, 2 H) 2.6 (m, 4 H) 2.8 (s, 3 H) 2.8 (m, 2 H) 8.0 (s, 1 H) 12.4 (s, 1 H)

### EXAMPLE 80

### 5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol

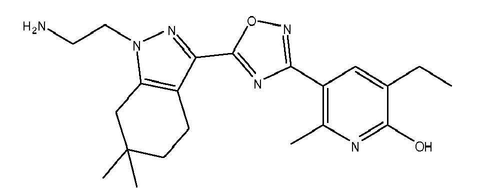

Obtained (82% yield) from Preparation 137 following the experimental procedure described for Example 18.
LRMS: m/z 397 (M+1)⁺
Retention time: 11.07 min (Method C)
¹H NMR (300 MHz, METHANOL-D4) δ ppm 1.1 (s, 6 H) 1.2 (t, *J*=7.5 Hz, 3 H) 1.7 (t, *J*=6.3 Hz, 2 H) 2.6 (m, 4 H) 2.7 (s, 3 H) 2.9 (t, *J*=6.2 Hz, 2 H) 3.5 (m, 2 H) 4.4(m,2H)8.0(s,1H)

### EXAMPLE 81

### 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine

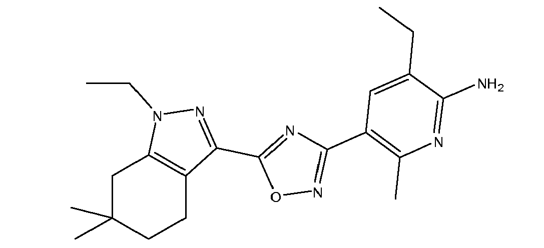

Obtained (28% yield) from Preparation 140 following the experimental procedure described for Example 41.
LRMS: m/z 381 (M+1)⁺
Retention time: 13.75 min (Method C)

### EXAMPLE 82

### 2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine

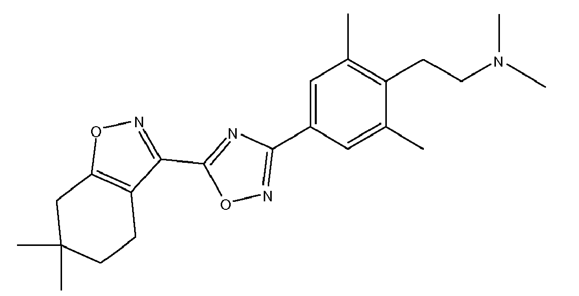

Obtained (40% yield) from Example 72 and formaldehyde following the General Method 8.
LRMS: m/z 395 (M+1)⁺
Retention time: 13.72 min (Method C)
¹H NMR (400 MHz, DMSO-D6) δ ppm 1.0 (s, 6 H) 1.6 (t, 2 H) 2.4 (s, 6 H) 2.5 (d, *J*=2.0 Hz, 2 H) 2.6 (s, 2 H) 2.7 (m, *J*=9.4 Hz, 2 H) 2.8 (s, 6 H) 3.1 (m, 2 H) 6.5 (s, 1 H) 7.7 (s, 2 H)

### EXAMPLE 83

### 3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid

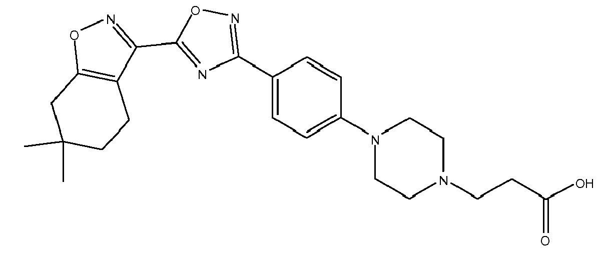

Obtained (47% yield) from Preparation 147 following the General Method 3.
LRMS: m/z 452 (M+1)⁺
Retention time: 14.28 min (Method C)
¹H NMR (400 MHz, DMSO-D6) δ ppm 1.0 (s, 6 H) 1.6 (t, *J*=6.1 Hz, 2 H) 2.4 (m, *J*=7.0, 7.0 Hz, 2 H) 2.6 (m, 2 H) 2.6 (s, 2 H) 2.8 (t, *J*=6.1 Hz, 2 H) 3.3 (s, 8 H) 7.1 (d, *J*=9.0 Hz, 2 H) 7.9 (d, *J*=9.0 Hz, 2 H)

### EXAMPLE 84

### 3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one

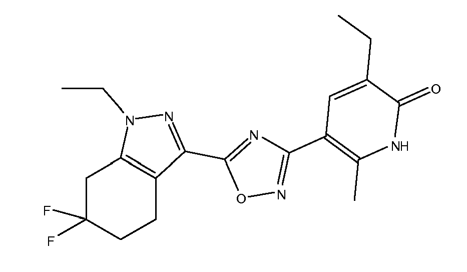

Obtained (30% yield) from Preparation 148 following the experimental procedure described for Example 18.
LRMS: m/z 390 (M+1)⁺
Retention time: 15.66 min (Method C)
¹H NMR (300 MHz, CHLOROFORM-D) δ ppm 1.3 (m, 3 H) 1.5 (t, *J*=7.1 Hz, 3 H) 2.3 (m, 2 H) 2.6 (q, *J*=7.3 Hz, 2 H) 2.8 (s, 3 H) 3.2 (m, 4 H) 4.2 (q, *J*=7.1 Hz, 2 H) 8.1 (s, 1 H) 12.3 (s, 1 H)

### EXAMPLE 85

### 3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid

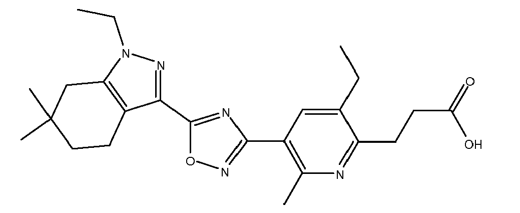

Obtained (96% yield) from Preparation 154 following the General Method 3.
LRMS: m/z 438 (M+1)⁺
Retention time: 18.79 min (Method C)
¹H NMR (300 MHz, CHLOROFORM-D) δ ppm 1.1 (s, 6 H) 1.3 (m, 3 H) 1.5 (t, *J*=7.1 Hz, 3 H) 1.7 (m, 2 H) 2.4 (s, 2 H) 2.8 (m, 2 H) 2.9 (m, 7 H) 3.2 (m, 2 H) 4.2 (m, 2 H) 8.4 (s, 1 H)

### EXAMPLE 86

### 5-(5-(1-(Cyclopropylmethyl)-6,6-dimethyl-4,5,6,7-tetrahydro-1H-indazol-3-yl)-1 ,2,4-oxadiazol-3-yl)-3-ethyl-6-methylpyridin-2(1H)-one

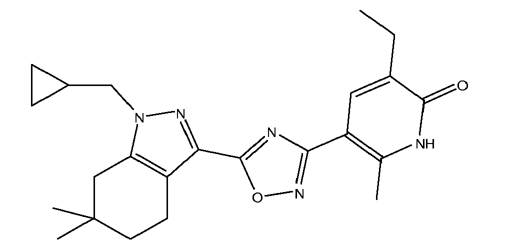

Obtained (30% yield) from Preparation 159 following the experimental procedure described for Example 18.
LRMS: m/z 408 (M+1)⁺
Retention time: 19.41 min (Method C)
¹H NMR (300 MHz, CHLOROFORM-D) δ ppm 0.4 (m, 2 H) 0.6 (m, 2 H) 1.1 (s, 6 H) 1.3 (t, *J*=7.4 Hz, 3 H) 1.3 (m, 1 H) 1.6 (t, *J*=6.3 Hz, 2 H) 2.4 (s, 2 H) 2.6 (q, *J*=7.6 Hz, 2 H) 2.8 (s, 3 H) 2.9 (t, *J*=6.2 Hz, 2 H) 4.0 (d, *J*=6.9 Hz, 2 H) 8.1 (s, 1 H) 12.3 (s, 1 H)

### EXAMPLE 87

### 3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol

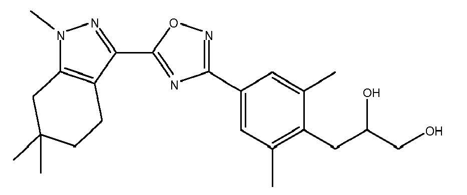

To a solution of Preparation 162 (1,7g, 4,5mmol) in a mixture of THF/tert-butanol (35ml/5ml) was added 4-methylmorpholine 4-oxide (1,07g, 9,1mmol) and osmium(VIII) oxide (555 I, 0,09mmol). The reaction mixture was stirred overnight at r.t. Then 40% solution of Na2SO3 was added and the mixture stirred for 30min. Ethyl acetate was added and the organic layer separated, washed twice with water, dried over magnesium sulphate and concentrated to give 1,72 g of the title compound (92% yield).
LRMS: m/z 411 (M+1)⁺
Retention time: 17.80 min (Method C)

### EXAMPLE 88

### N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine

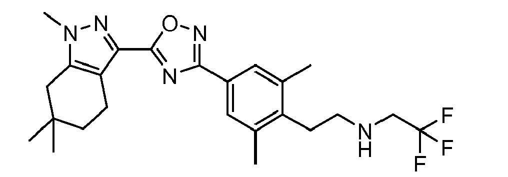

Obtained (82% yield) from Preparation 163 and 2,2,2-trifluoroethanamine following the General Method 8.
LRMS: m/z 462 (M+1)⁺
Retention time: 19.74 min (Method C)
¹H NMR (400 MHz, DMSO-D6) δ ppm 1.0 (s, 6 H) 1.5 (t, *J*=6.1 Hz, 2 H) 2.4 (s, 6 H) 2.5 (m, 2 H) 2.5 (m, 8 H) 3.8 (s, 3 H) 7.7 (s, 2 H)

### EXAMPLE 89

### 2-(2,6-Dimethyl-4-(5-(1,6,6-trimethyl-4,5,6,7-tetrahydro-1H-indazol-3-yl)-1,2,4-oxadiazol-3-yl)phenethylamino)ethanol

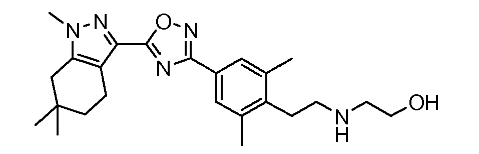

Obtained (48% yield) from Preparation 163 and 2-aminoethanol following the General Method 8.
LRMS: m/z 424 (M+1)⁺
Retention time: 12.86 min (Method C)
¹H NMR (400 MHz, DMSO-D6) δ ppm 1.0 (s, 6 H) 1.5 (t, *J*=6.1 Hz, 2 H) 2.4 (s, 6 H) 2.5 (s, 2 H) 2.8 (t, *J*=5.9 Hz, 2 H) 2.9 (s, 2 H) 3.1 (m, 4 H) 3.7 (s, 2 H) 3.8 (s, 3 H) 5.3 (s, 1 H) 7.7 (s, 2 H)

### EXAMPLE 90

### 2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid

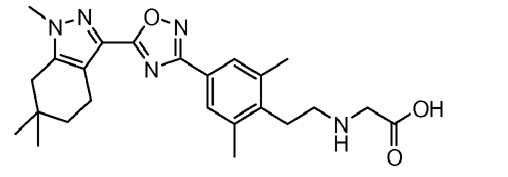

Obtained (25% yield) from Preparation 163 and 2-aminoacetic acid following the General Method 8.
LRMS: m/z 438 (M+1)⁺
Retention time: 15.57 min (Method C)
¹H NMR (400 MHz, DMSO-D6) δ ppm 1.0 (s, 6 H) 1.5 (t, *J*=6.3 Hz, 2 H) 2.4 (s, 6 H) 2.8 (m, *J*=5.9, 5.9 Hz, 2 H) 3.0 (m, *J*=15.3 Hz, 2 H) 3.1 (m, *J*=10.6 Hz, 2 H) 3.7 (dd, *J*=14.5, 4.7 Hz, 2 H) 3.8 (s, 3 H) 3.9 (s, 2 H) 7.7 (s, 2 H) 9.5 (s, 1 H)

### EXAMPLE 91

### 1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid

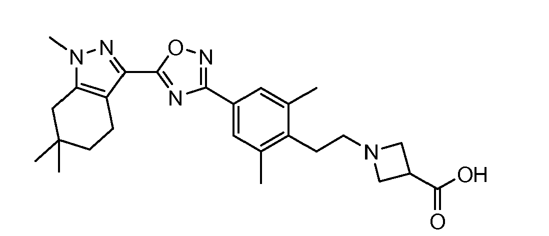

Obtained (74% yield) from Preparation 163 and azetidine-3-carboxilic acid following the General Method 8.
LRMS: m/z 464 (M+1)⁺
Retention time: 14.72 min (Method C)
¹H NMR (400 MHz, DMSO-D6) δ ppm 1.0 (s, 6 H) 1.5 (t, *J*=6.3 Hz, 2 H) 2.4 (s, 6 H) 2.4 (s, 2 H) 2.8 (t, *J*=6.3 Hz, 2 H) 2.9 (m, 2 H) 3.2 (m, 2 H) 3.6 (m, 1 H) 3.8 (s, 3 H) 4.2 (m, 4 H) 7.7 (s, 2 H)

### EXAMPLE 92

### 3-(2-Methyl-4-(5-(1,6,6-trimethyl-4,5,6,7-tetrahydro-1H-indazol-3-yl)-1,2,4-oxadiazol-3-yl)phenyl)propanoic acid

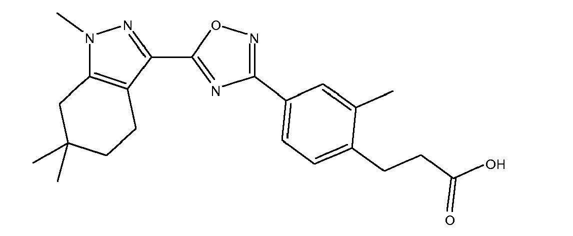

Obtained (20% yield) from Preparation 164 following the General Method 3.
LRMS: m/z 395 (M+1)⁺
Retention time: 18.71 min (Method C)
¹H NMR (200 MHz, DMSO-D6) δ ppm 1.0 (s, 6 H) 1.6 (s, 2 H) 2.4 (s, 3 H) 2.5 (m, 4 H) 2.8 (m, 4 H) 3.8 (s, 3 H) 7.4 (d, *J*=7.4 Hz, 1 H) 7.8 (m, *J*=10.2 Hz, 2 H)

### EXAMPLE 93

### 4-(2,6-Dimethyl-4-(5-(1,6,6-trimethyl-4,5,6,7-tetrahydro-1H-indazol-3-yl)-1,2,4-oxadiazol-3-yl)phenethyl)morpholine

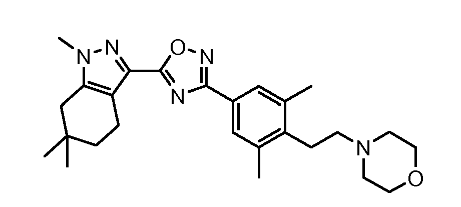

Obtained (87% yield) from Preparation 163 and morpholine following the General Method 8.
LRMS: m/z 450 (M+1)⁺
Retention time: 13.20 min (Method C)
¹H NMR (200 MHz, DMSO-D6) d ppm 1.0 (s, 6 H) 1.5 (t, *J*=6.2 Hz, 2 H) 2.5 (s, 6 H) 2.5 (s, 2 H) 2.8 (t, *J*=5.7 Hz, 2 H) 3.2 (m, 4 H) 3.6 (m, 4 H) 3.8 (s, 3 H) 4.0 (m,4H)7.7(s,2H)11.6(s, 1 H)

### EXAMPLE 94

### 3-(4-(5-(6,6-Dimethyl-4,5,6,7-tetrahydro-1H-indazol-3-yl)-1,2,4-oxadiazol-3-yl)-2,6-dimethylphenyl)propane-1,2-diol

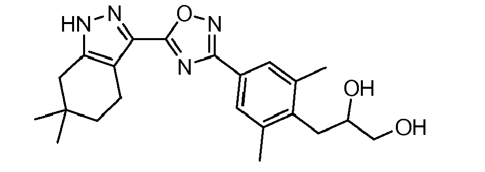

Obtained (68% yield) from Preparation 168 following the experimental procedure described in Example 87.
LRMS: m/z 397 (M+1)⁺
Retention time: 17.12 min (Method C)
¹H NMR (200 MHz, DMSO-D6) δ ppm 1.0 (s, 6 H) 1.6 (t, *J*=6.2 Hz, 2 H) 2.4 (s, 6 H) 2.5 (s, 2 H) 2.8 (m, 4 H) 3.4 (m, 2 H) 3.6 (m, 1 H) 4.7 (m, 2 H) 7.7 (s, 2 H)

### EXAMPLE 95

### 3-(4-(5-(1-Ethyl-6,6-dimethyl-4,5,6,7-tetrahydro-1H-indazol-3-yl)-1,2,4-oxadiazol-3-yl)phenylamido)propanoic acid

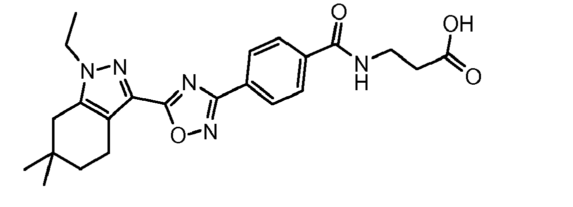

Obtained (47% yield) from Preparation 170 following the General Method 4.
LRMS: m/z 438 (M+1)⁺
Retention time: 17.19 min (Method C)
¹H NMR (300 MHz, METHANOL-D4) δ ppm 1.1 (s, 6 H) 1.4 (t, *J*=7.3 Hz, 3 H) 1.6 (t, *J*=6.3 Hz, 2 H) 2.5 (s, 2 H) 2.6 (t, *J*=6.8 Hz, 2 H) 2.9 (t, *J*=6.3 Hz, 2 H) 3.7 (t, *J*=6.8 Hz, 2 H) 4.2 (q, *J*=7.3 Hz, 2 H) 8.0 (d, *J*=8.4 Hz, 2 H) 8.2 (d, *J*=8.4 Hz, 2 H)

### EXAMPLE 96

### 3-(4-(5-(6,6-Dimethyl-1-(pyridin-3-ylmethyl)-4,5,6,7-tetrahydro-1H-indazol-3-yl)-1 ,2,4-oxadiazol-3-yl)-2,6-dimethylphenyl)propane-1 ,2-diol

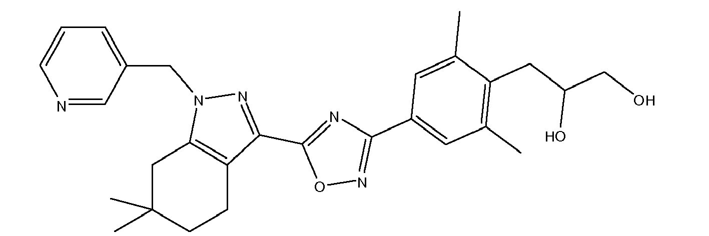

Obtained (52% yield) from Preparation 171 following the experimental procedure described in Example 87.
LRMS: m/z 488 (M+1)⁺
Retention time: 17.14 min (Method C)
¹H NMR (300 MHz, CHLOROFORM-D) δ ppm 1.0 (s, 6 H) 1.6 (m, 2 H) 2.4 (s, 6 H) 2.9 (m, 4 H) 3.6 (m, 1 H) 3.7 (m, 1 H) 4.0 (m, 1 H) 5.4 (s, 2 H) 7.3 (m, 1 H) 7.5 (m, 1 H) 7.9 (s, 2 H) 8.5 (d, *J*=1.6 Hz, 1 H) 8.6 (dd, *J*=4.8, 1.5 Hz, 1 H)

### EXAMPLE 97

### 3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid

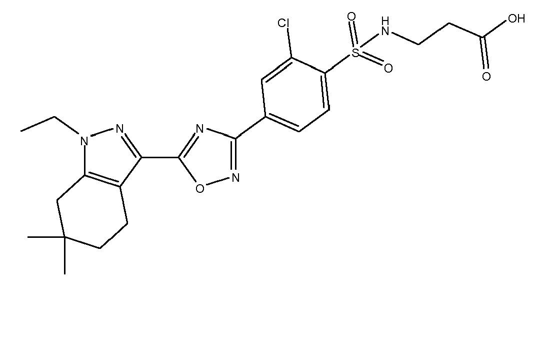

Obtained (84% yield) from Preparation 173 following the General Method 4.
LRMS: m/z 423 (M+1)⁺
Retention time: 5,08 min (Method B)
¹H NMR (300 MHz, DMSO-D6) δ ppm 1.1 (s, 6 H) 1.4 (t, *J*=7.3 Hz, 3 H) 1.6 (t, *J*=6.2 Hz, 2 H) 2.4 (t, *J*=6.9 Hz, 2 H) 2.6 (s, 2 H) 2.9 (t, *J*=5.9 Hz, 2 H) 3.2 (t, *J*=6.6 Hz, 2 H) 4.2 (q, *J*=7.3 Hz, 2 H) 8.2 (m, 3 H)

### EXAMPLE 98

### 3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol

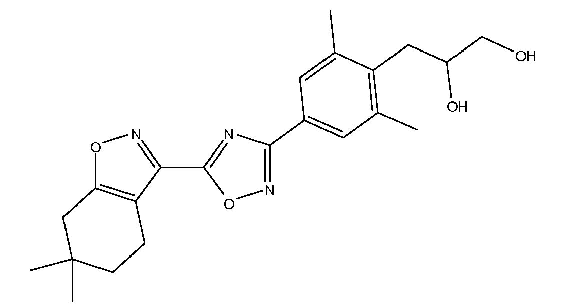

Obtained (52% yield) from Preparation 176 following the experimental procedure described in Example 87.
LRMS: m/z 398 (M+1)⁺
Retention time: 5,47min (Method B)
¹H NMR (300 MHz, DMSO-D6) δ ppm 1.0 (s, 6 H) 1.6 (t, *J*=6.2 Hz, 2 H) 2.4 (s, 6 H) 2.6 (s, 2 H) 2.7 (m, 1 H) 2.8 (t, *J*=6.2 Hz, 2 H) 2.9 (dd, *J*=13.6, 3.7 Hz, 1 H) 3.4 (m, 2 H) 3.7 (m, 1 H) 4.7 (m, 2 H) 7.7 (s, 2 H)

### EXAMPLE 99

### 3-(1-Ethyl-6,6-dimethyl-4,5,6,7-tetrahydro-1H-indazol-3-yl)-5-(pyridin-4-yl)-1,2,4-oxadiazole

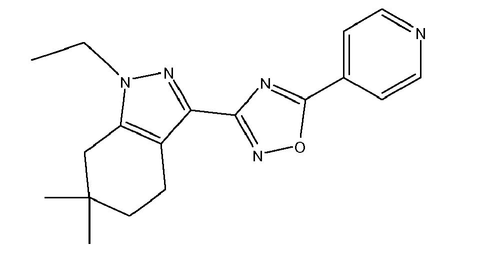

Obtained (72%) from isonicotinic acid and Preparation 179 following the General Method 2.
LRMS: m/z 324 (M+1)⁺
Retention time: 17.77 min (Method C)
¹H NMR (400 MHz, DMSO-D6) δ ppm 1.0 (s, 6 H) 1.3 (t, *J*=7.2 Hz, 3 H) 1.5 (t, *J*=6.5 Hz, 2 H) 2.5 (s, 2 H) 2.7 (t, *J*=6.3 Hz, 2 H) 4.1 (q, *J*=7.0 Hz, 2 H) 8.1 (dd, 2 H) 8.9 (dd, 2 H)

### EXAMPLE 100

### 5-(1-Ethyl-6,6-dimethyl-4,5,6,7-tetrahydro-1H-indazol-3-yl)-3-o-tolyl-1,2,4-oxadiazole

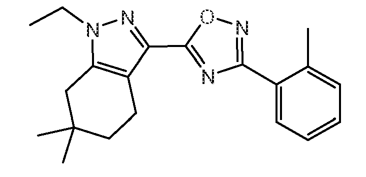

To a solution of Preparation 181 (50mg, 0,12mmol) in a mixture of DMF (2ml) and water (1ml), PdCl2 (6mg, 0,04mmol) and Cs2CO3 (196mg, 0,6mmol) were added and it was heated at 120°C in microwave conditions during 2h. The reaction creude was purified following General Purification Method to yield 17 mg of the title compund (42% yield).
LRMS: m/z 337 (M+1)⁺
Retention time: 20.82 min (Method C)
¹H NMR (400 MHz, DMSO-D6) δ ppm 1.0 (s, 6 H) 1.3 (t, *J*=7.2 Hz, 3 H) 1.5 (t, *J*=6.3 Hz, 2 H) 2.47 (S, 3 H) 2.48 (S, 2 H) 2.8 (t, *J*=6.1 Hz, 2 H) 4.1 (q, *J*=7.0 Hz, 2 H) 7.4 (m, 3 H) 8.0 (d, *J*= 7.8 Hz, 1 H)

### EXAMPLE 101

### 3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid

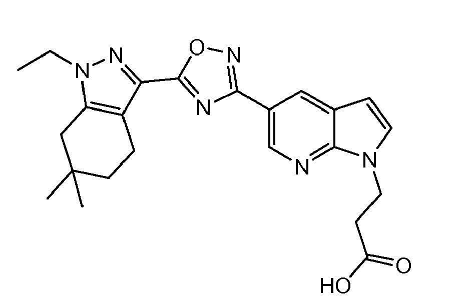

Obtained (82%) from Preparation 183 following the General Method 3.
LRMS: m/z 435 (M+1)⁺
Retention time: 18.68 min (Method C)
¹H NMR (300 MHz, CHLOROFORM-D) δ ppm 1.1 (s, 6 H) 1.5 (t, *J*=7.3 Hz, 3 H) 1.6 (t, *J*=6.5 Hz, 2 H) 2.4 (s, 2 H) 2.9 (t, *J*=6.2 Hz, 2 H) 3.0 (t, *J*=6.7 Hz, 2 H) 4.2 (q, *J*=7.2 Hz, 2 H) 4.6 (t, *J*=6.7 Hz, 2 H) 6.5 (d, *J*=3.3 Hz, 1 H) 7.3 (d, *J*=3.3 Hz, 1 H) 8.8 (s, 1 H) 9.2 (s, 1 H)

### EXAMPLE 102

### 3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid

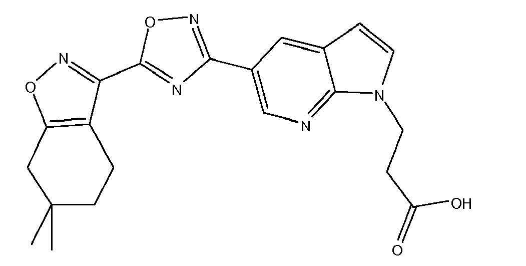

Obtained (86%) from Preparation 186 following the General Method 3.
LRMS: m/z 408 (M+1)⁺
Retention time: 18.68 min (Method C)
¹H NMR (300 MHz, CHLOROFORM-D) δ ppm 1.1 (s, 6 H) 1.7 (t, *J*=6.0 Hz, 2 H) 2.6 (s, 2 H) 2.9 (t, *J*=6.0 Hz, 2 H) 3.0 (t, *J*=6.5 Hz, 2 H) 4.7 (t, *J*=6.5 Hz, 1 H) 6.6 (d, *J*=3.6 Hz, 1 H) 7.4 (d, *J*=3.6 Hz, 1 H) 8.7 (d, *J*=1.6 Hz, 1 H) 9.1 (d, *J*=1.6 Hz, 1 H)

### PHARMACOLOGICAL ACTIVITY

### 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plates scintillant liquid were added to each well and 35S-GTPyS binding was measured on a Trilux Scintillation Counter.

The results are shown in Table 1.

**Table 1**

| **EXAMPLES** | **EC₅₀ (nM)** |
|---|---|
| 5 | 40 |
| 8 | 17 |
| 21 | 18 |
| 36 | 48 |
| 42 | 16 |
| 48 | 8 |
| 56 | 31 |
| 68 | 1.9 |
| 74 | 1.7 |
| 91 | 16 |
| 97 | 26 |
| 99 | 146 |

The 5-indazole derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) beta interferons such as Betaseron, Avonex or Rebif, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide, (i) fumaric acid esters, such as *BG-12*, (j) immunomodulators such as Laquinimod, (k) anti-CD20 monoclonal antibodies such as Rituximab, Ocrelizumab Ofatumumab or TRU-015, (l) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as CP-690550 or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1),. Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Genuair® (formerly known as Novolizer SD2FL) which is described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

### The following preparations forms are cited as formulation examples:

### COMPOSITION EXAMPLE 1

50,000 capsules, each containing 100 mg of 5-(1-Ethyl-6,6-dimethyl-4,5,6,7-tetrahydro-1H-indazol-3-yl)-3-(3-methylpyridin-2-yl)-1,2,4-oxadiazole (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 5-(1-Ethyl-6,6-dimethyl-4,5,6,7-tetrahydro-1H-indazol-3-yl)-3-(3-methylpyridin-2-yl)-1,2,4-oxadiazole (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of general formula (I), or a pharmaceutically acceptable salt or N-oxide thereof: 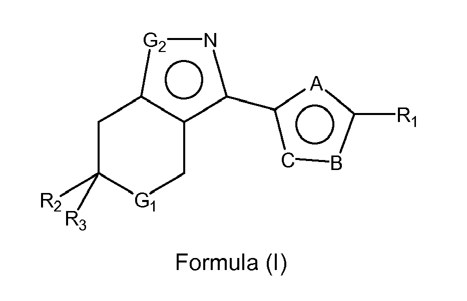 Wherein,
A is selected from the group consisting of -N-, -O- and -S-;
B and C are independently selected from the group consisting of -N- and -O-, with the proviso that at least two of A, B and C are nitrogen atoms;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 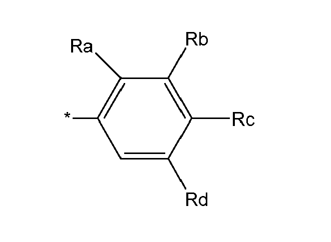 wherein:
• R^{a} represents a hydrogen atom, a halogen atom, a C₁₋₄ alkyl group, C₃₋₄ cycloalkyl group or a -CF₃ group;
• R^{b} represents a hydrogen atom, a halogen atom, a C₁₋₄ alkyl group, a -CF₃ group or a C₁₋₄ alkoxy group;
• R^{d} represents a hydrogen atom, a C₁₋₄ alkyl group or a C₁₋₄ alkoxy group;
• R^{c} represents:
○ a hydrogen atom, a C₁₋₄ hydroxyalkyl group, a C₁₋₄ aminoalkyl group which is optionally substituted with one or more substituents selected from halogen atoms, hydroxy groups and -CF₃ groups;
○ a 4 to 6-membered saturated N-containing heterocyclic ring optionally substituted with a C₁₋₂ carboxyalkyl group;
○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH₂)₍₀₋₃₎-NH-(CH₂)₍₁₋₃₎-(NH)₍₀₋₁)S(O)₂R' wherein,
■ R' represents a hydrogen atom or a C₁₋₄ alkyl group,
■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

2. A compound according to claim 1, wherein A is selected from the group consisting of -N- and -O-.

3. A compound according to claim 2, wherein A represents -N-.

4. A compound according to any one of claims 1 to 3, wherein both A and B represent -N- and C represents -O-.

5. A compound according to any one of the preceding claims, wherein G¹ represents a -CH₂- or a -O- group.

6. A compound according to claim 5, wherein G¹ represents a -CH₂- group.

7. A compound according to any one of the preceding claims, wherein R² and R³ are independently selected from the group consisting of hydrogen atoms, fluorine atoms and methyl groups.

8. A compound according to claim 7, wherein both R² and R³ are methyl groups.

9. A compound according to any one of claims 1 to 8, wherein R⁴ is selected from the group consisting of a C₃₋₄ cycloalkyl-C₁₋₄ alkyl group, C₁₋₄ haloalkyl group and a linear or branched unsubstituted C₁₋₄ alkyl group.

10. A compound according to claim 9, wherein G² represents-NR⁴-, and wherein R⁴ is selected from the group consisting of a methyl group, ethyl group, t-butyl group, cyclopropylmethyl group and 2,2,2-trifluoroethyl group.

11. A compound according to claim 10, wherein R⁴ represents a methyl or an ethyl group.

12. A compound according to any one of the preceding claims, wherein R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 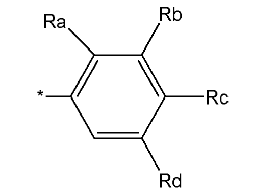 wherein:
• R^{a} represents a hydrogen atom or a C₁₋₄ alkyl group;
• R^{b} represents a hydrogen atom or C₁₋₄ alkyl group;
• R^{d} represents a hydrogen atom or a C₁₋₄ alkyl group;
• R^{c} represents:
○ a C₁₋₄ hydroxyalkyl group or a C₁₋₄ aminoalkyl substitued by one or more halogen atoms;
○ -(CH₂)₍₂₋₃₎-C(O)OR', -(CH₂)₍₀₋₂₎-C(O)NR'R", -O-(CH₂)₍₂₋₃₎NR'R", -(CH₂)₍₂₋₃₎-NHC(O)R", -S(O)₂NR'R", -(CH₂)₍₀₋₃₎-NR'R" or -(CH₂)₍₁₋₂₎-CONHS(O)₂R' wherein,
■ R' represents a hydrogen atom or a methyl group,
■ R" represents a hydrogen atom, a C₁₋₄ alkyl group, C₁₋₄ carboxyalkyl group, C₁₋₄ haloalkyl group or a C₁₋₄ hydroxyalkyl group, or
■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
or R^{c} together with R^{d} form a cyclohexyl group substituted with a carboxymethylamino group.

13. A compound according to claim 12, wherein R¹ represents:
➢ a pyridyl group substituted with two or three substituents selected from hydroxy groups, methyl and ethyl groups, or
➢ a group of formula: 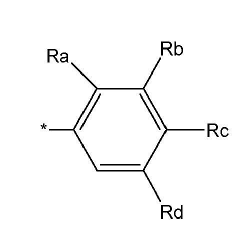 wherein:
• R^{a} represents a hydrogen atom or a methyl group;
• R^{b} represents a hydrogen atom, a methyl group;
• R^{d} represents a hydrogen atom or a methyl group;
• R^{c} represents: -(CH₂)₍₂₋₃₎-C(O)OR', -(CH₂)₂-C(O)NR'R" or -(CH₂)₍₂₋₃₎-NR'R", wherein
○ R' represents a hydrogen atom;
○ R" represents a hydrogen atom, a C₁₋₂ carboxyalkyl group, a C₁₋₄ haloalkyl group or a C₁₋₂ hydroxyalkyl group; or
○ R' and R" together with the nitrogen atom to which they are attached form a 4 membered saturated heterocyclic group, which contains as heteroatom, one nitrogen atom and which is substituted with a carboxy group.

14. A compound according to claim 13, wherein R¹ represents a group of formula: 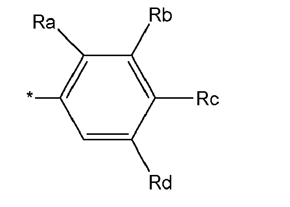 wherein:
○ R^{a} represents a hydrogen atom;
○ both R^{b} and R^{d} represent methyl groups; and
○ R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

15. A compound according to any one of the preceding claims wherein:
G¹ represents a -CH₂- group,
G² represents a -NR₄- group, wherein R⁴ represents a methyl or ethyl group, both R² and R³ represent a methyl group, and
R¹ represents:
➢ a pyridyl group substituted with two or three substituents selected from hydroxy groups, methyl or ethyl groups, or
➢ a group of formula: 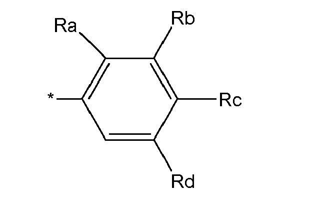 wherein:
• R^{a} represents a hydrogen atom or a methyl group;
• R^{b} represents a hydrogen atom, a methyl group,
• R^{d} represents a hydrogen atom or a methyl group,
• R^{c} represents: -(CH₂)₍₂₋₃₎-C(O)OR', -(CH₂)₂-C(O)NR'R" or -(CH₂)₍₂₋₃₎-NR'R", wherein:
○ R' represents a hydrogen atom;
○ R" represents a hydrogen atom, a C₁₋₂ carboxyalkyl group, a C₁₋₄ haloalkyl group or a C₁₋₂ hydroxyalkyl group, or
○ R' and R" together with the nitrogen atom to which they are attached form a 4 membered saturated heterocyclic group, which contains as heteroatom, one nitrogen atom and which is substituted with a carboxy group.

16. A compound according to claim 15, wherein R¹ represents a group of formula: 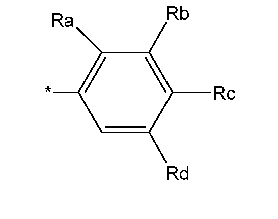 wherein
○ R^{a} represents a hydrogen atom;
○ both R^{b} and R^{d} represents a methyl group and
○ R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group and C₁₋₂ hydroxyalkyl group.

17. A compound of according to claim 1, wherein R¹ represents:
➢ an imidazo[1,2-a]pyridyl group or a 3*H*-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 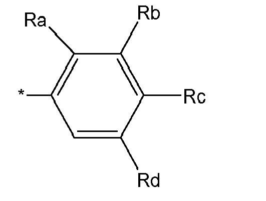 wherein:
• R^{a} represents a hydrogen atom, a methyl group, cyclopropyl group or a CF₃ group;
• R^{b} represents a hydrogen atom, a chlorine atom or a methyl group;
• R^{d} represents a hydrogen atom or a methyl group;
• R^{c} represents:
○ a C₁₋₄ hydroxyalkyl group or a C₁₋₄ aminoalkyl group substituted with one or more substituents selected from fluorine atoms and hydroxy groups;
○ a 4 to 6-membered saturated N-containing heterocyclic ring which is optionally substituted with a C₁₋₂ carboxyalkyl group
○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CONHS(O)₂R' -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein
○ R' represents a hydrogen atom or a methyl group,
○ R" represents a hydrogen atom, a methyl group, a cyclopropyl group, a piperidyl group, a C₁₋₂ carboxyalkyl group, a CF₃ group, C₁₋₄ hydroxyalkyl group, or
○ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

18. A compound according to claim 1, which is a compound of general formula (I'), or a pharmaceutically acceptable salt or N-oxide thereof: 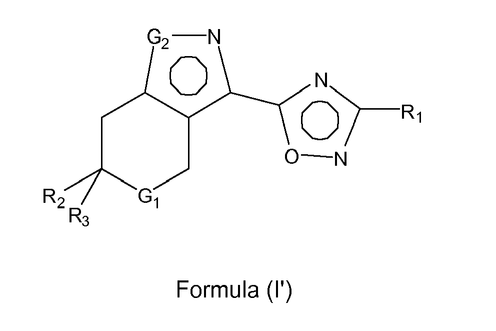 wherein,
G¹ is selected from the group consisting of -CH₂-, and -O-;
G² is selected from the group consisting of -NR⁴- and -O-;
R¹ represents:
➢ a pyrrolopyridyl group, which is unsubstituted or substituted with a C₁₋₂ carboxyalkyl group;
➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
➢ a pyridone group substituted with 1, 2 or 3 C₁₋₂ alkyl groups; or
➢ a group of formula: 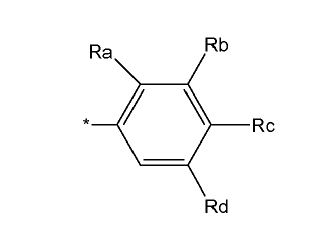 wherein:
• R^{a} represents a hydrogen atom, a C₁₋₂ alkyl group, a cyclopropyl group or a -CF₃ group;
• R^{b} represents a hydrogen atom, a chlorine atom, or a C₁₋₂ alkyl group;
• R^{d} represents a hydrogen atom, or a C₁₋₂ alkyl group;
• R^{c} represents:
○ a C₁₋₃ hydroxyalkyl group;
○ a carboxyethylpiperazine group;
○ -(CH₂)₍₀₋₂₎-C(O)OR', -(CH₂)₍₀₋₂₎-C(O)NR'R", -S(O)₂NR'R", or -(CH₂)₍₀₋₄₎-NR'R", wherein,
■ R' represents a hydrogen atom,
■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group.

19. A compound accroding to claim 1 which is one of:
4-(5-(6,6-Dimethyl-4,5,6,7-tetrahydro-2H-indazol-3-yl)-1,2,4-oxadiazol-3-yl)benzenesulfonamide
(4-(5-(6,6-Dimethyl-4,5,6,7-tetrahydro-2H-indazol-3-yl)-1,2,4-oxadiazol-3-yl)phenyl)metanol
(4-(5-(1,6,6-Trimethyl-4,5,6,7-tetrahydro-1H-indazol-3-yl)-1,2,4-oxadiazol-3-yl)phenyl)methanol
4-(5-(1,6,6-Trimethyl-4,5,6,7-tetrahydro-1H-indazol-3-yl)-1,2,4-oxadiazol-3-yl)benzenesulfonamide
4-(5-(1-Ethyl-6,6-dimethyl-4,5,6,7-tetrahydro-1H-indazol-3-yl)-1,2,4-oxadiazol-3-yl)benzenesulfonamide
5-(1-Ethyl-6,6-dimethyl-4,5,6,7-tetrahydro-1H-indazol-3-yl)-3-(pyridin-4-yl)-1,2,4-oxadiazole
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid
4-(5-(1-Ethyl-6,6-dimethyl-4,5,6,7-tetrahydro-1H-indazol-3-yl)-1,2,4-oxadiazol-3-yl)benzoic acid
4-(5-(1-Ethyl-6,6-dimethyl-4,5,6,7-tetrahydro-1H-indazol-3-yl)-1,2,4-oxadiazol-3-yl)benzamide
5-(1-Ethyl-6,6-dimethyl-4,5,6,7-tetrahydro-1H-indazol-3-yl)-3-(4-(piperazin-1-yl)phenyl)-1,2,4-oxadiazole
2-(4-(5-(1-Ethyl-6,6-dimethyl-4,5,6,7-tetrahydro-1H-indazol-3-yl)-1,2,4-oxadiazol-3-yl)phenyl)acetic acid
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid
2-(4-(5-(1-Ethyl-6,6-dimethyl-4,5,6,7-tetrahydro-1H-indazol-3-yl)-1,2,4-oxadiazol-3-yl)phenyl)acetamide
4-(5-(1-Ethyl-6,6-dimethyl-4,5,6,7-tetrahydro-1H-indazol-3-yl)-1,2,4-oxadiazol-3-yl)pyridine 1-oxide
N-(4-(5-(1-Ethyl-6,6-dimethyl-4,5,6,7-tetrahydro-1H-indazol-3-yl)-1,2,4-oxadiazol-3-yl)phenyl)piperidin-4-amine
5-(1-Ethyl-6,6-dimethyl-4,5,6,7-tetrahydro-1H-indazol-3-yl)-3-(6-methoxypyridin-3-yl)-1,2,4-oxadiazole
5-(5-(1-Ethyl-6,6-dimethyl-4,5,6,7-tetrahydro-1H-indazol-3-yl)-1,2,4-oxadiazol-3-yl)pyridin-2-ol
4-(5-(1-Ethyl-6,6-dimethyl-4,5,6,7-tetrahydro-1H-indazol-3-yl)-1,2,4-oxadiazol-3-yl)pyridin-2-ol
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide
4-(5-(1-Benzyl-6,6-dimethyl-4,5,6,7-tetrahydro-1H-indazol-3-yl)-1,2,4-oxadiazol-3-yl)benzamide
4-(5-(1-tert-Butyl-6,6-dimethyl-4,5,6,7-tetrahydro-1H-indazol-3-yl)-1,2,4-oxadiazol-3-yl)benzamide
5-(1-Ethyl-6,6-dimethyl-4,5,6,7-tetrahydro-1H-indazol-3-yl)-3-(2-methoxypyridin-4-yl)-1,2,4-oxadiazole
4-(5-(1-Ethyl-6,6-dimethyl-4,5,6,7-tetrahydro-1H-indazol-3-yl)-1,2,4-oxadiazol-3-yl)-1-methylpyridin-2(1H)-one
1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid
(4-(5-(1-Ethyl-6,6-dimethyl-4,5,6,7-tetrahydro-1H-indazol-3-yl)-1,2,4-oxadiazol-3-yl)phenyl)methanol
4-(5-(6,6-Dimethyl-1-phenyl-4,5,6,7-tetrahydro-1H-indazol-3-yl)-1,2,4-oxadiazol-3-yl)benzamide
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol
5-(5-(1-Ethyl-6,6-dimethyl-4,5,6,7-tetrahydro-1H-indazol-3-yl)-1,2,4-oxadiazol-3-yl)-3-methylpyridin-2-ol
4-(5-(1-Ethyl-6,6-dimethyl-4,5,6,7-tetrahydro-1H-indazol-3-yl)-1,2,4-oxadiazol-3-yl)-3-methylbenzamide
4-(4-(5-(1-Ethyl-6,6-dimethyl-4,5,6,7-tetrahydro-1H-indazol-3-yl)-1,2,4-oxadiazol-3-yl)benzyl)morpholine
3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one
5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one
N-Cyclopropyl-4-(5-(1-ethyl-6,6-dimethyl-4,5,6,7-tetrahydro-1H-indazol-3-yl)-1,2,4-oxadiazol-3-yl)benzamide
1-(4-(5-(1-Ethyl-6,6-dimethyl-4,5,6,7-tetrahydro-1H-indazol-3-yl)-1,2,4-oxadiazol-3-yl)phenyl)-N-methylmethanamine
5-(1-Ethyl-6,6-dimethyl-4,5,6,7-tetrahydro-1H-indazol-3-yl)-3-(3-methylpyridin-4-yl)-1,2,4-oxadiazole
4-(5-(6,6-Dimethyl-1-(2,2,2-thfluoroethyl)-4,5,6,7-tetrahydro-1H-indazol-3-yl)-1,2,4-oxadiazol-3-yl)benzamide
(4-(5-(1-Ethyl-6,6-dimethyl-4,5,6,7-tetrahydro-1H-indazol-3-yl)-1,2,4-oxadiazol-3-yl)phenyl)methanamine
5-(1-Ethyl-6,6-dimethyl-4,5,6,7-tetrahydro-1H-indazol-3-yl)-3-(3-methylpyridin-2-yl)-1,2,4-oxadiazole
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine
5-(5-(1-Ethyl-6,6-dimethyl-4,5,6,7-tetrahydro-1H-indazol-3-yl)-1,2,4-oxadiazol-3-yl)-4-methylpyridin-2-ol
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid
5-(5-(1-Ethyl-6,6-dimethyl-4,5,6,7-tetrahydro-1H-indazol-3-yl)-1,2,4-oxadiazol-3-yl)-6-methylpyridin-2-ol
5-(1-Ethyl-6,6-dimethyl-4,5,6,7-tetrahydro-1H-indazol-3-yl)-3-(4-methylpyridin-3-yl)-1,2,4-oxadiazole
5-(1-Ethyl-6,6-dimethyl-4,5,6,7-tetrahydro-1H-indazol-3-yl)-3-(4-(trifluoromethyl)pyridin-3-yl)-1,2,4-oxadiazole
5-(1-Ethyl-6,6-dimethyl-4,5,6,7-tetrahydro-1H-indazol-3-yl)-3-(imidazo[1,2-a]pyridin-6-yl)-1,2,4-oxadiazole
3-(4-(5-(1-Ethyl-6,6-dimethyl-4,5,6,7-tetrahydro-1H-indazol-3-yl)-1,2,4-oxadiazol-3-yl)-3-methylphenyl)propanamide
2-(4-(5-(1-Ethyl-6,6-dimethyl-4,5,6,7-tetrahydro-1H-indazol-3-yl)-1,2,4-oxadiazol-3-yl)-3-methylphenyl)ethanamine
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine
4-(5-(1-Ethyl-6,6-dimethyl-4,5,6,7-tetrahydro-1H-indazol-3-yl)-1,2,4-oxadiazol-3-yl)-3-(trifluoromethyl)benzoic acid
4-(5-(1-Ethyl-6,6-dimethyl-4,5,6,7-tetrahydro-1H-indazol-3-yl)-1,2,4-oxadiazol-3-yl)-3-(trifluoromethyl)benzamide
5-(1-Ethyl-6,6-dimethyl-4,5,6,7-tetrahydro-1H-indazol-3-yl)-3-(2-methylpyridin-3-yl)-1,2,4-oxadiazole
5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine
3-Cyclopropyl-4-[5-(1-ethyl-6,6-dimethyl-4,5,6,7-tetrahydro-1H-indazol-3-yl)-1,2,4-oxadiazol-3-yl]benzamide
5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol
5-(5-(6,6-Dimethyl-1-(2,2,2-thfluoroethyl)-4,5,6,7-tetrahydro-1H-indazol-3-yl)-1,2,4-oxadiazol-3-yl)-3-ethyl-6-methylpyridin-2-ol
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide
3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol
3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine
2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine
3-(2,6-Dimethyl-4-(5-(1,6,6-trimethyl-4,5,6,7-tetrahydro-1H-indazol-3-yl)-1,2,4-oxadiazol-3-yl)phenyl)propanoic acid
3-(2,6-Dimethyl-4-(5-(1,6,6-trimethyl-4,5,6,7-tetrahydro-1H-indazol-3-yl)-1,2,4-oxadiazol-3-yl)phenyl)propanamide
3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine
2-(2,6-Dimethyl-4-(5-(1,6,6-trimethyl-4,5,6,7-tetrahydro-1H-indazol-3-yl)-1,2,4-oxadiazol-3-yl)phenyl)ethanamine
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine
5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one
2-(Dimethyl-4,5,6,7-tetrahydrobenzo[d]isoxazol-3-yl)-1,2,4-oxadiazol-3-yl)-2,6-dimethylphenethylamino)ethanoic acid
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid
3-(Dimethyl-4,5,6,7-tetrahydrobenzo[d]isoxazol-3-yl)-1,2,4-oxadiazol-3-yl)-2,6-dimethylphenethylamino)propanoic acid
3-Ethyl-5-(5-(1-ethyl-4,5,6,7-tetrahydro-1H-indazol-3-yl)-1,2,4-oxadiazol-3-yl)-6-methylpyridin-2(1H)-one
3-Ethyl-6-methyl-5-(5-(1,6,6-trimethyl-4,5,6,7-tetrahydro-1H-indazol-3-yl)-1,2,4-oxadiazol-3-yl)pyridin-2-ol
5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one
5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine
2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine
3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid
3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one
3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid
3-(2-Methyl-4-(5-(1,6,6-trimethyl-4,5,6,7-tetrahydro-1H-indazol-3-yl)-1,2,4-oxadiazol-3-yl)phenyl)propanoic acid
4-(2,6-Dimethyl-4-(5-(1,6,6-trimethyl-4,5,6,7-tetrahydro-1H-indazol-3-yl)-1,2,4-oxadiazol-3-yl)phenethyl)morpholine
3-(4-(5-(6,6-Dimethyl-4,5,6,7-tetrahydro-1H-indazol-3-yl)-1,2,4-oxadiazol-3-yl)-2,6-dimethylphenyl)propane-1,2-diol
3-(4-(5-(1-Ethyl-6,6-dimethyl-4,5,6,7-tetrahydro-1H-indazol-3-yl)-1,2,4-oxadiazol-3-yl)phenylamido)propanoic acid
3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol
3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid
3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol
3-(1-Ethyl-6,6-dimethyl-4,5,6,7-tetrahydro-1H-indazol-3-yl)-5-(pyridin-4-yl)-1,2,4-oxadiazole
5-(1-Ethyl-6,6-dimethyl-4,5,6,7-tetrahydro-1H-indazol-3-yl)-3-o-tolyl-1,2,4-oxadiazole
3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid
3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid
or a pharmaceutically acceptable salt or N-oxide thereof

20. A compound according to any one of claims 1 to 19 for use in the treatment of a pathological condition or disease susceptible to amelioration by sphingosine-1-phosphate receptors (S1P1) agonists.

21. A compound according to claim 20, wherein the pathological condition or disease is selected from autoimmune diseases, chronic immune and inflammatory diseases, transplant rejection, malignant neoplastic diseases, angiogenic-related disorders, pain, neurological diseases, viral and infectious diseases.

22. A compound according to claim 21 wherein the pathological condition or disease is selected from multiple sclerosis, transplant rejection, systemic lupus erythematosus, asthma, psoriasis, rheumatoid arthritis, psoriatic arthritis and Crohn's disease,

23. A pharmaceutical composition comprising a compound as defined in any one of claims 1 to 19 in association with a pharmaceutically acceptable diluent or carrier.

24. Use of a compound as defined in any one of claims 1 to 19 in the manufacture of a medicament for the treatment of a pathological condition or disease as defined in claims 20 to 22.

25. A method for treating a subject afflicted with a pathological condition or disease as defined in claims 20 to 22, which comprises administering to said subject a therapeutically effective amount of a compound as defined in any one of claims 1 to 19.

26. A combination product comprising (i) a compound according to any one of claims 1 to 19; and (ii) another compound selected from:
a) Beta interferons such as Betaseron, Avonex or Rebif
b) Immunomodulators such as glatiramer acetate
c) Inhibitors of DNA synthesis and repair, such as Mitoxantrone
d) Anti-alpha 4 integrin antibodies, such as Natalizumab (Tysabri)
e) Alpha 4 integrin antagonists such as R-1295 , TBC-4746, CDP-323, ELND-002, Firategrast and TMC-2003
f) Dyhydrofolate reductase inhibitors, such as Methotrexate or CH-1504
g) Glucocorticoids such as prednisone or methylprednisolone
h) DHODH inhibitors such as teriflunomide
*i)* Fumaric acid esters, such as *BG-12*
*j)* Immunomodulators such as Laquinimod
k) Anti-CD20 monoclonal antibodies such as Rituximab, Ocrelizumab Ofatumumab or TRU-015
l) Anti-CD52 such as alemtuzumab
m) Anti-CD25 such as daclizumab
n) Anti-CD88, such as eculizumab or pexilizumab
o) Calcineurin inhibitors such as cyclosporine A or tacrolimus
p) IMPDH inhibitors, such as mycophenolate mophetyl
q) Cannabinoid receptor agonists such as Sativex
r) Chemokine CCR1 antagonists such as MLN-3897 or PS-031291
s) Chemokine CCR2 antagonists such as INCB-8696
t) Interferon alpha such as Sumiferon MP
u) NF-kappaB activation inhibitors such as FAE and MLN-0415
v) JAK inhibitors such as CP-690550 or INCB018424
w) Syk inhibitors, such as R-112
x) PKC inhibitors, such as NVP-AEB071
y) Phosphosdiesterase IV inhibitors such as GRC-4039
z) P38 Inhibitors such as ARRY-797
aa) MEK inhibitors, such as ARRY-142886 or ARRY-438162
